(19) **Europäisches Patentamt / European Patent Office / Office européen des brevets**

(11) **EP 1 587 478 B1**

(12) **EUROPEAN PATENT SPECIFICATION**

(45) Date of publication and mention of the grant of the patent:
**10.07.2013 Bulletin 2013/28**

(21) Application number: **04701047.5**

(22) Date of filing: **09.01.2004**

(51) Int Cl.:
**A61K 6/00** (2006.01)

(86) International application number:
**PCT/GB2004/000044**

(87) International publication number:
**WO 2004/062560 (29.07.2004 Gazette 2004/31)**

(54) **PHARMACEUTICAL COMPOSITION**

PHARMAZEUTISCHE ZUSAMMENSETZUNG

COMPOSITION PHARMACEUTIQUE

(84) Designated Contracting States:
**AT BE BG CH CY CZ DE DK EE ES FI FR GB GR HU IE IT LI LU MC NL PT RO SE SI SK TR**

(30) Priority: **09.01.2003 GB 0300427**

(43) Date of publication of application:
**26.10.2005 Bulletin 2005/43**

(73) Proprietors:
- **University of Strathclyde**
  **Glasgow G1 1XQ (GB)**
- **University Court of The University of Glasgow**
  **Glasgow G12 8QQ (GB)**

(72) Inventors:
- **MOORE, Barry, Douglas**
  **Crosshill,**
  **Glasgow G42 8DT (GB)**
- **PARKER, Marie, Claire**
  **Crosshill,**
  **Glasgow G42 8DT (GB)**
- **PARTRIDGE, Johann**
  **Jordanhill,**
  **Glasgow G14 9LX (GB)**
- **VOS, Jan**
  **Glasgow G5 9JG (GB)**
- **KREINER, Michaela, Maria**
  **Glasgow G12 9AE (GB)**
- **STEVENS, Howard, Norman, Ernest**
  **Dalserf ML9 3BN (GB)**
- **FLORES, Maria, Victoria**
  **Thornaby TS17 6DG (GB)**
- **ROSS, Alistair**
  **Glasgow G12 8QQ (GB)**

(74) Representative: **Wilson, Gary et al**
**Harrison Goddard Foote LLP**
**Delta House**
**50 West Nile Street**
**Glasgow G1 2NP (GB)**

(56) References cited:
| | |
|---|---|
| WO-A-01/32125 | WO-A-03/080034 |
| DE-A- 19 925 184 | US-A- 5 945 126 |

**Description**

**Field of the Invention**

This invention relates in general to pharmaceutical formulations comprising particles with a substantially non-hygroscopic inner crystalline core and an outer coating comprising at least one bioactive molecule, as well as methods of forming particles comprising a substantially non-hygroscopic inner crystalline core and an outer coating comprising at least one bioactive molecule.

**Background of the Invention**

WO 0069887, which is a previous application by the present inventors, relating to protein coated microcrystals. However, there is no specific disclosure of pharmaceutical formulations or other bioactive molecules. The coated crystals disclosed in WO 0069887 are generally coprecipitated from saturated solutions and there is no disclosure that it would be advantageous to use a less than saturated solution.

In WO 00/69887 production of PCMCs by addition of an excess of saturated aqueous solution to solvent is described. The PCMCs described are not suitable for pharmaceutical use. The preferred method in WO 00/69887 for obtaining efficient admixing was to dropwise add the aqueous solution to an excess of organic miscible solvent with vigorous mixing. However, this batch type process suffers from a number of drawbacks:

a) the precipitation conditions are continuously varying because the water content of the solvent is increasing throughout. It has been found that different initial water content leads to different sizes and shapes of crystals;
b) the precipitation is carried out into a suspension that contains an increasing quantity of crystals already in suspension. This will enhance the likelihood of nascent crystals fusing onto already formed crystals; and
c) if a large-scale batch is required it is difficult to obtain high efficiency agitation with stirred batch reactors without excessive shear forces. High efficiency agitation is generally required to minimise crystal size and prevent cementing of crystals into aggregates. However, high shear forces can initiate damage to the bioactive molecule such as protein denaturation or nicking of nucleic acids. Alternative approaches to rapid mixing such as nebulising the aqueous inflow to provide very small droplets also have potential problems arising from shear forces and interfacial denaturation processes.

Taken together, there is a need to develop improved methods for obtaining consistent and reproducible pharmaceutical formulations of the particles on a large scale in order to enable to support clinical trials and manufacture.

The present inventors have now discovered that many of the above problems can be solved using a flow precipitator. This operates by mixing together a continuous stream of the saturated aqueous solution and a continuous stream of the solvent in a small mixing flow chamber similar to those used for creating solvent gradients for HPLC chromatography. The co-precipitation process is initiated in the mixing chamber and the particles then flow out as a suspension in the solvent stream to be collected in a holding vessel. Surprisingly, it is found that the process can be operated for extended periods with no blocking of the inlet tubes as might be expected with such a co-precipitation process. Advantageously, the particles exiting the mixing chamber are found to be highly consistent in size, shape and yield over the whole operating cycle indicating the co-precipitation conditions remain constant. A further advantage is that the flow system can run for many hours unattended and in so doing produce large quantities of particles.

Since the overall system may be sealed and sterilised and each solvent stream can be independently filtered through a sterile filter, the whole process can also be made sterile as required for pharmaceutical formulation manufacture.

**Summary of the Invention**

According to a first aspect of the present invention there is provided a continuous method of forming particles comprising the following steps:

(a) providing an aqueous solution comprising coprecipitant molecules and bioactive molecules, each coprecipitant molecule substantially having a molecular weight of less than 4kDa, wherein the aqueous solution is capable of forming a coprecipitate which comprises the coprecipitant and bioactive molecules with a melting point of above about 90°C;
(b) rapidly admixing the bioactive molecule/coprecipitant molecule solution with a greater volume of a substantially water miscible organic solvent such that the coprecipitant and bioactive molecules coprecipitate from solution forming said particles; and
(c) optionally isolating the particles from the organic solvent.

By continuous process herein is meant a process which is constantly repeated over a time period and is therefore different from a batch process i.e. continuous process means uninterrupted addition of the bioactive molecule/coprecipitant molecule solution with the water miscible organic solvent. A feature of the continuous process is that the particles are in, for example, a mixing chamber for a minimal period. This may prevent fusion and may also minimise protein degradation.

In the continuous process steps (a) and (b) are cyclically repeated.

The bioactive molecule may be provided as a solid, for example, as a powder, which is to be dissolved in the aqueous solution of coprecipitant. Alternatively, the bioactive molecule may be in a solution or suspension prior to mixing with the aqueous solution of coprecipitant. Typically, the coprecipitant may be prepared as a substantially saturated or highly concentrated solution. Following mixing with the bioactive molecule the coprecipitant will typically be at between 5 and 100 % of its aqueous saturation solubility. Preferably it will be between 20 and 80% of its saturation solubility.

The coprecipitant must be sufficiently soluble in the aqueous solution such that a suitable weight fraction may be obtained relative to the bioactive molecule in solution. Preferably, the coprecipitant has a substantially lower solubility in the miscible organic solvent than in the aqueous solution. The concentration of coprecipitant required is a function of the amount of bioactive molecule in the solution and the molecular mass of the bioactive molecule.

The skilled addressee will appreciate that the coprecipitant should be chosen so that it does not substantially react and/or cause an adverse reaction with the bioactive molecule.

The bioactive/coprecipitant solution is admixed with a substantially water miscible organic solvent or water miscible mixture of solvents, preferably one where the solvent or solvent mixture is substantially fully miscible. Typically, the bioactive molecule /coprecipitant solution is added to an excess of water miscible organic solvent. The excess of fully water miscible organic solvent is such that the final water content of the solvent/aqueous solution is generally less than 30%, typically less than 10-20 vol% and conveniently less than 8 vol%. In this manner, the organic solvent should preferably initially contain less than 0.5-5 vol% water or be substantially dry, but may not necessarily be completely dry.

Typical water miscible organic solvents may, for example, be: methanol; ethanol; propan-1-ol; propan-2-ol; acetone, ethyl lactate, tetrahydrofuran, 2-methyl-2,4-pentanediol, 1,5-pentane diol, and various size polyethylene glycol (PEGS) and polyols; or any combination thereof.

In certain circumstances, the organic solvent may be pre-saturated with the bioactive molecule and/or coprecipitate to ensure that on addition of the aqueous solution the two components precipitate out together.

It should be understood that the term "admixed" refers to a process step wherein the water miscible organic solvent is mixed or agitated with the aqueous solution while the aqueous solution is added. The mixing needs to be efficient so that the bioactive molecule is in contact with a mixture of intermediate composition i.e. aqueous solution and organic solvent, for example, between 25% and 60% solvent, for a minimal time. Thus, the aqueous solution may be added to the organic solvent using a wide range of methods such as a continual stream, spray or mist. Typically the admixing of the bioactive molecule and coprecipitate solution may occur in a process wherein a continuous stream of bioactive molecules and coprecipitate are mixed together with an amount of solvent.

The present inventors have now found that a continuous, as opposed to batch-wise co-precipitation process is advantageous which may operate by mixing together two or more continuous streams. Thus a continuous stream of water miscible organic solvent or mixture of solvents may be mixed with a continuous aqueous stream comprising a bioactive molecule/co-precipitant solution in, for example, a small mixing flow chamber. The water miscible solvent stream may contain water at less than 5 vol% and/or be substantially saturated with coprecipitant to aid coprecipitation. The aqueous stream or solvent stream may also contain other excipients typically employed in pharmaceutical formulations such as buffers, salts and/or surfactants. The co-precipitation process may be initiated in the mixing chamber with the formed particles flowing out as a suspension in the mixed solvent stream to be collected in a holding vessel. The particles exiting the mixing chamber have been found to be substantially consistent in size, shape and yield. Advantageously this continuous process may be carried out over a wide temperature range including temperature between 0 °C and ambient temperature as well as elevated temperatures. Also advantageously the particles may be collected as a suspension in solvent using a holding vessel held at various pressures including atmospheric pressure. Running a continuous process under conditions close to ambient may lead to reduced capital and operating costs relative to conventional methods of forming particles for pharmaceutical applications such as spray-drying or super-critical fluid processing. It is envisaged that large quantities of bioactive molecule coated particles, for example, may be produced in this manner on an industrial scale.

Alternatively, the bioactive molecule or coprecipitant may be omitted from the aqueous stream and the process used to form uncoated particles. The uncoated particles may for example comprise an excipient or drug useful for pharmaceutical formulation purposes. This can provide a convenient method for producing microcrystals of an excipient or drug in a cost effective process. Excipients or drugs produced in a microcrystalline form may show enhanced properties such as improved flow or compressibility characteristics.

In the continuous co-precipitation system one pump may continuously deliver aqueous solution containing concentrated coprecipitant and bioactive molecule while another pump may deliver a coprecipitant saturated solvent phase. Further pumps may be used if a third component such as a particle coating material is required.

The pumps may be of many different kinds but must accurately deliver the solutions at a defined flow rate and be compatible with the bioactive molecules employed. Conveniently, HPLC pumps or the like can be used since these are optimised for delivering aqueous solutions and water miscible solvents over a range of flow rates. Typically, the aqueous solution will be delivered at flow rates between 0.1 ml/min and 20 ml/min. The aqueous pump head and lines may be made of material that resists fouling by the bioactive molecule. The solvent may generally be delivered 4-100 times faster than the aqueous and so a more powerful/efficient pump may be required. Typically the solvent may be delivered at between 2 ml/min and 200 ml/min.

A mixing device may provide a method for rapidly and intimately admixing a continuous aqueous stream with a continuous water miscible solvent stream such that precipitation begins to occur almost immediately.

The mixing device may be any device that achieves rapid mixing of the two flows. Thus it can, for example, be a static device that operates by shaping/combining the incoming liquid flow patterns or else a dynamic device that actively agitates the two fluid streams together. Preferably, it is a dynamic device. Agitation of the two streams may be achieved by use of a variety of means such as stirring, sonication, shaking or the like. Methods of stirring include a paddle stirrer, a screw and a magnetic stirrer. If magnetic stirring is used a variety of stirring bars can be used with different profiles such as, for example, a simple rod or a Maltese cross. The material lining the interior of the mixing device may preferably be chosen to prevent significant binding of the bioactive molecule or the particles onto it. Suitable materials may include 316 stainless steel, titanium, silicone and Teflon (Registered Trade Mark).

Depending on the production scale required the mixing device may be produced in different sizes and geometries. The size of the mixing chamber required is a function of the rate of flow of the two solvent streams. For flow rates of about 0.025 - 2 ml/min of aqueous and 2.5-20 ml/min of solvent it is convenient to use a 0.2 ml mixing chamber.

Typically, in a continuous process the bioactive/coprecipitate solution is added to an excess of water miscible organic solvent. This entails the smaller volume of bioactive molecule/coprecipitate solution being added to the larger volume of the excess of organic solvent such that rapid dilution of water from the bioactive molecule/coprecipitate solution into the organic solvent occurs with an accompanying rapid dehydration of the bioactive molecule and formation of particles according to the first aspect. The temperature at which the precipitation is carried out may be varied. For example, the aqueous solution and the solvent may be either heated or cooled. Cooling may be useful where the bioactive molecule is fragile. Alternatively, the solvent and aqueous mixtures may be at different temperatures. For example, the solvent may be held at a temperature below the freezing point of the aqueous mixture. Moreover, the pressure may also be varied, for example, higher pressures may be useful to reduce the volatility of the solvent.

Upon admixing the bioactive molecule/coprecipitant solution to the excess of the water miscible organic solvent, precipitation of the bioactive and coprecipitant occurs substantially instantaneously.

Typically, the precipitated particles may be further dehydrated by rinsing with fresh organic solvent containing low amounts of water. This may also be useful to remove residual solvent saturated in coprecipitant. On drying this residual coprecipitant may otherwise serve to cement particles together leading to the formation of aggregates. Rinsing with solutions of excipients prior to drying or storage may also be used to introduce other excipients onto the particles.

It has advantageously been found that the precipitated particles may be stored in an organic solvent and that the bioactive molecules display extremely good retention of activity and stability over an extended period of time. Moreover, precipitated bioactive molecules stored in an organic solvent, will typically be resistant to attack by bacteria, thus increasing their storage lifetime.

With time the coprecipitate will settle, which allows easy recovery of a concentrated suspension of particles by decanting off excess solvent. The copecipitate may, however, be subjected to, for example, centrifugation and/or filtration in order to more rapidly recover the precipitated particles. Conventional drying procedures known in the art such as air drying, vacuum drying or fluidised bed drying may be used to evaporate any residual solvent to leave solvent free particles.

Alternatively, solvent may be removed from the particles in a drying procedure using supercritical $CO_2$. Typically, particles in a solvent prepared in a continuous process, and also using a batch-type process and non-pharmaceutical particles in a solvent prepared as defined in WO 0069887 may be loaded into a high pressure chamber with supercritical fluid $CO_2$ flowing through the suspension until the solvent (or as much as possible) has been removed. This technique removes virtually all residual solvent from the particles. This is of particular benefit for pharmaceutical formulation since residual solvent may lead to unexpected physiological effects. A further advantage of super-critical fluid drying of the suspensions is that it can be used to produce powders and pharmaceutical formulations with much lower bulk density than obtained by other isolation techniques. Typically bulk densities lower than 0.75 g/ml may be obtained. Low bulk density formulations are particularly useful for pulmonary delivery of bioactive molecules since they generally contain fewer strongly bound aggregates. The critical point drying may be carried out in a number of different ways known in the art.

It is therefore possible to set up a continuous co-precipitation system to form particles according to the first aspect and, in fact, any other type of particles and then dry the particles using supercritical $CO_2$.

For pharmaceutical applications dry precipitated particles may be typically introduced into a sterile delivery device or vial under sterile conditions prior to use. Alternatively the particles may be transferred into the sterile delivery device or vial as a suspension in solvent under sterile conditions. They may then be optionally dried in situ using for example supercritical $CO_2$ drying.

The methods described herein may also allow organic soluble components present in the aqueous solution to be separated from the bioactive molecules. For example, a buffer such as Tris which in its free base form is soluble in an organic solvent like ethanol may be separated from the bioactive molecule during precipitation. However, it may be necessary to convert all the buffer to the free base by the addition of another organic soluble base to the aqueous solution or organic solvent. Thus the present invention also discloses a method of removing undesirable components from the bioactive molecule such that the undesirable components are not co-precipitated with the bioactive molecule and so remain dissolved in the organic phase. This may be achieved by the inclusion of additives such as acids, bases, ion-pairing and chelating agents in aqueous or organic solvent prior to bioactive molecule precipitation of the non-hygroscopic coated particles. The bioactive molecules may therefore be coated in a highly pure form.

The formulations described in the invention may typically be produced at a number of dosage strengths. The dosage may be conveniently varied by varying the percentage weight of bioactive molecule per particle from below 0.1 wt% up to about 50 wt%. For bioactive molecules that have low solubility in aqueous solution or else are unstable at high aqueous concentrations, it is advantageous to use carriers that form saturated aqueous solutions at low concentrations. This then allows high loadings to be achieved using low concentrations of the bioactive molecule. The carrier solubility may provide the possibility of producing particles that contain bioactive molecules at loadings from 50 wt% to <0.1 wt% so that the dosage strength of the pharmaceutical formulation can be conveniently varied. The carrier solubility in aqueous solution at room temperature may range from 2-200 mg/ml and more preferable in the range 10-150 mg/ml.

The use of carrier dissolved at concentrations lower than 80 mg/ml can advantageously be used to produce pharmaceutical formulations containing free-flowing particles that span a narrow size distribution with a mean particle size of less than 50 microns. Formulations containing a narrow size distribution of coated crystals provide improved delivery reproducibility and hence better clinical performance.

The pharmaceutical formulations described can be conveniently produced in a sterile form by pre-filtering the aqueous and organic solutions through 0.2 micron filters prior to admixing them in a contained sterile environment. Pharmaceutical formulations should be substantially free of harmful residual solvents and this invention typically provides powders containing less than 0.5 wt% of a Class 3 solvent following conventional drying procedures. Substantially lower solvent levels are obtainable by flowing supercritical fluid $CO_2$ through a suspension of the crystals in a dry water miscible and $CO_2$ miscible solvent.

The method may also be used to make bioactive molecule coated microcrystals suitable for pharmaceutical formulations using water-soluble bioactive compounds that are much smaller than typical biological macromolecules. These formulations may be made either by a batch or a continuous process and may advantageously employ a non-hygroscopic carrier such as D,L-valine. Water-soluble antibiotic drugs such as tobramycin sulphate and other water-soluble bioactive molecules may be used. Preferably, the bioactive molecule may be polar and contain one or more functional groups that is ionised at the pH used for coprecipitation. The bioactive molecule should also preferably have a largest dimension greater than that of the unit cell formed by the core material on crystallisation. This will favour formation of bioactive molecule coated microcrystals and minimise the possibility of inclusion of the bioactive molecule within the crystal lattice.

According to a second aspect of the present invention there is provided a pharmaceutical formulation comprising particles wherein the particles comprise:

   (a) a substantially non-hygroscopic inner crystalline core comprising coprecipitant molecules wherein said coprecipitant molecules have a molecular weight of less than 4kDa; and
   (b) an outer coating comprising one or more bioactive molecules

wherein the particles have been formed in a single step by coprecipitating said core forming coprecipitant molecules and said bioactive molecule(s) together and wherein the particles have a melting point of above about 90°C.

The particles may be made by either a continuous process according to the first aspect or an a batch process.

By substantially non-hygroscopic herein is meant that the crystalline core does not readily take-up and retain moisture. Typically, the particles will not aggregate nor will the core under-go significant changes in morphology or crystallinity on exposure to about 80% relative humidity at room temperature.

By crystalline core is meant that the constituent molecules or ions are organised into a solid 3-dimensional crystal lattice of repeating symmetry that remains substantially unchanged on heating until a well-defined melting transition temperature is reached. Conveniently, the molecules form a crystalline core with a high degree of crystallinity. Typically, a well-defined melting endotherm (i.e. not a glass transition) may be observed on heating the particles in a differential scanning calorimeter (DSC). This is a well-known characteristic showing crystallinity and also shows that the crystalline core may be generally substantially composed of solid-state phases that are thermodynamically stable at room temperature and ambient humidity. The particles according to the present invention may also show birefringence which is also a characteristic of crystallinity. The particles may also shown an X-ray diffraction pattern which is yet again evidence of crystallinity.

By single step is meant that the molecules or ions that provide the crystalline core and the bioactive molecules that provide the outer coating precipitate out of solution together directly in the form of coated particles. i.e. in a one-step procedure. There is therefore no requirement for a separate coating or milling step. It should also be understood that particle formation does not require any evaporative processes such as occur for example in spray-drying or freeze-drying.

The particles may be used in a medical application such as a therapy or a diagnostic method such as in a kit form to detect, for example, the presence of a disease. Diseases which may include diseases of the lung such as lung cancer, pneumonia, bronchitis and the like, where the particles may be delivered to the lung and the lung capacity/effectiveness tested, or disease causing agents identified. The particles may be used in veterinary uses.

Typically, the coating of bioactive molecules may be substantially continuous. Alternatively, it may be advantageous to have a pharmaceutical formulation comprising particles with a substantially discontinuous coating of bioactive molecules. The coating may also vary in thickness and may range from about 0.01 to 1000 microns, about 1 to 100 microns, about 5 to 50 microns or about 10 to 20 microns.

The pharmaceutical formulation may desirably comprise particles with a narrow size distribution. Typically, the pharmaceutical formulation may therefore comprise a substantially homogeneous system with a significant number of particles having generally the same or similar size.

Microcrystals and bioactive molecule coated microcrystals produced by a continuous process typically exhibit a narrow size distribution with a Span less than 5, preferably less than 2 and more preferably less than 1.5 Bioactive molecule coated microcrystals produced by coprecipitation are typically advantageously smaller than microcrystals produced by precipitation of the pure carrier material. This is consistent with coating of the bioactive molecule on the microcrystal surface. Span values are calculated as follows:

d(0.1) ($\mu$m) = 10% of the particles are below this particle size.
d(0.5) ($\mu$m) = 50% of the particles are above and below this particle size.
d(0.9) ($\mu$m) = 90% of the particles are below this particle size.

$$\texttt{Span = d(0.9) - d(0.1) / d(0.5).}$$

The particles may have a maximum cross-sectional dimension of less than about 80$\mu$m, preferably less than 50$\mu$m across or more preferably less than 20$\mu$m. By maximal cross-sectional dimension is meant the largest distance measurable between the diametrically opposite points.

The molecules making up the crystalline core may typically each have a molecular weight less than 2kDa. Preferably, the molecules making up the crystalline core each have a molecular weight of less than 1kDa. More preferably, the molecules making up the crystalline core each have a molecular weight of less than 500 Daltons. Preferred molecules are those that can be rapidly nucleated to form crystals on undergoing precipitation. Molecules that provide particles that consist substantially of amorphous aggregates or glasses are therefore generally not suitable as core materials.

Typically, the molecules forming the crystalline core have a solubility in water of less than 150 mg/ml and preferably less than 80 mg/ml. Surprisingly, it has been found by the present inventors that molecules with solubilities less than these values tend to produce crystals with improved flow properties. Free-flowing particles are generally preferred for many pharmaceutical manufacturing processes since they, for example, facilitate filling capsules with precise dosages and can be conveniently used for further manipulation such as coating. Free flowing particles are generally of regular size and dimensions, with low static charge. Needle shaped crystals of high aspect ratio are, for example, generally not free flowing and are therefore not preferred in certain formulations.

The molecules which make up the crystalline core may, for example, be: amino acids, zwitterions, peptides, sugars, buffer components, water soluble drugs, organic and inorganic salts, compounds that form strongly hydrogen bonded lattices or derivatives or any combinations thereof. Typically, the molecules are chosen so as to minimise adverse physiological responses following administration to a recipient.

Amino acids suitable for forming the crystalline core may be in the form of pure enantiomers or racemates, Examples include: alanine, arginine, asparagine, glycine, glutamine, histidine, lysine, leucine, isoleucine, norleucine, D-valine, L-valine, mixtures of D,L-valine, methionine, phenylalanine, proline and serine or any combination thereof. In particular, L-glutamine, L-histidine, L-serine, L-methionine, L-isoleucine, L-valine or D,L-valine are preferred. For amino-acids that have side-chains that substantially ionise under coprecipitation conditions it is preferable to use counterions that generate crystalline salts with low solubility and which are non-hygroscopic. Examples of other molecules and salts for forming the crystalline core may include, but are not limited to $\alpha$-lactose, $\beta$-lactose, mannitol, ammonium bicarbonate, sodium glutamate, arginine phosphate and betaines.

Typically, the molecules forming the crystalline core have a low solubility in water of, for example, between about 12-150 mg/ml and preferably about 20-80 mg/ml at about 25°C. Molecules with a solubility of above about 150 mg/ml in water may also be used to obtain free flowing particles provided that they are coprecipitated from a sub-saturated aqueous solution. Preferably they are coprecipitated at a concentration of 150 mg/ml or less and more preferably of 80 mg/ml or less. For molecules of high aqueous solubility at 25°C it may also be advantageous to use lower coprecipitation temperatures such as 10°C or 4°C so that they are closer to saturation at concentrations of 150 mg/ml or less. Similarly higher temperatures such as 35°C or 50°C may be used for coprecipitation of core forming molecules poorly soluble at 25°C.

The molecules forming the crystalline core have a melting point of greater than 90°C such as above 120°C and preferably above 150°C. Having a high melting point means that that the crystals formed have a high lattice energy. A high lattice energy increases the likelihood of the particles formed having a crystalline core with the bioactive molecule coated on the surface and will tend to minimise the amorphous content of the particles. Particles which contain amorphous material can undergo undesirable changes in physical properties on exposure to high humidities or temperatures and this can lead to changes in bioactivity and solubility which are undesirable for pharmaceutical formulation. It is therefore advantageous to use coprecipitant that results in particles with a high melting point since these will tend to form more stable pharmaceutical formulations.

A typical weight ratio of the solvent:$H_2O$:carrier:bioactive agent in a suspension of freshly formed particles may range from about 1000:100:5:3 to about 1000:100:5:0.03. The weight ratio of the solvent:$H_2O$ may range between about 100:1 to about 4:1.

Conveniently, bioactive molecules forming a coating on the crystalline core may be selected from any molecule capable of producing a therapeutic effect such as for example an active pharmaceutical ingredient (API) or diagnostic effect. By therapeutic effect is meant any effect which cures, alleviates, removes or lessens the symptoms of, or prevents or reduces the possibility of contracting any disorder or malfunction of the human or animal body and therefore encompasses prophylactic effects.

The coating of bioactive molecules may also comprise excipients commonly used in pharmaceutical formulations such as stabilizers, surfactants, isotonicity modifiers and pH/buffering agents..

The bioactive molecules may, for example, be: any drug, peptide, polypeptide, protein, nucleic acid, sugar, vaccine component, or any derivative thereof or any combination which produces a therapeutic effect.

Examples of bioactive molecules include, but are not limited to drugs such as: anti-inflammatories, anti-cancer, anti-psychotic, anti-bacterial, anti-fungal; natural or unnatural peptides; proteins such as insulin, $\alpha 1$ - antitrypsin, $\alpha$ - chymotrypsin, albumin, interferons, antibodies; nucleic acids such as fragments of genes, DNA from natural sources or synthetic oligonucleotides and anti-sense nucleotides; sugars such as any mono-, di- or polysaccharides; and plasmids.

Nucleic acids may for example be capable of being expressed once introduced into a recipient. The nucleic acid may thus include appropriate regulatory control elements (e.g. promoters, enhancers, terminators etc) for controlling expression of the nucleic acid. The bioactive molecule may also be a chemically modified derivative of a natural or synthetic therapeutic agent such as a PEG-protein.

The nucleic acid may be comprised within a vector such as a plasmid, phagemid or virus vector. Any suitable vector known to a man skilled in the art may be used.

Vaccine coating components may, for example, include antigenic components of a disease causing agent, for example a bacterium or virus, such as diptheria toxoid and/or tetanus toxoid. A particular advantage of such vaccine formulations is that they generally show greatly enhanced stability on exposure to high temperature when compared with conventional liquid preparations. Such formulations prepared according to the present invention can, for example, be exposed to temperatures of greater than 45°C for 48 hours and retain their ability to illicit an immune response when tested in vivo, whereas standard liquid samples are generally found to be completely inactivated. Vaccines that exhibit high temperature stability do not need to be refrigerated and therefore provide considerable cost savings in terms of storage and ease of distribution particularly in developing countries. Vaccines are useful for the prevention and/or treatment of infections caused by pathogenic microorganisms, including viral, fungal, protozoal, amoebic and bacterial infections and the like. Examples of vaccine formulations that can be prepared according to the present invention include sub-unit, attenuated or inactivated organism vaccines including, but not limited to, diphtheria, tetanus, polio, pertussus and hepatitis A, B and C, HIV, rabies and influenza.

Exemplary formulations are comprised of diphtheria taxoid coated D,L-valine or L-glutamine crystals. The present inventors have found that samples of diphtheria taxoid coated L-glutamine crystals, for example, may be stored under a range of different conditions and following reconstitution and inoculation may be found to illicit strong primary and secondary immune response in mice. Vaccine coated crystals may be formulated for delivery to a recipient by a number of routes including parenteral, pulmonary and nasal administration. Pulmonary delivery may be particularly efficacious for very young children.

Particles according to the present invention are also applicable to administration of polysaccharides linked to proteins such as HiB (haemopholis influenza B) and pneumococcal vaccines and live virus vaccines, such as mumps, measles and rubella. Particles according to the present invention may also be prepared with modern flu vaccine components such as MV A vectored influenza vaccine.

In addition vaccine component coated micro-crystals may be useful for formulation of vaccines developed for cancers, especially human cancers, including melanomas; a skin cancer; lung cancer; breast cancer; colon cancer and other cancers. Pulmonary formulations as described herein may be particularly suited for treatment of lung cancer. It should be noted that in addition to protein based vaccines (i.e. protein/peptide components coated on an inner substantially non-hygroscopic crystalline core) nucleic acid based vaccine formulations may also be prepared according to the present invention, wherein nucleic acid molecules are coated on an inner substantially non-hygroscopic crystalline core.

Examples of non-hygroscopic coated particles which have been found to have advantageous properties include those with a crystalline core of D,L-valine and a coating of insulin; a crystalline core of L-glycine and a coating of antitrypsin, a crystalline core of Na glutamate and a coating of insulin; a crystalline core of L-methionine and a coating of insulin; a crystalline core of L-alanine and a coating of insulin; a crystalline core of L-valine and a coating of insulin; a crystalline core of L-histidine and a coating of insulin; a crystalline core of L-glycine and a coating of $\alpha$ - antitrypsin; a crystalline core of L-glutamine and a coating of albumin: a crystalline core of D,L-valine and a coating of oligonucleotides DQA-HEX; a crystalline core of D,L-valine and a coating of $\alpha$1-antitrypsin with a further anti-oxidant outer coating of N-acetyl cystein; a crystalline core of D,L-valine and a coating of ovalbumin; a crystalline core of L-glutamine and a coating of ovalbumin, a crystalline core of D,L-valine and a coating of diptheria taxoid; a crystalline core of L-glutamine and a coating of diptheria taxoid; a crystalline core of D,L-valine and a coating of diptheria taxoid; a crystalline core of the L-glutamine and a coating of tetanus taxoid; a crystalline core of the D,L-valine and a coating of a mixture of diptheria taxoid and tetanus taxoid; a crystalline core of L-glutamine and a coating of a mixture of diptheria taxoid and tetanus taxoid.

Typically a batch of particles formed under well controlled conditions is composed of individual microcrystals that all exhibit substantially the same morphology or crystal-shape and which have a narrow size distribution. This can be conveniently observed in SEM images and verified by particle size measurements. The microcrystals according to the present invention typically have a maximum cross-sectional dimension and largest dimension of less than 80 microns. Preferably they have a maximum cross-sectional dimension of less than 40 microns and more preferably less than 20 microns. Particles with a maximum cross-sectional dimension of between 0.5 and 20 micron are most preferred. Alternatively free-flowing powders of spherical aggregates of similar sized microcrystals may be formed with maximum cross-sectional dimension of less than 50 microns and preferably less than 20 microns. A notable aspect of the particles formed with preferred coprecipitants is that their size and morphology remain substantially constant on exposure to high humidities such as up to 80 % RH. In addition their free-flowing characteristics and aerodynamic properties may be retained on re-drying.

The amount of bioactive molecule coated onto each particle can be conveniently varied by changing the ratio of bioactive molecule to core molecule in the initial aqueous solution prior to coprecipitation. Typically the bioactive molecule will make up between 0.1 wt% and 50 wt% of each coated microcrystal. More preferably the loading of bioactive molecule in the particles will be between 1 wt% and 40 wt%.

Typically, at least some of the bioactive molecules retain a high level of activity even after exposure to high humidity.

Typically, the non-hygroscopic coated particles are stable (i.e. substantially retain their bio-activity) on exposure to elevated temperatures and may be stable at up to 60°C for more than 1 week. This aids the storage and shows pharmaceutical formulations formed from the non-hygroscopic coated particles may be expected to have extended shelf-lives even under non-refrigerated conditions.

Typically, the core material of the non-hygroscopic coated particles will absorb less than 5 wt% of water and preferably less than 0.5 wt% at relative humidities of up to 80%. Particles comprising biomolecules will typically absorb higher amounts of water with the wt% depending on the loading

Typically, the bioactive molecules coated on the crystalline core retain a native or near-native configuration i.e. the bioactive molecules are not irreversibly denatured during the production process. Coating of the bioactive molecules onto the crystalline core is also advantageously found to lead to enhanced stability on storage of the particles at ambient or elevated temperatures. For example, typically the bioactive molecule may retain most of its bioactivity when reconstituted in aqueous media. Preferably the bioactive molecule will retain greater than 50% of it's initial bioactivity after storage at 25 C for 6 months. More preferably the bioactive molecule will retain greater then 80% of its bioactivity and most preferably greater than 95% bioactivity.

The fine free-flowing particles or suspensions described typically do not adhere to the walls of a glass vial. The particles typically re-dissolve rapidly and completely in water, aqueous solutions (containing buffers and salts such as those commonly used for reconstitution) or else in physiological fluids. Full re-dissolution of a dry powder or suspension will generally take place in less than 2 minutes, preferably in less than 60 seconds and most preferably in less than 30 seconds. Formulations reconstituted in aqueous buffer are typically low turbidity, colourless solutions with clarity better than 15 FNU and preferably better than 6 FNU (FNU = Formazine nephelometric units).

Commonly bioactive molecules require excipients or stabilising agents to be present when dissolved in aqueous solution such as buffer compounds, salts, sugars, surfactants and antioxidants. These may be included in the starting aqueous solution and incorporated into the particles during the coprecipitation process. They will then be present on reconstitution of the particles for example as a pharmaceutical formulation. Typically following coprecipitation of all the components the excipients will be concentrated on the outer surface of the particle and will permeate into the coating of bioactive molecules. A typical antioxidant may, for example, be cysteine such as in the form of N-acetyl cysteine while a typical surfactant may be Tween. During coprecipitation it is possible for the relative ratio of excipients to bioactive molecule to change due to dissolution into the solvent. This may be controlled by pre-addition of selected excipients to either the initial aqueous solution, the coprecipitation solvent or the rinse solvent such that on drying the desired ratio is obtained in the particles. Thus, for example, organic soluble sugars or polymers may be coated onto the surface of protein coated particles by inclusion in the rinse solvent in order to provide enhanced storage stability. Alternatively additives may be included in the rinse solvent and coated onto the outer surface of the particles in order to improve the physical properties of the particles themselves. For example it is found to be advantageous to provide isoleucine coated insulin-glycine particles by rinsing the formed microcrystals with a solution of isoleucine in 2-propanol prior to drying. These particles have enhanced flow and aerodynamic properties relative to the uncoated ones.

According to a third aspect of the present invention there is provided a pharmaceutical formulation for pulmonary delivery comprising particles formed according to the first aspect or particles formed in a batch process.

In order to use inhalation to administer drug molecules into the bloodstream, the drug must be made into a formulation capable of being delivered to the deep lung. In the case of dry-powder, this generally requires particles with mass median dimensions in the range 1-5 microns, although it has been demonstrated that larger particles with special aerodynamic properties may be used. Certain formulations of particles according to the present invention are suitable for forming pulmonary formulations as they can be used to generate fine free-flowing particles well suited to delivery by inhalation. Given that the bioactive molecule is on the surface of these non-hygroscopic coated particles, the particles generally exhibit unexpectedly low static charge and are straight-forward to handle and use in a delivery device as a dry powder. Alternatively, for example, they can be used as a suspension in a nebulisor.

In particular, bioactive molecules suitable for the formation of pulmonary pharmaceutical formulations may include but are not restricted to any of the following: therapeutic proteins such as insulin, $\alpha$1-antitrypsin, interferons; antibodies and antibody fragments and derivatives; therapeutic peptides and hormones; synthetic and natural DNA including DNA based medicines; enzymes; vaccine components; antibiotics; pain-killers; water-soluble drugs; water-sensitive drugs; lipids and surfactants; polysaccharides; or any combination or derivatives thereof. The pulmonary formulation comprising particles may be used directly in an inhaler device to provide high emitted doses and high fine particle fractions. Thus emitted doses measured in a MSLI (stages 1-5) are typically greater than 70%. The fine particle fractions measured in a MSLI (stages 3-5) are typically greater than 20% and preferably greater than 30%. The fine particle fraction is defined as the fraction collected on the lower stages of a multi-stage liquid impinger (MSLI) and corresponds to particles with aerodynamic properties suitable for administration to the deep lung by inhalation i.e. less than about 3.3 microns. The pulmonary formulation may be used in a dry powder delivery device without any further formulation with, for example, larger carrier particles such as lactose.

For pulmonary formulations, particles with a mass median aerodynamic diameter less than 10 microns and more preferably less than 5 microns are preferred. These will typically have a mass median diameter similar to their mass median aerodynamic diameter. Typically free-flowing, non-hygroscopic low static particles with maximum cross-sectional diameters in the range of 1-5 microns are preferred. These can be obtained using amino-acids such as for example, L-glutamine to form the crystalline core. However, the inventors have surprisingly discovered that bioactive molecule coated particles that take the form of high aspect ratio flakes may advantageously have mass median aerodynamic diameters smaller then their maximum cross-sectional diameters. Suitable shapes may be, for example, leaf shaped or tile shaped. With such particles the preferred range of maximum cross-sectional diameters may be greater than 1-5 microns and may for example be 1-10 microns. Coprecipitants which typically form bioactive molecule coated crystalline particles of this shape include histidine, and D,L-valine. For dry powder pulmonary formulations, particles made with coprecipitants that produce high aspect ratio flakes are therefore also preferred..

In particular, pulmonary formulations may preferably be selected to have crystalline cores comprised of amino-acids such as valine, histidine, isoleucine, glycine or glutamine and which, for example, include: a crystalline core of valine and a coating of a therapeutic protein such as insulin; a crystalline core of histidine and a coating of an enzyme; a crystalline core of valine and a coating of an enzyme inhibitor such as $\alpha$ -antitrypsin; a crystalline core of valine and a coating of DNA; a crystalline core of valine and a vaccine coating; a crystalline core of glutamine and a vaccine coating; a crystalline core of glutamine and a coating of albumin. It is preferred when forming the particles for the formulation that co-precipitants are used which give discrete particles which do not aggregate on exposure to high humidity. In addition it is preferable that the coprecipitant does not leave an unpleasant taste in the patients mouth following administration. Glutamine is therefore highly preferred since it can be exposed to high humidity and has a bland taste.

According to a fourth aspect of the present invention there is provided a parenteral formulation comprising particles or suspensions of particles according to the second aspect or particles formed in a batch process. Such formulations may be delivered by a variety of methods including intravenous, subcutaneous or intra-muscular injection or else may be used in sustained or controlled release formulations. The particles may be advantageously produced in a cost effective process to provide sterile parenteral formulations that exhibit extended shelf-life at ambient temperatures.

Formulations in the form of powders or suspensions may be preferably reconstituted in aqueous solution in less than 60 seconds to provide low turbidity solutions suitable for injection. Reconstitution of suspensions may be preferred where the bioactive molecule is particularly toxic or potent and therefore difficult to manufacture or handle as a dry powder. Alternatively concentrated suspensions of particles in a solvent such as, for example, ethanol may be used for direct parenteral administration without reconstitution. This may provide advantages for bioactive molecules that require to be delivered at very high dosage forms to provide therapeutic effectiveness. Such bioactive molecules may include therapeutic antibodies and derivatives thereof. These may undergo aggregation on reconstitution or else may form highly viscous solutions that are difficult to administer. Concentrated suspensions of particles containing a high dosage of bioactive molecule may therefore be used to provide an alternative more convenient and therapeutically effective way of delivering such molecules. Bioactive molecule coated particles are particularly suited to this application because they reconstitute very rapidly and show minimal aggregation of the bioactive molecule. Administration of aggregates is undesirable because it may lead to initiation of an adverse immune response.

Bioactive molecules suitable for administration by parenteral delivery include those described in the third aspect of this invention. In addition parenteral administration can be used to deliver larger biomolecules such as vaccines or antibodies not suited to administration into the subject's blood-stream via the lung because of poor systemic bioavailability. Preferred crystalline core materials include excipients commonly used in parenteral formulations such as mannitol and sucrose. Also preferred are natural amino-acids such as L-glutamine that can be used to form particles that reconstitute rapidly, are stable even at high temperature and are easy to process and handle. L-glutamine is also preferred because it has been administered to patients at high dosages with no adverse side-effects.

According to a fifth aspect of the present invention there is provided a sustained or controlled release pharmaceutical formulation (or a depots) comprising particles or suspensions of particles according to the first aspect or in a batch process. For certain applications it is preferable to produce parenteral or pulmonary formulations or other formulations that on administration provide sustained or extended therapeutic effects. This may, for example, be used to limit the maximum concentration of bioactive molecule that is attained in the subject's bloodstream or else be used to extend the period required between repeat administrations. Alternatively it may be necessary to change the surface characteristic of the particles to improve their bioavailability. The bioactive molecule coated particles can be conveniently used to produce sustained or controlled release formulations. This can be achieved by coating the particles or incorporating them in another matrix material such as a gel or polymer or by immobilising them within a delivery device.

For example each of the particles may be evenly coated with a material which alters the release or delivery of the components of the particles using techniques known in the art.

Materials which may be used to coat the particles may, for example, be: poorly water-soluble biodegradable polymers such as, for example, polylactide or polyglycolide and copolymers thereof; polyamino-acids; hydrogels; and other materials known in the art that change their solubility or degree of cross-linking in response to exposure to physiological conditions. The coating may for example be applied by contacting a suspension of particles with a solution of the coating material and then drying the resulting particles. If required the process can be repeated to extend the release profile. The coated particles may be found to provide a substantially constant rate of release of the bioactive molecule into solution. Alternatively, a plurality of the particles may be combined into, for example, a single tablet form by, for example, by a binding agent. The binding agent may dissolve in solution whereupon the particles may be continually released into solution as the binding agent holding the tablet together progressively dissolves.

Those skilled in the art will realise that using combinations of the above teaching it is possible to provide other pharmaceutical formulations such as for example nasal formulations, oral formulations and topical formulations. Nasal formulations and oral formulations may require coating of the particles with alternate materials that provide adhesion to for example mucosal membranes.

According to a sixth aspect of the present invention there is provided a pulmonary drug delivery device comprising particles according to the second aspect or formed in a batch process.

The pulmonary drug delivery device may, for example, be a liquid nebulizer, aerosol-based metered dose inhaler or dry powder dispersion device.

Brief Description of the Drawings

Embodiments of the present invention will now be described, by way of example, with reference to the accompanying drawings in which:

Figure 1 is a representation of the particle size distribution for insulin/glycine precipitated in propan-2-ol;

Figure 2 is a representation of the particle size distribution for $\alpha$-chymotrypsin/L-alanine precipitated in propan-2-ol;

Figure 3 is a representation of the particle size distribution for $\alpha$-chymotrypsin/D,L-valine precipitated in propan-2-ol;

Figure 4 is a representation of the particle size distribution for D,L-valine precipitated in propan-2-ol;

Figure 5 is a representation of the particle size distribution for insulin/L-histidine precipitated in propan-2-ol;

Figure 6 is a representation of the particle size distribution for D,L-valine precipitated in propan-2-ol;

Figure 7 is a representation of the particle size distribution for L-glutamine precipitated in propan-2-ol;

Figure 8 is a representation of the particle size distribution for L-glutamine precipitated in propan-2-ol;

Figure 9 is a representation of the particle size distribution for albumin/L-glutamine precipitated in propan-2-ol;

Figure 10 is a Differential Vapour Sorption (DVS) graph of L-glutamine;

Figure 11 is a DVS graph of L- glycine;

Figure 12 is a DVS graph of L-glycine/insulin PCMCs;

Figure 13 is a DVS graph of D,L-valine/insulin PCMCs;

Figure 14 is a DVS graph of D,L-valine;

Figure 15 is a DVS graph of albumin/L-glutamine;

Figure 16 is a representation of a continuous flow precipitation apparatus;

Figure 17 shows the distribution of DQA-HEX and crude oligonucleotide/D,L-valine in an artificial lung;

Figure 18 is an image of diptheria toxoid (DT) PCMCs;

Figure 19 shows the bioactive response afforded by insulin/D,L-valine particles similar to that of USP insulin;

Figure 20 is a representation of wire myograph studies showing again bioactive response afforded by insulin/D,L-valine particles similar to that of USP insulin;

Figure 21 is an SEM image of insulin/D,L-valine PCMCs;

Figure 22 is an SEM image of insulin/D,L-valine PCMCs;

Figure 23 is an SEM image of albumin/L-glutamine PCMCs;

Figure 24 is an SEM image of insulin/L-histidine PCMCs; and

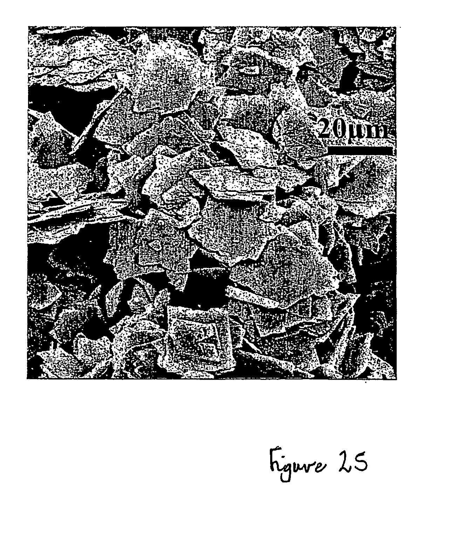

Figure 25 is an SEM image of $\alpha$-antitrypsin/D,L-valine PCMCs;

Figure 26 is an SEM image of tobramycin/D,L-valine crystals with a theoretical antibiotic loading of 9.1% w/w prepared by a batch process;

Figure 27 is an SEM image of tobramycin/D,L-valine crystals with a theoretical antibiotic loading of 1.6% w/w prepared by a continuous process;

Figure 28 is an SEM image of subtilisin/glutamine crystals with a theoretical protein loading of 0.7% w/w dried from solvent directly onto a SEM stub;

Figure 29 is an SEM image of subtilisin/glutamine crystals with a theoretical protein loading of 0.7% w/w dried in air following filtration on a Durapore 0.4 micron filter;

Figure 30 is an SEM image of subtilisin/glutamine crystals with a theoretical protein loading of 6.4% w/w dried from solvent directly onto a SEM stub;

Figure 31 is an SEM image of subtilisin/glutamine crystals with a theoretical protein loading of 6.4% w/w dried in air following filtration on a Durapore 0.4 micron filter;

Figure 32 is powder X-ray diffraction data collected for glutamine (bottom trace) and albumin/glutamine (top trace) at 10% theoretical protein loading precipitated in ethanol; and

Figure 33 is 2 ml Vials containing equal weights 50 mg of subtilisin coated D,L-valine microcrystals dried either by critical point drying (A) or filtered on a Durapore 0.4 micron filter and air-dried (B).

(It should be noted that although in the following examples the coated particles are referred to as PCMCs, the particles need not necessarily be coated with a protein and may have any bioactive coating)

## Example Section

## Example 1

Table 1 shows the conditions used to produce a range of protein coated microcrystals (PCMCs) wherein the bioactive material which forms a coating is insulin and the crystalline core is formed from D,L-valine, L-valine, L-histidine and L-glycine. The microcrystals were made according to the entry under Crystallisation Process in glass vials or flasks and mixing was carried out by magnetic stirring.

Insulin used is bovine pancreas insulin (Sigma I5500) and USP bovine insulin (Sigma I8405).

Crystals were isolated by filtering through Durapore membrane filters (0.4 microns) and were then dried in air in a fume hood.

Protein loadings were determined using Biorad Protein Assay. Percentage of Fine Particle Fraction (FPF) was determined using a multi-stage liquid impinger.

Table 1

| Bioactive Molecule | Bioactive Molecule dissolved in Solvent | Solvent/ H₂O% (v/v) | Conc. of Bioactive Molecule in Solvent (mg/ml) | Addition of excipient | Wash Step | Crystallisation Process | % protein recovered | % protein in crystal | % FPF |
|---|---|---|---|---|---|---|---|---|---|
| 80mg Insulin (I5500) | 8ml of 0.01M HCl and then 400μl of 1M NaOH added | Propan-2-ol 9.1% H₂O | 0.44 | 8ml of distilled water saturated with D,L-valine added to insulin giving a final pH of 8.6 and a 49% saturation of D, L-valine | None | 14ml of insulin in D,L-valine added dropwise to 140 ml of propan-2-ol with constant agitation at room temp | - | 18 | 40.0 |
| 10mg Insulin (I5500) | 1ml of 0.01M HCl and then 50μl of 1M NaOH added | Propan-2-ol 4.8% H₂O | 0.23 | 1ml of distilled water saturated with D,L-valine added to insulin giving a final pH of 8.68 and a 49% saturation of D,L-valine | None | 1.75ml of insulin in D,L-valine added dropwise to 35 ml of propan-2-ol with constant agitation at room temp | - | 14 | 32.1 |
| 20mg Insulin (I5500) | 2ml of 0.01M HCl and then 100μl of 1M NaOH added | Propan-1-ol 9.1% H₂O | 0.44 | 2ml of distilled water saturated with D,L-valine added to insulin giving a final pH of 8.61 and a 49% saturation of D,L-valine | None | 3.5ml of insulin in D,L-valine added dropwise to 35 ml of propan-1-ol with constant agitation at room temp | - | 33 | 32.0 |

| Bioactive Molecule | Bioactive Molecule dissolved in Solvent | Solvent/ H$_2$O% (v/v) | Conc. of Bioactive Molecule in Solvent (mg/ml) | Addition of excipient | Wash Step | Crystallisation Process | % protein recovered | % protein in crystal | % FPF |
|---|---|---|---|---|---|---|---|---|---|
| 20mg Insulin (I5500) | 2ml of 0.01MHCland then 100μl of 1M NaOH added | Ethanol 9.1% H$_2$O | 0.44 | 2ml of distilled water saturated with D,L-valine added to insulin giving a final pH of 8.65 and a 49% saturation of D,L-valine | None | 3.5ml of insulin in D,L-valine added dropwise to 35 ml of ethanol with constant agitation at room temp | - | 18 | 27.0 |
| 20mg Insulin (I5500) | 2ml of 0.01MHCland then 100μl of 1M NaOH added | Propan-2-ol 9.01% H$_2$O | 0.44 | 2ml of distilled water saturated with D,L-valine and 0.41ml of dry propan-2-ol added to insulin giving 44% saturation of D,L-valine (9.1% v/v propan-2-ol in the aqueous phase | Propan-2-ol (9.1% H$_2$O v/v) | 3.85ml of insulin in D,L-valine added dropwise to 35 ml of propan-2-ol with constant agitation at room temp | - | 20 | 31.0 |

EP 1 587 478 B1

13

(continued)

| Bioactive Molecule | Bioactive Molecule dissolved in Solvent | Solvent/ H$_2$O% (v/v) | Conc. of Bioactive Molecule in Solvent (mg/ml) | Addition of excipient | Wash Step | Crystallisation Process | % protein recovered | % protein in crystal | % FPF |
|---|---|---|---|---|---|---|---|---|---|
| 20mg Insulin (I5500) | 2ml of 0.01M HCl and then 100μl of 1M NaOH added | Propan-2-ol 9.01% H$_2$O | 0.44 | 2ml of distilled water saturated with D,L-valine and 0.82ml of dry propan-2-ol added to insulin giving 41% saturation of D,L-valine (17% v/v propan-2-ol in the aqueous phase | Propan-2-ol (8.9% H$_2$O v/v) | 4.2ml of insulin in D,L-valine added dropwise to 35 ml of propan-2-ol with constant agitation* at room temp | - | 23 | 49.7 |
| 20mg Insulin (USP) | 2ml of 0.01M HCl and then 100μl of 1M NaOH added | Propan-2-ol 9.1% H$_2$O | 0.44 | 2ml of distilled water saturated with L-valine added to insulin giving a final pH of 8.61 and a 49% saturation of L-valine | None | 3.5ml of insulin in L-valine added dropwise to 35 ml of propan-2-ol with constant agitation at room temp | - | 18 | 23.0 |

EP 1 587 478 B1

14

| Bioactive Molecule | Bioactive Molecule dissolved in Solvent | Solvent/ H$_2$O% (v/v) | Conc. of Bioactive Molecule in Solvent (mg/ml) | Addition of excipient | Wash Step | Crystallisation Process | % protein recovered | % protein in crystal | % FPF |
|---|---|---|---|---|---|---|---|---|---|
| 80mg Insulin (USP) | 8ml of 0.01MHCl and then 400μl of 1M NaOH added | Propan-2-ol 9.1 % H$_2$O | 0.44 | 8ml of distilled water saturated with L-histidine added to insulin giving a final pH of 8.5 and a 49% saturation of L-histidine | None | 14ml of insulin in L-histidine added dropwise to 140 ml of propan-2-ol with constant agitation at room temp | - | 27.6 | 30.2 |
| 10mg Insulin (I5500) | 1ml of 0.01MHCl and then 50μl of 1M NaOH added | Propan-2-ol 4.8% H$_2$O | 0.23 | 1ml of distilled water saturated with L-glycine added to insulin giving a final pH of 8.08 and a 49% saturation of L-glycine | Propan-2-ol saturated with isoleucine | 1.75ml of insulin in L-glycine added dropwise to 35 ml of propan-2-ol with constant agitation at room temp | - | 4.1 | 27.6 |

Table 1 demonstrates that insulin coated particles with free-flowing physical properties suitable for pharmaceutical formulations can be made with a range of different coprecipitants. The coprecipitations were all carried out at concentrations of excipient below 80 mg/ml except for the last entry. In the latter case a modified rinsing procedure was used to further coat the crystals with isoleucine. The consistently high fine particle fractions (FPF) and emitted dose (not shown) illustrate the free flowing nature of the particles and demonstrates that a significant proportion have an effective aerodynamic dimension below 3 microns. It is also clear from Table 1 that it is possible to change process conditions to alter the loading of insulin and the physical properties of the particles.

**Example 2**

Table 2 shows a range of further insulin coated PCMCs made as in Example 1 wherein the crystalline core is formed from L-glycine, L-alanine and L-arginine.

Insulin used is bovine pancreas insulin (Sigma I5500) and USP bovine insulin (Sigma I8405).

Table 2

| Bioactive Molecule | Bioactive Molecule dissolved in Solvent | Solvent/ H$_2$O% (v/v) | Conc. of Bioactive Molecule in Solvent (mg/ml) | Addition of excipient | Wash Step | Crystallisation Process | % protein recovered | % protein in crystal | % FPF |
|---|---|---|---|---|---|---|---|---|---|
| 20mg Insulin (I5500) | 2ml of 0.01MHCl and then 100µl of 1M NaOH added | Propan-2-ol 9.1% H$_2$O | 0.44 | 2ml of distilled water saturated with L-glycine added to insulin giving a final pH of 8.66 and a 49% saturation of L-glycine | None | 3.5ml of insulin in L-glycine added dropwise to 35 ml of propan-2-ol with constant agitation at room temp | - | 5.4 | 7.2 |
| 80mg Insulin (I5500) | 8ml of 0.01MHCl and then 400µl of 1M NaOH added | Propan-2-ol 9.1% H$_2$O | 0.44 | 8ml of distilled water saturated with L-alanine added to insulin giving a final pH of 8.26 and a 49% saturation of L-alanine | None | 14ml of insulin in L-alanine added dropwise to 140 ml of propan-2-ol with constant agitation at room temp | - | 7.0 | 10.5 |
| 20mg Insulin (USP) | 2ml of 0.01MHCl and then 100µl of 1M NaOH added | Propan-2-ol 9.1% H$_2$O | 0.44 | 2ml of distilled water saturated with L-arginine added to insulin giving a final pH>10 and a 49% saturation of L-arginine | None | 3.5ml of insulin in L-arginine added dropwise to 35 ml of propan-2-ol with constant agitation at room temp | - | 1.3 | 1.1 |

EP 1 587 478 B1

17

Table 2 shows that particles produced from coprecipitants with high solubilities have inferior properties in the MSLI. Particle size measurements described below also show the presence of large aggregates of individual crystals. Another point illustrated is that particles with high loadings of the bioactive molecule (insulin) cannot be obtained when such high solubility compounds are used at close to saturation. In order to produce particles useful for pharmaceutical formulations it is therefore preferable to use lower solubility coprecipitants and/or to amend the process described in WO 0069887 by using sub-saturated solutions

**Example 3**

Table 3 shows a range of insulin PCMCs with a crystalline core of D,L-valine. The water miscible solvent used is propan-2-ol. The microcrystals were made according to the method of Example 1.

Table 3

| Bioactive Molecule | Bioactive Molecule dissolved in Solvent | $H_2O$% (v/v) | Conc. of Bioactive Molecule in Solvent (mg/ml) | Addition of excipient | Wash Step | Crystallisation Process | % protein recovered | % max protein in crystal |
|---|---|---|---|---|---|---|---|---|
| 4mg Insulin (I5500) | 6.4ml of 0.01MHCl and then 320µl of 1M NaOH added | 9.1 | 0.028 | 6.4ml of distilled water saturated with D,L-valine added to insulin giving a final pH of 8.8 and a 49% saturation of D,L-valine | Dry propan-2-ol | 0.7ml of insulin in D,L-valine added dropwise (0.1ml/min) to 7ml of propan-2-ol with constant agitation at room temp | - | 1.3 |
| 4mg Insulin (I5500) | 3.2ml of 0.01MHCl and then 160µl of 1M NaOH added | 9.1 | 0.055 | 3.2ml of distilled water saturated with D,L-valine added to insulin giving a final pH of 8.8 and a 49% saturation of D,L-valine | Dry propan-2-ol | 0.7ml of insulin in D,L-valine added dropwise (0.1ml/min) to 7ml of propan-2-ol with constant agitation at room temp | - | 2.6 |

(continued)

|  | Bioactive Molecule | Bioactive Molecule dissolved in Solvent | H$_2$O% (v/v) | Conc. of Bioactive Molecule in Solvent (mg/ml) | Addition of excipient | Wash Step | Crystallisation Process | % protein recovered | % max protein in crystal |
|---|---|---|---|---|---|---|---|---|---|
|  | 4mg Insulin (I5500) | 1.6ml of 0.01MHCl and then 80μl of 1M NaOH added | 9.1 | 0.11 | 1.6ml of distilled water saturated with D,L-valine added to insulin giving a final pH of 8.8 and a 49% saturation of D,L-valine | Dry propan-2-ol | 0.7ml of insulin in D,L-valine added dropwise (0.1ml/min) to 7ml of propan-2-ol with constant agitation at room temp | - | 5.1 |
|  | 4mg Insulin (I5500) | 0.8ml of 0.01MHCl and then 40μl of 1M NaOH added | 9.1 | 0.22 | 0.8ml of distilled water saturated with D,L-valine added to insulin giving a final pH of 8.8 and a 49% saturation of D,L-valine | Dry propan-2-ol | 0.7ml of insulin in D,L-valine added dropwise (0.1ml/min) to 7ml of propan-2-ol with constant agitation at room temp | - | 9.5 |
|  | 4mg Insulin (I5500) | 0.4ml of 0.01MHCl and then 20μl of 1M NaOH added | 9.1 | 0.44 | 0.4ml of distilled water saturated with D,L-valine added to insulin giving a final pH of 8.8 and a 49% saturation of D,L-valine | Dry propan-2-ol | 0.7ml of insulin in D,L-valine added dropwise (0.1ml/min) to 7ml of propan-2-ol with constant agitation at room temp | - | 18 |

(continued)

| Bioactive Molecule | Bioactive Molecule dissolved in Solvent | H$_2$O% (v/v) | Conc. of Bioactive Molecule in Solvent (mg/ml) | Addition of excipient | Wash Step | Crystallisation Process | % protein recovered | % max protein in crystal |
|---|---|---|---|---|---|---|---|---|
| 6mg Insulin (I5500) | 0.4ml of 0.01MHCl and then 20μl of 1M NaOH added | 9.1 | 0.67 | 0.4ml of distilled water saturated with D,L-valine added to insulin giving a final pH of 8.8 and a 49% saturation of D,L-valine | Dry propan-2-ol | 0.7ml of insulin in D,L-valine added dropwise (0.1ml/min) to 7ml of propan-2-ol with constant agitation at room temp | - | 24 |

It is therefore straightforward to alter the percentage of protein within the particles in order to provide pharmaceutical formulations with different dosage strengths.

## Example 4

Table 4 shows a series of further insulin coated PCMCs with a crystalline core of D,L-valine. The microcrystals were made according to Example 1.

Table 4

| Bioactive Molecule | Bioactive Molecule dissolved in Solvent | H₂O% (v/v) | Conc. of Bioactive Molecule in Solvent (mg/ml) | Addition of excipient | Wash Step | Crystallisation Process | % protein recovered | % max protein in crystal |
|---|---|---|---|---|---|---|---|---|
| 4mg USP Insulin (18405) | 0.4ml of 0.01MHCl and then 20μl of 1M NaOH added | 9.1 | 0.44 | 0.4ml of distilled water saturated with D,L-valine added to insulin giving a final pH of 8.8 and a 49% saturation of D, L-valine | Dry propan-2-ol | 0.7ml of insulin in D, L-valine added dropwise (0.1ml/min) to 7ml of propan-2-ol with constant agitation at room temp | - | 17 |
| 8mg USP Insulin (18405) | 0.4ml of 0.01MHCl and then 20μl of 1M NaOH added | 9.1 | 0.89 | 0.4ml of distilled water saturated with D,L-valine added to insulin giving a final pH of 8.8 and a 49% saturation of D, L-valine | Dry propan-2-ol | 0.7ml of insulin in D, L-valine added dropwise (0.1ml/min) to 7ml of propan-2-ol with constant agitation at room temp | - | 29 |
| 4mg USP Insulin (18405) | 0.4ml of 0.01MHCl and then 20μl of 1M NaOH added | 9.1 | 0.44 | 0.4ml of distilled water saturated with D,L-valine added to insulin giving a final pH of 8.8 and a 49% saturation of D, L-valine | Dry propan-2-ol | 0.7ml of insulin in D, L-valine added dropwise (0.1ml/min) to 7ml of propan-2-ol with constant agitation at room temp | - | 17 |

| Bioactive Molecule | Bioactive Molecule dissolved in Solvent | H$_2$O% (v/v) | Conc. of Bioactive Molecule in Solvent (mg/ml) | Addition of excipient | Wash Step | Crystallisation Process | % protein recovered | % max protein in crystal |
|---|---|---|---|---|---|---|---|---|
| 8mg USP Insulin (18405) | 0.4ml of 0.01MHCl and then 20μl of 1M NaOH added | 9.1 | 0.89 | 0.4ml of distilled water saturated with D,L-valine added to insulin giving a final pH of 8.8 and a 49% saturation of D, L-vali.ne | Dry propan-2-ol | 0.7ml of insulin in D, L-valine added dropwise (0.1ml/min) to 7ml of propan-2-ol with constant agitation at room temp | - | 29 |
| 4mg USP Insulin (I8405) | 0.4ml of 0.01MHCl and then 20μl of 1M NaOH added | 9.1 | 0.44 | 0.4ml of distilled water saturated with D,L-valine added to insulin giving a final pH of 8.8 and a 49% saturation of D, L-valine | Dry propan-2-ol | 0.7ml of insulin in D, L-valine added dropwise (0.1ml/min) to 7ml of propan-2-ol with constant agitation at room temp | - | 16 |
| 8mg USP Insulin (I8405) | 0.4ml of 0.01MHCl and then 20μl of 1M NaOH added | 9.1 | 0.89 | 0.4ml of distilled water saturated with D,L-valine added to insulin giving a final pH of 8.8 and a 49% saturation of D, L-valine | Dry propan-2-ol | 0.7ml of insulin in D, L-valine added dropwise (0.1ml/min) to 7ml of propan-2-ol with constant agitation at room temp | - | 30 |

| Bioactive Molecule | Bioactive Molecule dissolved in Solvent | H₂O% (v/v) | Conc. of Bioactive Molecule in Solvent (mg/ml) | Addition of excipient | Wash Step | Crystallisation Process | % protein recovered | % max protein in crystal |
|---|---|---|---|---|---|---|---|---|
| 20mg USP Insulin (I8405) | 2ml of 0.01MHCl and then 100μl of 1M NaOH added | 9.1 | 0.44 | 2ml of distilled water saturated with D,L-valine added to insulin giving a final pH of 8.8 and a 49% saturation of D, L-valine | Dry propan-2-ol | 3.5ml of insulin in D, L-valine added dropwise to 35ml of propan-2-ol with constant agitation at room temp | - | 17 |
| 20mg USP Insulin (I8405) | 2ml of 0.01MHCl and then 100μl of 1M NaOH added | 9.1 | 0.44 | 2ml of distilled water saturated with D,L-valine added to insulin giving a final pH of 8.8 and a 49% saturation of D, L-valine | Dry propan-2-ol | 3.5ml of insulin in D, L-valine added dropwise to 35ml of propan-2-ol with constant agitation at room temp | - | 17 |
| 16mg USP Insulin (I8405) | 1.6ml of 0.01MHCl | 9.1 | 0.44 | 1.6ml of distilled water saturated with D,L-valine added to insulin giving a 49% saturation of D, L-valine | Dry propan-2-ol | 2.8ml of insulin in D, L-valine added dropwise to 28ml of propan-2-ol with constant agitation at room temp | | 17 |
| 12mg USP Insulin (I8405) | 1.2ml of 0.01MHCl and then 60μl of 1M NaOH added | 9.1 | 0.44 | 1.2ml of distilled water saturated with D,L-valine added to insulin giving a 49% saturation of D, L-valine | Dry propan-2-ol | 2.1ml of insulin in D, L-valine added dropwise to 21ml of propan-2-ol with constant agitation at room temp | | 17 |

EP 1 587 478 B1

(continued)

| Bioactive Molecule | Bioactive Molecule dissolved in Solvent | $H_2O\%$ (v/v) | Conc. of Bioactive Molecule in Solvent (mg/ml) | Addition of excipient | Wash Step | Crystallisation Process | % protein recovered | % max protein in crystal |
|---|---|---|---|---|---|---|---|---|
| 12mg USP Insulin (I8405) | 1.2ml of 0.01MHCl and then 60µl of 1M NaOH added | 9.1 | 0.44 | 1.2ml of distilled water saturated with D,L-valine added to insulin giving a 49% saturation of D, L-valine | Dry propan-2-ol | 2.1ml of insulin in D, L-valine added dropwise to 21ml of propan-2-ol with constant agitation at room temp | | 17 |
| 12mg USP Insulin (I8405) | 1.2ml of 0.01MHCl | 9.1 | 0.44 | 1.2ml of distilled water saturated with D,L-valine added to insulin giving a 49% saturation of D, L-valine | Dry propan-2-ol | 2.1ml of insulin in D, L-valine added dropwise to 21ml of propan-2-ol with constant agitation at room temp | | 17 |
| 12mg USP Insulin (I8405) | 1.2ml of 0.01MHCl | 9.1 | 0.44 | 1.2ml of distilled water saturated with D,L-valine added to insulin giving a 49% saturation of D, L-valine | Dry propan-2-ol | 2.1mlofinsulininD,L-valine added dropwise to 21ml of propan-2-ol with constant agitation at room temp | | 17 |
| 20mg USP Insulin | 2.0ml of 0.01MHCl and then | 9.1 | 0.44 | 2ml of distilled water saturated with D,L-valine added to insulin giving a | Dry propan-2-ol | 3.5ml of insulin in D, L-valine added dropwise to 35ml of propan-2-ol with | | 17 |
| (I8405) | 100µl of 1M NaOH added | | | 49% saturation of D,L-valine | | constant agitation at room temp | | |

| Bioactive Molecule | Bioactive Molecule dissolved in Solvent | H$_2$O% (v/v) | Conc. of Bioactive Molecule in Solvent (mg/ml) | Addition of excipient | Wash Step | Crystallisation Process | % protein recovered | % max protein in crystal |
|---|---|---|---|---|---|---|---|---|
| 17mg USP Insulin (I8405) | 1.7ml of 0.01MHCl and then 85μl of 1M NaOH added | 9.1 | 0.44 | 1.7ml of distilled water saturated with D,L-valine added to insulin giving a 49% saturation of D, L-valine | Dry propan-2-ol | 3.4ml of insulin in D, L-valine added dropwise to 34ml of propan-2-ol with constant agitation at room temp | | 17 |
| 17mg USP Insulin (18405) | 1.7ml of 0.01MHCl and then 85μl of 1M NaOH added | 9.1 | 0.44 | 1.7ml of distilled water saturated with D,L-valine added to insulin giving a 49% saturation of D, L-valine | Dry propan-2-ol | 3.4ml of insulin in D, L-valine added dropwise to 34ml of propan-2-ol with constant agitation at room temp | | 17 |

These results demonstrate that the particles can be produced reproducibly.

## Example 5

Particle Size Analysis

Laser diffraction particle size analysis was carried out on bioactive coated particles using a Mastersizer 2000. Briefly, enough PCMC was added to the sample holder of the Mastersizer 2000 containing 60ml of 2-propanol to ensure a laser obscuration of between 10 and 20%. Measurements were then taken using a previously set up Standard Operating Procedure.

d(0.1) ($\mu$m) = 10% of the particles are below this particle size
d(0.5) ($\mu$m) = 50% of the particles are above and below this particle size
d(0.9) ($\mu$m) = 90% of the particles are below this particle size.

$$\text{Span} = d(0.9) - d(0.1) / d(0.5)$$

Span gives a good indication of population homogeneity. Thus, span values below 5 are preferred and span values below 2 are particularly preferred.

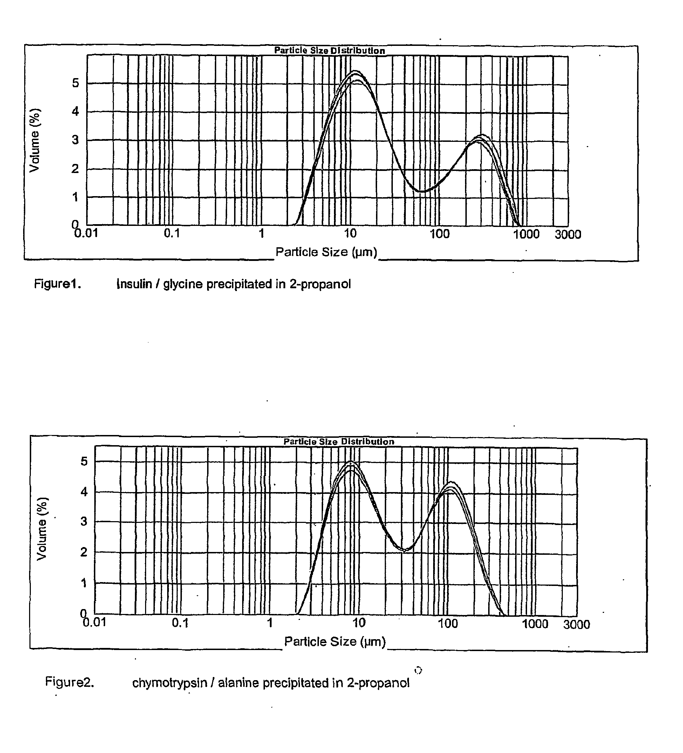

Typical size distribution patterns produced when saturated solutions of glycine and alanine are used as the core excipients are shown in Figures 1 and 2. Figure 1 shows the particle size distribution for insulin/glycine precipitated in propan-2-ol. Figure 2 shows $\alpha$-chymotrypsin/alanine precipitated in propan-2-ol.

Figures 1 and 2 demonstrate a large particle size distribution when saturated solutions or concentrated solutions of very soluble excipients (e.g. glycine and alanine) are used as the core material in the co-precipitation process carried out according to WO 0069887. In particular it can be seen that there are two populations one composed of the particles and the larger composed of agglomerates of the smaller particles. This is not desirable for the production of pharmaceutical formulations with homogeneous solubility and bioavailabilty properties.

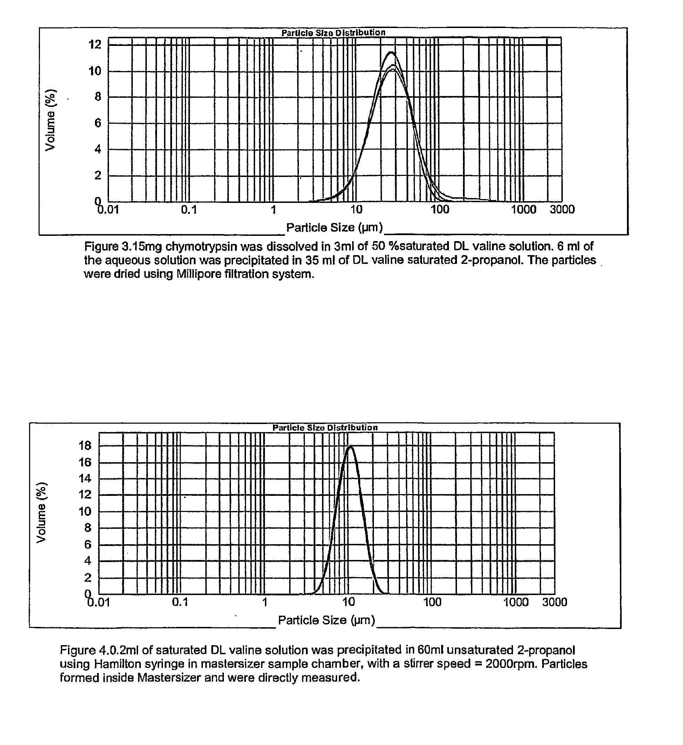

In contrast Figures 3-9 show a much narrower particle size distribution is obtained when less soluble excipients such as D,L-valine, L-glutamine and L-histidine make up the core of the particles. They also demonstrate that little or no large aggregates are formed. These particles may be expected to provide pharmaceutical formulations with homogeneous solubility and bio-availabilty properties.

Figure 3 represents PCMCs formed when 15mg chymotrypsin was dissolved in 3ml of 50% saturated DL-valine solution. 6 ml of the aqueous solution was precipitated in 35 ml of D,L-valine saturated 2-propanol. The particles were dried using Millipore filtration system.

Figure 4 represents PCMCs formed when 0.2ml of saturated D,L-valine solution was precipitated in 60ml unsaturated 2-propanol using a Hamilton syringe in a Mastersizer sample chamber, with a stirrer speed = 2000rpm. Particles were formed inside the Mastersizer and were directly measured. The narrower size distribution seen in this sample is thought to arise because a high agitation speed was used and because the particles have not been isolated in the form of a dry powder. Using conventional isolation techniques typically leads to more aggregated formulations.

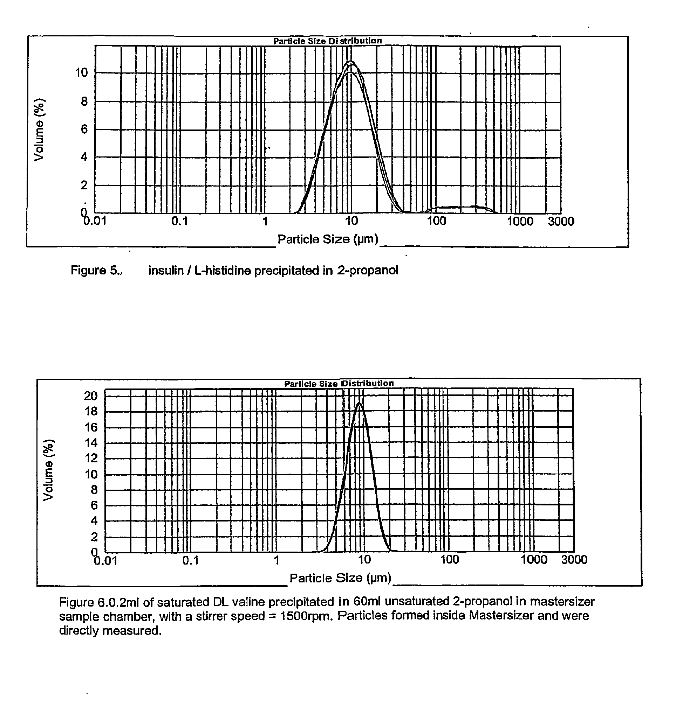

Figure 5 represents PCMCs formed when 14ml of saturated L-histidine is precipitated in 140ml L-histidine saturated 2-propanol using a magnetic stirrer. The particles were dried using Millipore filtration system.

Figure 6 represents PCMCs formed when 0.2ml of saturated D,L-valine is precipitated in 60ml unsaturated 2-propanol in Mastersizer sample chamber, with a stirrer speed = 1500rpm. Particles were formed inside Mastersizer and were directly measured.

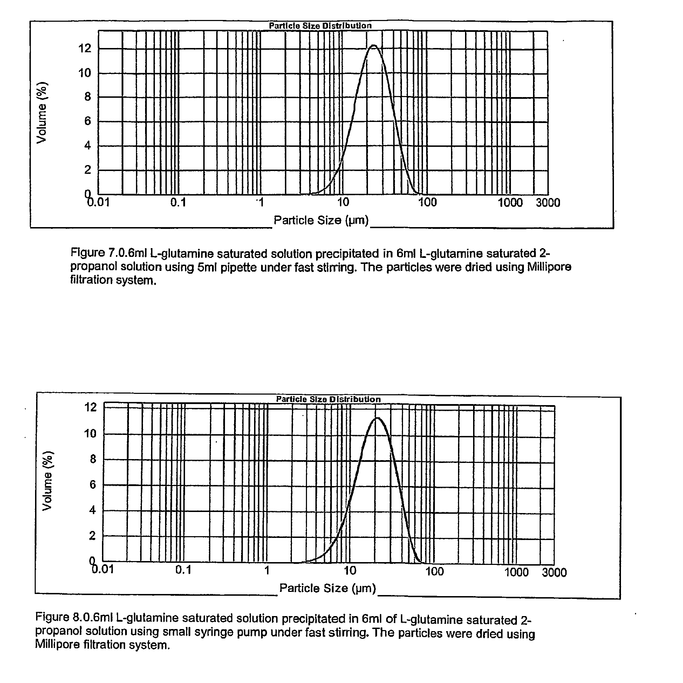

Figure 7 represents PCMCs formed when 0.6ml L-glutamine saturated solution is precipitated in 6ml L-glutamine saturated 2-propanol solution using 5ml pipette under fast stirring. The particles were dried using Millipore filtration system.

Figure 8 represents PCMCs formed when 0.6ml L-glutamine saturated solution is precipitated in 6ml of L-glutamine saturated 2-propanol solution using small syringe pump under fast stirring. The particles were dried using Millipore filtration system.

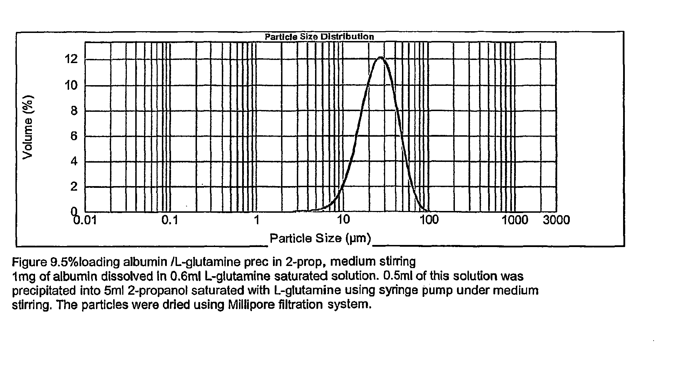

Figure 9 represents PCMCs formed when 5% loading albumin/L-glutamine was precipitated in propan-2-ol, medium stirring. 1mg of albumin was dissolved in 0.6ml L-glutamine saturated solution. 0.5ml of this solution was precipitated into 5ml 2-propanol saturated with L-glutamine using syringe pump under medium stirring. The particles were dried using Millipore filtration system.

Table 5 shown below summarises the results shown in Figures 1 to 9.

Table 5

| Formulation | d(0.1) μm (SD) | d(0.5) μm (SD) | D(0.9) μm (SD) | Span (SD) |
|---|---|---|---|---|
| Figure 1 | 5.719 (0.062) | 19.790 (0.557) | 317.870 (8.207) | 15.777 (0.146) |
| Figure 2 | 4.779 (0.092) | 17.995 (1.567) | 137.383 (9.808) | 7.720 (0.139) |
| Figure 3 | 10.823 (0.163) | 22.243 (0.343) | 42.241 (0.191) | 1.412 (0.012) |
| Figure 4 | 6.869 (0.097) | 10.662 (0.168) | 16.162 (0.268) | 0.871 (0.003) |
| Figure 5 | 4.917 (0.105) | 9.940 (0.147) | 21.156 (1.085) | 1.431 (0.228) |
| Figure 6 | 5.965 (0.076) | 9.002 (0.125) | 13.321 (0.197) | 0.815 (0.005) |
| Figure 7 | 11.914 (0.057) | 23.227 (0.144) | 42.006 (0.400) | 1.292 (0.002) |
| Figure 8 | 9.615 (0.160) | 20.046 (0.245) | 37.665 (0.462) | 1.399 (0.001) |
| Figure 9 | 13.485 (0.190) | 26.281 (0.317) | 48.044 (0.567) | 1.314 (0.003) |
| d(0.1), d(0.5), d(0.9) and span mean values and standard deviation (n=3). | | | | |

The results in Table 5 show that formulations with a relatively narrow size distributions and which exhibit minimal aggregation can be reproducibly obtained by selecting preferred coprecipitants. It can also be seen that the volume median diameters of these particles as determined by the mastersizer is typically less than 30 microns and may be less than 10 microns. SEM images of the particles typically demonstrate that the mean maximum cross-sectional dimensions is qualitatively lower than the mean mass dimension measured by the Mastersizer.

Microcrystals and bioactive molecule coated microcrystals produced by a continuous process typically exhibit a narrow size distribution with a Span less than 5, preferably less than 2 and more preferably less than 1.5. Bioactive molecule coated microcrystals produced by coprecipitation are typically advantageously smaller than microcrystals produced by precipitation of the pure carrier material. This is consistent with coating of the bioactive molecule on the microcrystal surface.

Cytochrome c coated microcrystals of D,L-valine (Cytc/val), glycine (Cytc/gly)and L-glutamine (Cytc/gln) all with a protein loading of 10% were prepared by coprecipitation into isopropanol using the continuous flow precipitator described in example 9. Table Size distribution, shows the average size and span obtained

Table Size distribution

| sample | d(0.5) /microns | Span |
|---|---|---|
| D,L-valine | 21.810 | 1.32 |
| Cytc/val | 12.65 | 1.22 |
| glyine | 58.370 | 1.72 |
| Cytc/gly | 31.949 | 2.07 |
| L-glutamine | 36.373 | 1.88 |
| Cytc/gln | 20.355 | 1.71 |

These results clearly show the reduction in size of bioactive molecule coated microcrystal relative to bare microcrystals.

The measured span is in each case less than 5 and may be less than 1.5. Further reductions in the size of particles may be achieved by changing process conditions such as temperature or by increasing the mixing efficiency.

### Example 6

### Dose Emissions from Dry Powder Inhalers

Dose emissions from dry powder inhalers were determined using an Astra Draco Multi-Stage Liquid Impinger (MSLI). A useful part of the dose is called the Fine Particle Fraction (FPF). The Fine Particle Fraction (FPF) is generally collected on the lower Stages of the MSLI as shown in Table 6 below. Table 6 was used to work out the cut-off dimension of the important Stages.

Table 6

| Stage | Cut-off dimension ($\mu$m) | Flow rate (1 min$^{-1}$) |
|---|---|---|
| Stage 4 | $ECD_4 = 1.7 \, (Q/60)^{1/2}$ | $30 \leq Q \leq 100$ |
| Stage 3 | $ECD_3 = 3.1 \, (Q/60)^{1/2}$ | $30 \leq Q \leq 100$ |
| Stage 2 | $ECD_2 = 6.8 \, (Q/60)^{1/2}$ | $30 \leq Q \leq 100$ |

In the following experiments a flow rate (Q) of 60 1 min $^{-1}$ was used, giving the following cut-off dimensions of Stages 2, 3 & 4 of 6.8, 3.1 and 1.7$\mu$m, respectively.

The following procedure was used in all MSLI experiments:

(a) for initial work on commercially available salbutamol sulphate formulations (e.g. Ventolin) the formulations were used as received.

(b) for PCMC formulations Size 3 capsules were filled with an amount of dry powder PCMC commonly between 10-20mg.

(c) a filter paper was added to Stage 5 of the MSLI prior to clamping of Stages 1 to 4. To each of Stages 1 to 4 was added 20ml of water. After attaching the neck section to the top of Stage 1, the adaptor piece was attached to the end of the neck. Use of the dry powder inhaler was initiated by piercing holes in either the blister pack in the case of the diskhaler or Size 3 capsules in the case of the aerohaler. The dry powder inhaler was subsequently housed in the adaptor and the pump was switched on for 4 seconds to deliver the formulation from the inhaler to the MSLI. An actuation was carried out for each blister or capsule inside the inhaler.

In every case, PCMC formulation dose emissions were delivered to the MSLI using the aerohaler.

After delivery of the formulation to the MSLI sample collection was carried out as follows:

(a) the device was removed from the adaptor and the capsules removed and placed in a petri dish followed by the addition of 20ml of water.

(b) the adaptor was removed from the neck of the MSLI and placed in a petri dish followed by the addition of 10ml of water.

(c) the neck was removed from the MSLI and rinsed out with 20ml water into a petri dish.

(d) Stages 1 to 4 were unclamped from the filter stage and the opening of Stage 1 was rinsed with 20ml of water. This was followed by agitation to dissolve all powder.

(e) the filter was removed from the MSLI and placed in a petri dish followed by the addition of 10ml of water.

(f) 5 ml aliquots were removed from each Stage and assayed by HPLC to determine salbutamol sulphate concentration. A Bio Rad Protein microassay was used to determine PCMC protein concentration.

### Initial Work using Salbutamol Sulphate Formulations

Results of Salbutamol sulphate emissions from the Diskhaler (Tables 7 and 8) and the Aerohaler (Inhalator) (Tables 9 and 10) are shown below.

Table 7- Diskhaler

| Stage | % recovered of total emitted dose |
|---|---|
| Device and blister pack | 12.6 |
| Neck and adaptor | 14.3 |
| Stage 1 | 41.9 |
| Stage 2 | 6.9 |
| Stage 3 | 7.5 |
| Stage 4 | 9.1 |
| Stage 5 | 7.9 |
| FPF = 25%<br>Total drug amount recovered of dose claim 98% | |

Table 8 - Diskhaler

| Stage | % recovered of total emitted dose |
|---|---|
| Device and blister pack | 12.9 |
| Neck and adaptor | 17.1 |
| Stage 1 | 37.8 |
| Stage 2 | 6.7 |
| Stage 3 | 8.3 |
| Stage 4 | 9.4 |
| Stage 5 | 7.8 |
| Fine Particle Fraction (Stages 3,4 & 5) = 26%<br>Total drug amount recovered of dose claim 92% | |

Table 9 - Aerohaler

| Stage | % recovered of total emitted dose |
|---|---|
| Device and blister pack | 11.3 |
| Neck and adaptor | 25.2 |
| Stage 1 | 33.4 |
| Stage 2 | 7.2 |
| Stage 3 | 8.7 |
| Stage 4 | 8.3 |
| Stage 5 | 5.9 |
| Fine Particle Fraction (Stages 3,4 & 5) = 23%<br>Total drug amount recovered of dose claim 92% | |

Table 10 - Aerohaler

| Stage | % recovered of total emitted dose |
|---|---|
| Device and blister pack | 11.0 |
| Neck and adaptor | 24.1 |

(continued)

| Stage | % recovered of total emitted dose |
|---|---|
| Stage 1 | 33.1 |
| Stage 2 | 9.0 |
| Stage 3 | 8.5 |
| Stage 4 | 8.6 |
| Stage 5 | 5.7 |
| Fine Particle Fraction (Stages 3,4 & 5) = 23% | |

The Ventolin Diskahler provided a Fine Particle Fraction (FPF) of almost 26% in the MSLI. About 70% of the dose from the ventolin diskhaler was delivered to the impactor. The Inhalator (Atrovent) provided a Fine Particle Fraction (FPF) of about 28% in the MSLI.

These values correspond to those reported in the literature for such formulations and devices and demonstrate that the MSLI was calibrated and operating correctly.

**PCMC Dose Emissions in the MSLI**

CHYMOTRYPSIN FORMULATIONS

Chymotrypsin PCMCs were produced using the following technique:

Chymotrypsin was dissolved in saturated amino acid solutions to give an aqueous solution with a concentration of 10mg/ml. The aqueous solution was precipitated in a volume of 2-propanol pre-saturated with an appropriate amino acid (e.g. L-glycine, L-alanine, D,L-valine, DL-serine, L-leucine and DL-isoleucine) 15 times that of the aqueous solution.

Table 11 - Chymotrypsin/L-glycine

| Stage | % recovered of total emitted dose |
|---|---|
| Stage 1 | 54.4 |
| Stage 2 | 5.6 |
| Stage 3 | 1.5 |
| Stage 4 | 2.5 |
| Stage 5 | 0.9 |
| Neck | 10.4 |
| Adaptor | 4.8 |
| device and capsules | 19.8 |
| FPF= 5.0% | |

Table 12 - Chymotrypsin/L-alanine

| Stage | % recovered of total emitted dose |
|---|---|
| Stage 1 | 47.6 |
| Stage 2 | 7.8 |
| Stage 3 | 5.4 |
| Stage 4 | 1.5 |
| Stage 5 | 1.4 |
| Neck | 2.7 |

(continued)

| Stage | % recovered of total emitted dose |
|---|---|
| Adaptor | 0.7 |
| device and capsules | 32.8 |
| FPF = 8.4% | |

Table 13 - Chymotrypsin/D,L-valine

| Stage | % recovered of total emitted dose |
|---|---|
| Stage 1 | 37.5 |
| Stage 2 | 13.4 |
| Stage 3 | 11.4 |
| Stage 4 | 4.5 |
| Stage 5 | 6.2 |
| Neck | 15.5 |
| Adaptor | 3.3 |
| device and capsules | 8.2 |
| FPF = 22.1% | |

Table 14 - chymotrypsin / DL-serine

| Stage | % recovered of total emitted dose |
|---|---|
| Stage 1 | 63.0 |
| Stage 2 | 6.4 |
| Stage 3 | 6.8 |
| Stage 4 | 6.9 |
| Stage 5 | 1.7 |
| Neck | 5.3 |
| Adaptor | 2.8 |
| device and capsules | 6.9 |
| FPF = 15.4% | |

Table 15 - Chymotrypsin / L-Leucine

| Stage | % recovered of total emitted dose |
|---|---|
| Stage 1 | 73.3 |
| Stage 2 | 9.6 |
| Stage 3 | 0.4 |
| Stage 4 | 0.7 |
| Stage 5 | 0.3 |
| Neck | 7.9 |
| Adaptor | 3.5 |

(continued)

| Stage | % recovered of total emitted dose |
|---|---|
| device and capsules | 2.4 |
| FPF = 1.4% | |

Table 16 - Chymotrypsin / DL-isoleucine

| Stage | % recovered of total emitted dose |
|---|---|
| Stage 1 | 47.4 |
| Stage 2 | 11.3 |
| Stage 3 | 9.8 |
| Stage 4 | 5.7 |
| Stage 5 | 1.1 |
| Neck | 14.7 |
| Adaptor | 4.9 |
| device and capsules | 5.2 |
| FPF = 16.6% | |

These results demonstrate that higher fine-particle fractions tend to be obtained using crystalline core materials with an aqueous solubility at 25 centigrade in the range 20 mg/ml to 80 mg/ml. Leucine shows a much lower fine particle fraction but nevertheless produces a relatively high emitted dose. The high emitted dose is an indication of the free flowing nature of this and the other preferred amino-acids.

INSULIN FORMULATIONS

Insulin PCMCs were then prepared in a similar fashion to the chymotrypsin PCMCs.

Table 17 - insulin/L-glycine

| Stage | % recovered of total emitted dose |
|---|---|
| Stage 1 | 64.2 |
| Stage 2 | 2.4 |
| Stage 3 | 4.3 |
| Stage 4 | 2.6 |
| Stage 5 | 0.3 |
| Neck | 6.6 |
| Adaptor | 0.8 |
| device and capsules | 18.7 |
| FPF = 7.2% | |

Table 18 - insulin/L-alanine

| Stage | % recovered of total emitted dose |
|---|---|
| Stage 1 | 66.8 |
| Stage 2 | 7.7 |

(continued)

| Stage | % recovered of total emitted dose |
|---|---|
| Stage 3 | 7.5 |
| Stage 4 | 2.4 |
| Stage 5 | 0.6 |
| Neck | 5.0 |
| Adaptor | 3.2 |
| device and capsules | 7.1 |
| FPF = 10.5% | |

Table 19 - insulin/D,L-valine

| Stage | % recovered of total emitted dose |
|---|---|
| Stage 1 | 29.5 |
| Stage 2 | 11.7 |
| Stage 3 | 20.0 |
| Stage 4 | 14.2 |
| Stage 5 | 5.8 |
| Neck | 8.6 |
| Adaptor | 3.4 |
| device and capsules | 6.9 |
| FPF = 40.0% | |

Table 20 - insulin/Na-glutamate

| Stage | % recovered of total emitted dose |
|---|---|
| Stage 1 | 30.3 |
| Stage 2 | 10.5 |
| Stage 3 | 15.2 |
| Stage 4 | 10.5 |
| Stage 5 | 4.9 |
| Neck | 15.2 |
| Adaptor | 4.4 |
| device and capsules | 9.0 |
| FPF = 30.6% | |

Table 21 - insulin/L-arginine

| Stage | % recovered of total emitted dose |
|---|---|
| Stage 1 | 53.9 |
| Stage 2 | 28.1 |
| Stage 3 | 0.5 |

(continued)

| Stage | % recovered of total emitted dose |
|---|---|
| Stage 4 | 0.2 |
| Stage 5 | 0.4 |
| Neck | 13.9 |
| Adaptor | 1.3 |
| device and capsules | 1.9 |
| FPF = 1.1% | |

Table 22 - insulin/L-val

| Stage | % recovered of total emitted dose |
|---|---|
| Stage 1 | 48.3 |
| Stage 2 | 11.6 |
| Stage 3 | 10.4 |
| Stage 4 | 9.6 |
| Stage 5 | 3.0 |
| Neck | 11.9 |
| Adaptor | 1.6 |
| device and capsules | 3.6 |
| FPF = 23.0% | |

Table 23 - insulin/L-histidine

| Stage | % recovered of total emitted dose |
|---|---|
| Stage 1 | 26.6 |
| Stage 2 | 19.0 |
| Stage 3 | 20.6 |
| Stage 4 | 5.6 |
| Stage 5 | 4.0 |
| Neck | 7.8 |
| Adaptor | 5.5 |
| device and capsules | 11.0 |
| FPF = 8.4% | |

These results also demonstrate that higher fine-particle fractions and free flowing powders tend to be obtained using crystalline core materials with an aqueous solubility at 25 centigrade in the range 20 mg/ml to 80 mg/ml. Na glutamate shows a higher fine particle fraction than expected but this is thought to arise from poor coating of the protein onto the particles resulting in the formation of separate protein particles. This is substantiated by the poorer emitted dose for this formulation due to aggregate formation.

**ALBUMIN FORMULATIONS**

75mg albumin was dissolved in a 15ml saturated solution of L-glutamine and dispensed by a syringe pump into 150ml 2-propanol in a dissolution vessel at 500 rpm.

Table 24 - insulin/L-glutamine

| Stage | % recovered of total emitted dose |
|---|---|
| Stage 1 | 46.0 |
| Stage 2 | 8.3 |
| Stage 3 | 12.8 |
| Stage 4 | 12.5 |
| Stage 5 | 3.8 |
| Neck | 7.1 |
| Adaptor | 2.9 |
| device and capsules | 6.6 |
| FPF = 29.1%. | |

Together these results back up the suggestion from the Mastersizer experiments that using concentrated solutions of very soluble excipients for the core material (e.g. glycine, alanine, arginine) results in bioactive molecule coated particles that are unsuitable for pharmaceutical formulations and in particular pulmonary drug delivery due to aggregation. It can be seen on the other hand that particles made with less soluble amino acids (e.g. histidine, glutamine and valine) produce free flowing powders. These may be used to provide formulations suited for pulmonary drug delivery. It is further anticipated that improvements to the production process may be used to provide particles with even higher fine particle fractions.

## Example 7

## Controlled Release Experiments

Poly-Lactic acid (PLA) coated albumin/L-glutamine PCMCs were used in controlled release experiments.

The following method was carried out to coat albumin/L-glutamine PCMCs with PLA. The albumin/L-glutamine PCMCs were prepared by dissolving 31mg of albumin in 6.2ml of 50% saturated L-glutamine solution. The aqueous solution was then precipitated in 40ml of L-glutamine saturated 2-propanol. The particles were dried using Millipore filtration system. The albumin/L-glutamine PCMCs were coated as follows:

Expt A: 20mg albumin/L-glutamine PCMCs were suspended in 2ml acetone/PLA solution (50mg/ml) followed by evaporation of acetone. The resultant formulation formed a very thick PLA solution that upon complete drying formed a very sticky, brittle precipitate.

Expt B: 20mg albumin/L-glutamine PCMCs were suspended in 2ml acetone/PLA solution (50mg/ml) and precipitated in 20ml 2-propanol under vigorous stirring. The resultant formulation formed a large insoluble pellet.

Expt C: 10mg albumin/L-glutamine PCMCs were suspended in 10ml 2-propanol followed by the addition of 0.4ml acetone/PLA solution (50mg/ml) under vigorous stirring.

Protein release studies were performed on the dried coated PCMCs as follows:

The coated PCMCs were added to 15ml of $H_2O$ and agitated. At defined time intervals 0.8ml aliquots of the aqueous solutions were added to 0.2ml of Bio Rad Protein microassay and assayed by UV at 595nm to determine the amount of protein released. The protein release from an uncoated PCMC control was also determined. The results of this study are shown in Table 25 below.

Table 25

| Time (min) | % protein released | | | |
|---|---|---|---|---|
| | uncoated PCMC | coated PCMC C | coated PCMC A | coated PCMC B |
| 1 | 100 | 13.0 | 3.1 | 0.4 |
| 40 | 100 | 27.2 | 11.9 | 2.8 |

(continued)

| Time (min) | % protein released | | | |
|---|---|---|---|---|
| | uncoated PCMC | coated PCMC C | coated PCMC A | coated PCMC B |
| 90 | 100 | 44.2 | 14.1 | 5.5 |
| 180 | 100 | 57.7 | 20.1 | 10.6 |
| 270 | 100 | 69.6 | 23.9 | 14.0 |
| 360 | 100 | 68.9 | 25.4 | 15.6 |

From Table 25 it is clear that the PLA coating afforded a sustained release profile compared to the uncoated PCMCs which were released into the aqueous solution within 1 min. By altering the coating it is also possible to modify release of the protein. It is therefore possible to customise the release of a protein from a PCMC for a specific use.

## Example 8

### Dynamic Vapour Sorption (DVS)

The uptake of water by bioactive molecule coated particles produced by the present co-precipitation process and of the core material precipitated alone under a controlled humidified environment was carried out by Dynamic Vapour Sorption (DVS) using Dynamic Vapour Sorption 1000 (Surface Measurement Systems).

The Experimental set-up was as follows.

The DVS used a 2 full-cycle experimental Special Automatic Operation (SAO) protocol that included an initial drying stage at 0% Relative Humidity (RH). This was followed by a sorption stage where the RH in each stage had an incremental increase of 10% up to 90% RH and then a final jump to 95% RH. This was proceeded by an identical desorption cycle down to 0% RH. This cycle was repeated. The following criteria was used to control the DVS stage change: either the rate of change of the increase in mass i.e. dm/dt dropped to 0.002, or the maximum stage time was 2000 minutes.

Prior to introduction of the sample, the balance was tared and the instrument was allowed to equilibrate until a stable baseline was observed. The particles were then loaded and the initial weight recorded, followed by switching on the SAO. The experiment ran until the completion of the SAO.

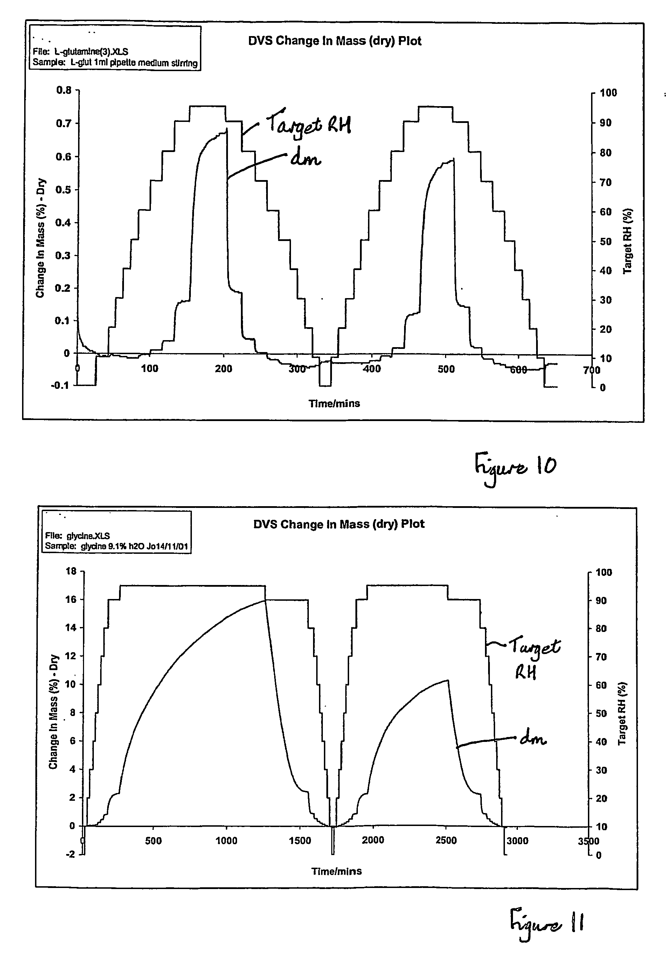

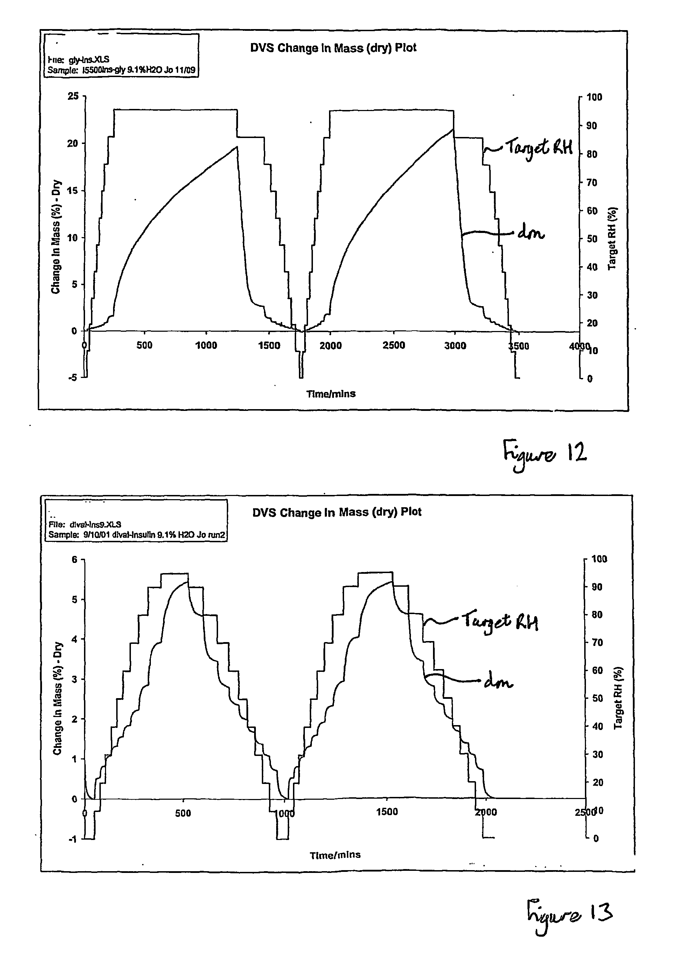

Figures 10 to 14 are DVS graphs of L-glutamine; L-glycine; L-glycine/insulin PCMCs; D,L-valine/insulin PCMCs; and D,L-valine, respectively.

Figures 10 to 14 show that the core coprecipitants exhibit very low hygroscopicity at relative humidities up to 80%. Above 80% RH more soluble coprecipitants like L-glycine (Figure 11) start to take up appreciable amounts of water. It is found that the coating of protein on the surface of the core material results in a formulation that takes up more water than the core material alone. This is expected because the protein is coated on the outside of the crystals. Importantly the samples typically exhibit minimal changes to their vapour sorption isotherm after passing through a complete cycle. i.e. the second sorption cycle is generally very similar to the first. Those skilled in the art will recognise that this illustrates that the particles do not undergo significant water vapour induced changes such as glass to crystalline transitions. The particles are therefore expected to be stable to storage at high humidity.

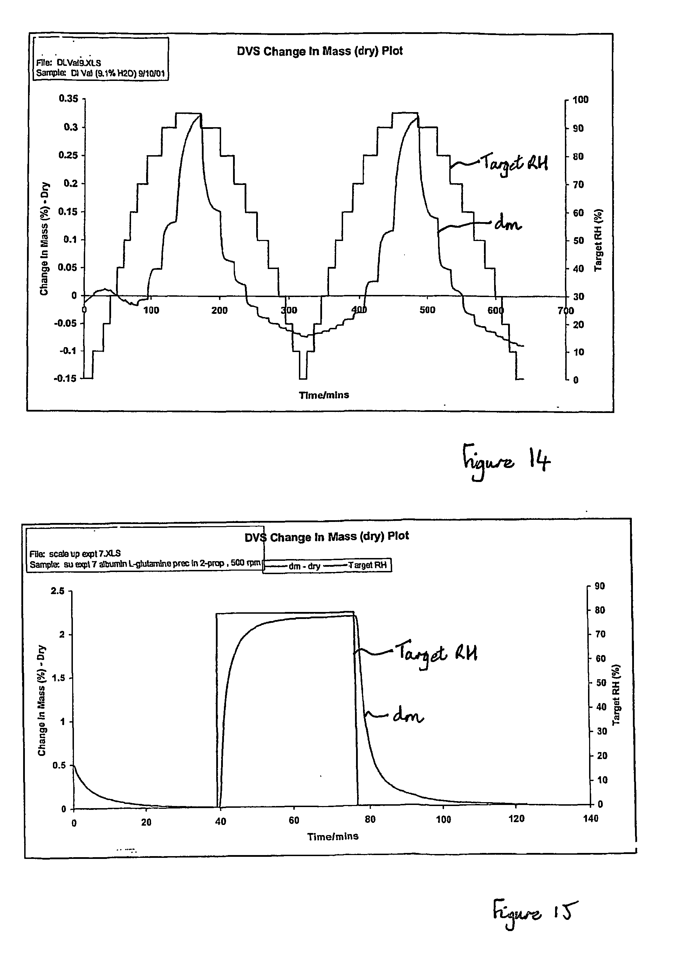

In another experiment a single cycle SAO (SAO2) was used that ramped the relative humidity from 0% to 80% after an initial drying phase, followed by an identical desorption stage. This is shown in Figure 15. The sample was collected and ran in the MSLI following the procedure previously described (MSLI section).

75mg albumin was dissolved in a 15ml saturated solution of L-glutamine and dispensed by a syringe pump into 150ml 2-propanol in a dissolution vessel at 500 rpm. 10mg of the dry powder formulation was ran in the MSLI before and after hydration in the DVS using SAO2.

Table 26 shows before incubation in the DVS

Table 26

| Stage | % recovered of total emitted dose |
|---|---|
| Stage 1 | 46.0 |
| Stage 2 | 8.3 |
| Stage 3 | 12.8 |

(continued)

| Stage | % recovered of total emitted dose |
|---|---|
| Stage 4 | 12.5 |
| Stage 5 | 3.8 |
| Neck | 7.1 |
| Adaptor | 2.9 |
| device and capsules | 6.6 |
| FPF = 29.1%. | |

Table 27 shows after incubation in the DVS

Table 27

| Stage | % recovered of total emitted dose |
|---|---|
| Stage 1 | 48.0 |
| Stage 2 | 8.8 |
| Stage 3 | 13.5 |
| Stage 4 | 14.9 |
| Stage 5 | 3.5 |
| Neck | 7.8 |
| Adaptor | 1.9 |
| device and capsules | 1.4 |
| FPF = 31.9% | |

The results shown in Tables 26 and 27 demonstrate that the free flowing nature, fine particle fraction and degree of aggregation of the particles is substantially unaffected by incubation at 80% RH in the DVS. This has important benefits for the production of pharmaceutical formulations and in particular pulmonary formulations since exposure to a humid atmosphere may occur in a delivery device.

Furthermore, consistent with the retention of aerodynamic properties, SEM images of bioactive molecule coated microcrystals equilibrated to high humidities show that the particles retain substantially the same shape and size as those stored under dry conditions.

**Example 9**

**Production of PCMCs in a Flow Precipitator**

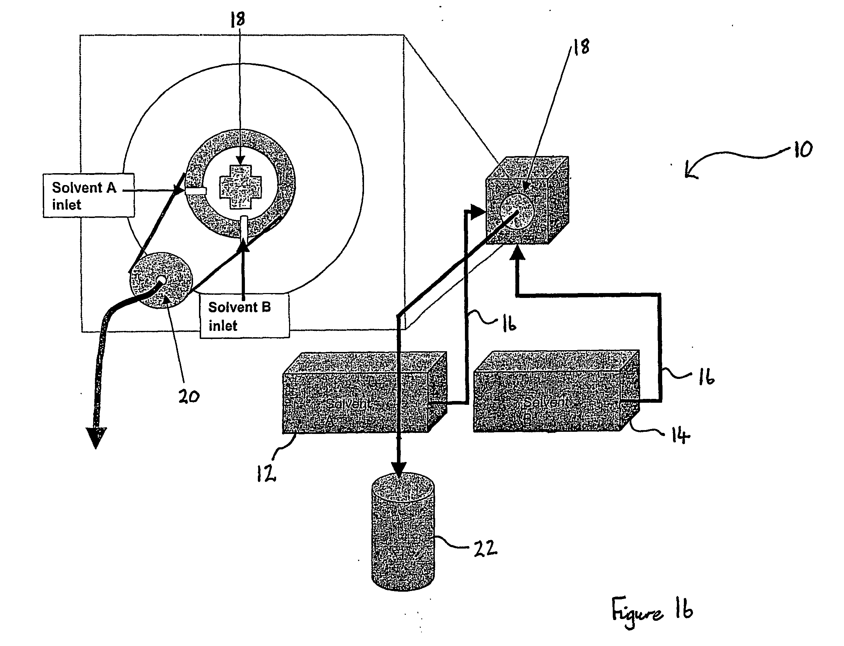

Figure 16 is a representation of a continuous flow precipitation apparatus, generally designated 10. The flow precipitation apparatus 10 comprises a source of solvent A 12 (e.g. aqueous solution containing the concentrated co-precipitant and bioactive molecules) and solvent B 14 (e.g. co-precipitant saturated solvent phase). The solvents 12, 14 are pumped by pumps (not shown) along biocompatible tubing 16 to a mixing device 18. A cross-section of the mixing device 18 is also shown which shows the solvents 12, 14 entering the mixing device 18 and an exit port and discharge pipe 20. A suspension collection vessel 22 is used to collect the formed PCMCs.

One pump continuously delivers the aqueous solution containing the concentrated coprecipitant and bioactive molecule while the other pump delivers the coprecipitant saturated solvent phase. Further pumps may be used if a third component such as a particle coating material is required.

The pumps can be of many different kinds but must accurately deliver the solutions at a defined flow rate and be compatible with the bioactive molecules employed. Conveniently, HPLC pumps can be used since these are optimised for delivering aqueous solutions and water miscible solvents over a range of flow rates. Typically, the aqueous solution will be delivered at flow rates between 0.1 ml/min and 20 ml/min. The aqueous pump head and lines should be made of material that resist fouling by the bioactive molecule. The solvent is generally delivered 4-100 times faster than the aqueous and so a more powerful pump may be required. Typically the solvent will be delivered at between 2 ml/min and 200 ml/min.

The mixing device 18 provides a method for rapidly and intimately admixing a continuous aqueous stream with a continuous water miscible solvent stream such that precipitation begins to occur almost immediately. The diagram in Figure 16 is for illustrative purposes only and many different geometries could be employed.

The mixing device 18 may be any device that achieves rapid mixing of the two flows. Thus it can, for example, be a static device that operates by shaping the incoming liquid flow patterns or else a dynamic device that actively agitates the two solvents streams together. Preferably, it is a dynamic device. Agitation of the two streams can be achieved by use of a variety of means such as stirring, sonication, shaking or the like. Methods of stirring include a paddle stirrer, a screw and a magnetic stirrer. If magnetic stirring is used a variety of stirring bars can be used with different profiles such as for example a simple rod or a Maltese cross. The material lining the interior of the mixing device should preferably be chosen to prevent significant binding of the bioactive molecule or the particles onto it. Suitable materials may include 316 stainless steel, titanium, silicone and Teflon (Registered Trade Mark).

Depending on the production scale required the mixing device may be produced in different sizes and geometries. The size of the mixing chamber required is a function of the rate of flow of the two solvent streams. For flow rates of about 0.025 - 2 ml/min of aqueous and 2.5-20 ml/min of solvent it is convenient to use a small mixing chamber such as 0.2ml.

### Experimental Protocol

### Continuous Flow Co-precipitator

A continuous co-precipitation system was developed using two HPLC pumps and a re-designed dynamic solvent mixing chamber. The pumps used were Gilson 303 HPLC pumps which allow variable flow rates from 0.01-9.99ml min$^{-1}$. The re-designed mixing chamber, previously a Gilson 811 C dynamic mixer, was modified to allow rapid mixing and crystallisation of co-precipitants. The aim of the design was to produce a flow cell with a low internal dwell volume that allowed rapid discharge of the product crystals.

The internal static mixer/filter element was removed from a Gilson 811 C mixing chamber and replaced by a custom made insert machined from PTFE. This insert was designed to provide a much reduced internal dwell volume and to increase the internal flow turbulence. Increased turbulence is expected to reduce both crystal size and minimise cementing of crystals to form aggregates. The internal turbulence was also further controlled by modifying the internal dynamic mixer. The original element was replaced with an alternate magnetic stirring bar, shaped like a Maltese cross and this was then coupled to a variable speed MINI MR standard magetic stirrer module, which allowed speeds from 0-1500 rpm to be attained.

The discharge tube had an internal dimension of approximately 0.5 mm and was linked to a sealed glass jar in which the suspension was continuously collected and allowed to settle.

### Continuous Flow Micro-crystal Precipitation of Pharmacologically Useful Materials

A saturated solution of the material of interest was prepared in a mainly aqueous solution that may if required contain some water miscible solvent. A saturated solution of the same material was prepared in a mainly water miscible solvent or mixture of solvents. The mainly aqueous solution is delivered by one pump into the dynamic mixer and the mainly solvent solution is delivered by another pump. The flow rates of the two pumps can be tuned to provide the most appropriate conditions for precipitation to occur. In general the flow rate of one pump will be at least 4 times greater than the other in order for the change in solvent conditions to be sufficiently rapid that precipitation begins to take place within the mixing chamber. In other words nucleation needs to be rapid in order for microcrystals (i.e. PCMCs) to form.

### Example: D,L- Valine microcrystals

The basic procedure starts by saturating the two selected solvents with D,L-valine. In this particular example, the two solvents were water and isopropanol. Water was obtained in-house from Millipore water purification system. Isopropanol (Propan-2-ol/GPR) Product No 296942D, Lot No K30897546 227, was supplied by BDH and D,L-Valine, Product No. 94640, Lot No. 410496/1 was supplied by Fluka Chemik. Both solutions were saturated by placing an excess of D,L-valine into a specified amount of solvent. This was then shaken overnight on an automatic shaking machine. After approximately 12 hours shaking at room temperature, solvents were filtered, through Whatman Durapore (0.45$\mu$m) membrane filters.

Following solution preparation, pump A was primed with the protein/D,L-valine aqueous solution. Pump B was primed with D,L-valine solution. Prior to beginning co-precipitation, magnetic stirrer speed was set at ~750 rpm. Pump A was set at 0.25 ml min⁻¹, pump B was set at 4.75 ml min⁻¹. Once prepared, pumps were simultanouesly started, thus beginning co-precipitation.

Isolation of the micro-crystals (i.e. PCMCs) by gravity filtration and agitation produced free flowing dry powders. SEM images of the crystals show a narrow size dispersion and a consistent plate-like morphology.

## L-glutamine microcrystals

The basic procedure starts by saturating the two selected solvents with L-glutamine. In this particular example, the two solvents were water and isopropanol. Water was obtained in-house from Millipore water purification system. Isopropanol (Propan-2-ol/GPR) Product No 296942D, Lot No K30897546 227, was supplied by BDH and D,L-Valine, Product No. 94640, Lot No. 410496/1, supplied by Fluka Chemika. Both solutions were saturated by placing an excess of L-glutamine into a specified amount of solvent. This was then shaken overnight on an automatic shaking machine. After approximately 12 hours shaking at room temperature, solvents were filtered, through Whatman Durapore (0.45μm) membrane filters.

Following solution preparation, pump A was primed with the aqueous L-glutamine solution. Pump B was primed with the isopropanol L-glutamine solution. Prior to beginning co-precipitation, magnetic stirrer speed was set at ~750 rpm. Pump A was set at 0.25ml min⁻¹ and pump B was set at 4.75 ml min⁻¹. Once prepared, pumps were simultanouesly started, thus initiating the continuous flow co-precipitation process.

Isolation of the micro-crystals by gravity filtration produced compacted dry powder. SEM images of the crystals show a narrow size dispersion and a consistent elongated plate-like morphology

A similar procedure was also used to precipitate glycine from saturated solution.

## Bioactive molecule Micro-crystal Co-precipitation (i.e. Formation of PCMCs)

Below describes a typical co-precipitation experiment, the principle of which was obtained from previous milligram batch preparations of protein coated microcrystals.

As a test platform, the protein Europa esterase 1 (Cc/F5), isolated from *Candida cyclindracea (rugosa)* Product No. EU122C, Lot No. LAY Y53-002, supplied by Europa Bioproducts Ltd. was precipitated on to D,L-Valine, Product No. 94640, Lot No. 410496/1, supplied by Fluka Chemika. The co-precipitated product was then isolated by filtration, whereupon it was analysed by scanning electron microscopy and enzymatic assay.

The basic procedure starts by saturating two solvent solutions with D,L-valine. In this particular example, these two solutions were water and isopropanol. Water was obtained in-house from Millipore water purification system. Isopropanol (Propan-2-ol/GPR) Product No 296942D, Lot No K30897546 227, was supplied by BDH. Both solutions were saturated by loading in an excess of D,L-valine into a specified amount of solvent. This was then shaken overnight on an automatic shaking machine. After approximately 12 hours shaking at room temperature, solvents were filtered, through Whatman Durapore (0.45μm) membrane filters.

To the filtered, saturated water solution was then added a prescribed amount of esterase protein, made up in buffer.

Following solution preparation, pump A was primed with the protein/D,L-valine aqueous solution. Pump B was primed with D,L-valine solution. Prior to beginning co-precipitation, magnetic stirrer speed was set at ~750 rpm. Pump A was set at 0.25 ml min⁻¹, pump B was set at 4.75 ml min⁻¹. Once prepared, pumps were simultanouesly started, thus being co-precipitation.

Co-precipitated crystal products (i.e. PCMCs) were collected in a flask, and allowed to settle overnight. After settling, 90% of supernatant solution was decanted off. The flask was refilled with fresh isopropanol, thus washing the product of excess D,L-valine. After washing, product was filtered again using Whatman Durapore (0.45μm) membrane filter.

## Analysis Procedure

After isolation of the co-precipitated crystals, characterisation of crystals was performed using optical light microscopy and scanning electron microscopy. Both techniques allowed size and shape determination of the crystals produced.

Assessing the activity of the protein post-co-precipitation was achieved by enzymatic assay. A specific assay was used, whereby the esterase protein enzyme catalyses the breakdown of p-nitrophenyl butyrate to butanol and p-nitrophenol.

Parallel studies between pure esterase supplied by Europa, and esterase co-precipitated onto D,L-valine crystals demonstrated that a substantial amount of activity had been retained.

The solvent may be removed from precipitated microcrystals. Suspensions produced by the above continuous flow system or the batch process described previously can be settled under gravity and excess solvent decanted to give a final suspension of around 5-20 % by weight. These can be further concentrated and/or dried by standard separation techniques such as filtration, centrifugation or fluidised bed.

For very low residual solvent, low bulk density pharmaceutical formulations and pharmaceutically useful materials the solvent can be removed from the above suspensions by critical point drying using supercritical $CO_2$. This technique is known to be useful for removing residual low levels of solvent from particles. We have discovered that surprisingly it also has the advantage that it may lead to powders and pharmaceutical formulations with much lower bulk density than obtained by other isolation techniques. Low bulk density formulations are particularly useful for pulmonary delivery of bioactive molecules. Critical point drying can be carried out in a number of ways known in the art.

## Example

25 ml of a 2.5 % w/v suspension of D,L-valine crystals in isopropanol (prepared as above) were loaded into a high pressure chamber and supercritical fluid $CO_2$ was flowed through the suspension until all the isopropanol was removed. The pressure was slowly released and the low residual solvent, low bulk density powder was transferred into a sealed container. The supercritical fluid drying process does not effect the narrow size dispersion.

## Example 10

## DNA Coated Micro-crystals

**Types of DNA tested:**

• Synthetic oligonucleotide DQA-HEX (Dept of Chemistry, Strathclyde University, UK)

5'HEX (T*C)$_6$ GTG CTG CAG GTG TAA ACT TGT ACC AG

HEX = 2,5,'2',4',5',7'-hexachloro-6-carboxyfluorescein
T* = 5-(3-aminopropynyl)-2'-deoxyuridine

Medical application: allele-specific oligonucleotide commonly used to investigate chromosome 6 in the HLA-DQ region, which encodes for the class II major histocompatibility antigens, the human leucocyte antigens, which are concerned with the immune response (D. Graham, B.J. Mallinder, D. Whitcombe, N.D Watson, and W.E Smith. Anal. Chem. 2002, 74, 1069-1074).

## Distribution of DNA coated crystals in artificial lung (MSLI)

Oligonucleotide coated crystals have been prepared and shown to form particles suitable for pulmonary administration.
Experiments were carried out with a pure fluorescent labelled oligonuclitide DQA-Hex and a blend of this with a crude oligonuleotide preparation obtained from herring sperm. The blending experiment allowed the loading of oligonucleotide to be varied even with limited supplies of DQA-Hex.

## Methods

## 1. Preparation of OCMC

## Sample 1: Blend of DQA-HEX and crude oligonucleotides

4.6mg crude oligonucleotides

DNA from herring sperm (Sigma D-3159, Lot 51K1281, was degraded to "crude oligonucleotides", less than 50bp, termed "crude oligos")
Add 300µl saturated D,L-valine solution, mix well and boil for 1 min, then put on ice.
Add 100µl DQA-Hex (=26.3ug), boiled for 1 min (then put on ice) prior to addition.
Add this solution drop-wise (Gilson pipettor, yellow tips) into 6ml of 2-PrOH/saturated with D,L-valine, while mixing on a magnetic stirrer at 500rpm (Heidolph MR3000) at room temperature, let settle for about 30min, then filter (Durapore membrane filters, type HVLPO4700), transfer crystals into glass vial and let air-dry.

**Sample 2: DQA-HEX only**

100μl DQA-Hex (=26.3ug), boiled for 1 min (then put on ice) prior to addition add 300μl saturated D,L-valine solution, mix well.
Precipitation as above.

**2. Distribution of Powders in artificial lung**

Capsule loaded with 15.41mg powder (sample 1) or 13.52mg powder (sample 2).

**3. Measurement of concentrations of oligonucleotides in fractions collected in artificial lung**

(a) UV260nm- total amount of oligonucleotides

Perkin Elmer - Lambda 3 - UV/VIS Spectrometer, calibration standards using crude oligonucleotides.

(b) Fluorescence of fluorescence marker HEX (556/535nm) in DQA-HEX.

Perkin Elmer - LS45 Luminiscence Spectrometer, calibration standards using DQA-HEX.

**Results**

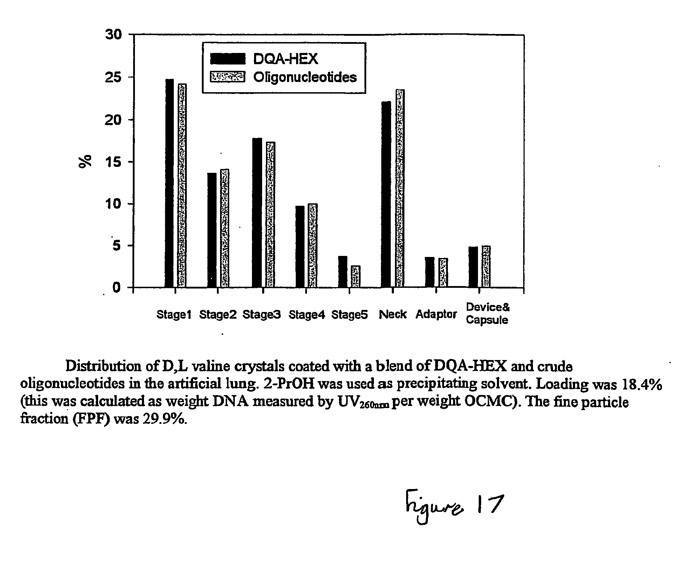

Figure 17 show the distribution of the micro-crystals in the artificial lung. The fine particle fraction (FPF) was 29.9% for micro-crystals coated with a blend of DQA-HEX and crude oligos and 24.4% for micro-crystals coated with DQA-HEX only. The results show that the MSLI protocol is robust since similar results were obtained using two different techniques for determining oligonucleotide concentration. Similarly it can be deduced that the two types of oligonucleotides were intimately mixed and are evenly distributed as a coating on the particles. It can also be seen from the high dose emission that the particles are free flowing and from the high FPF that they are useful for preparing pulmonary formulations.
PCR was performed using DQA-HEX, obtained on redissolving the DQA-HEX coated micro-crystals back into aqueous, as the primer. The correct gene product was amplified and sequencing of the PCR product showed that the sequence of the DQA-primer was unchanged. This result demonstrates that DNA coated onto microcrystals retains bioactivity and that no detectable degradation products are observed. This is advantageous for the production of pharmaceutical formulations.

**Example 11**

It is often difficult to ascertain that the bioactive molecule is coated on the surface of the particles since the coating may be very thin such as a monolayer. One method of checking if a coating has formed is to resuspend the particles back in a saturated solution of the crystalline core material. If the bioactive molecule is trapped with the matrix it will not redissolve but if it is a coating it will redissolve leaving behind uncoated crystals. This example shows that the oligonucleotides are coated on the surface of the crystals.

**Re-dissolution Experiment**

1. Production of OCMC: 2 mg crude oligonucleotides were dissolved in 50 μl TRIS (10 mM, pH=7.8) and 150 μl saturated aqueous solution of D,L-valine solution. This solution was added with a Gilson pipette (yellow tips, 0-200 μl) to 3 ml 2-PrOH saturated with D,L-valine, while stirred on a magnetic stirrer. The vial was left without stirring for at least further 30 min.

2. Aliquots of the OCMC suspensions (160 to 800 μl) were transferred into Eppendorf vials and spun at 9000 rpm (except A7/ B7/C7, which was separated by sedimentation). The supernatant was carefully removed and the remaining crystals air-dried.

3. Re-dissolution of crystals into known amount of saturated or near saturated aqueous solutions of D,L-valine.

4. Measurement of oligonucleotide concentration in aqueous phase after re-dissolution.
(oligonucleotide standards: 10 $\mu$g/ml: $OD_{260nm}$ = 0.226 or $OD_{260nm}$ = 1: 44.25 $\mu$g/ml; either dissolved into $H_2O$ (does not dissolve very well: ~2mg/ml) or saturated D,L-valine solution.

Table 28 summarises the conditions and results. From samples 1 (A1/B1/C1) and 2 (A2/B2/C2), where the crystals were completely dissolved, we get the maximum recovery rate of 84 ± 2 %, for samples no 3, 4, 6, 7 (D,L-valine crystals not dissolved). We find a mean recovery rate of 80 ± 4 %. From this we can conclude, that the oligonucleotides were completely dissolved in the saturated D,L-valine solution. This strongly indicates that the oligonucleotides are not in the matrix, but on the surface of the crystals. The same would apply for PCMCs.
Table 28 summarises the re-dissolution experiments and conditions.

Table 28

| Samples | Saturation of D,L valine solution | Mode of re-dissolution | Comments | DNA conc by $UV_{260nm}$ ($\mu$g/ml) | DNA conc calculated from initial weight ($\mu$g/ml) | % DNA re-dissolved |
|---|---|---|---|---|---|---|
| A1/B1/C1 | Near saturated | vortex | Crystals dissolved | 82 | 100 | 82 |
| A2/B2/C2 | Near saturated | vortex | Crystals dissolved | 85 | 100 | 85 |
| B3/C3 | At 40°C | Shake overnight | | 779 | 1000 | 78 |
| A4/B4/C4 | At 40°C, cooled to RT | vortex | | 753 | 1000 | 75 |
| A6/B6/C6 | At 40°C, cooled to RT | Shake overnight | | 1027 | 1250 | 82 |
| A7/B7/C7 | At 40°C, cooled to RT | vortex | | 353 | 417 | 85 |

## Example 12

Table 29 shows a range of conditions for forming $\alpha$1 - antitrypsin coated $\alpha$-lactose microcrystals wherein cystein (Cys) and N-acetyl cystein (NA Cys) were used as additives to prevent oxidation during the co-precipitation process.
Preparation of $\alpha$1 - antitrypsin coated $\alpha$-lactose microcrystals by precipitation into propanol generally leads to complete loss of bio-activity. The results are shown in Table 29 below.

Table 29

| Solvent | Antioxidant | Water (%) | lu.mg$^{-1}$ | % Activity Recovered | Protein mg.ml$^{-1}$ | % Protein Recovered |
|---|---|---|---|---|---|---|
| Propan-2-ol | Cys 10mg.ml$^{-1}$ | 0 | 0.93 | 38 | 11.4 | 100 |
| Propan-2-ol | Cys 10mg.ml$^{-1}$ | 1 | 0.6 | 25 | 11.7 | 100 |
| Propan-2-ol | Cys 10mg.ml$^{-1}$ | 10 | 0.5 | 20 | 4.30 | 38 |
| Propan-2-ol | NA Cys 0.22 mg.ml$^{-1}$ | 0 | 0.0 | 0 | 3.92 | 46 |
| Propan-2-ol | NA Cys 10 mg.ml$^{-1}$ | 0 | 0.008 | 0.32 | 3.45 | 44 |

Table 29 shows that cysteine and N-acetyl cystein produces $\alpha$ - antitrypsin coated microcrystals with a higher activity than those prepared without an antioxidant.
The experimental procedures are as defined below.

Cystein Addition During Precipitation and Dissolution

16mg of $\alpha 1$ - antitrypsin was dissolved in 0.4ml TRIS buffer (20mM, pH 8) containing 10 mg.ml$^{-1}$ cystein and added to 1.2ml of lactose-saturated TRIS buffer (20mM, pH 8) containing 10mg.ml$^{-1}$ cystein. 0.4ml of this solution was added dropwise to 6ml propanol containing different amounts of water. The activity and protein concentration in the final product was measured after dissolving the crystals in 0.8ml TRIS buffer containing 10mg.ml$^{-1}$ cysteine.

N-Acetyl Cystein Addition During Precipitation and Dissolution

10mg $\alpha 1$ - antitrypsin was dissolved in 1ml of lactose saturated TRIS buffer (20 mM, pH 8) containing 0.22mg.ml$^{-1}$ N-acetyl cystein. 0.4ml of this solution was added dropwise to 6ml of propan-2-ol containing either 0.22mg.ml$^{-1}$ or 10mg.ml$^{-1}$ N-acetyl cystein. For activity and protein concentration measurements, the crystal was dissolved in 0.4ml TRIS buffer containing the same concentration of N-acetyl cystein as the precipitation mixture.

These show that the excipient such as additives or anti-oxidants may be beneficially added to the co-precipitation to improve and retain the bio-activity.

## Example 13

### Vaccine PCMCs

PCMCs were made using ovalbumin, Diptheria Toxoid and Tetanus Toxoid with either D,L-valine or L-glutamine as the core crystalline material.

### Ovalbumin, Diptheria Toxoid (DT) and Tetanus Toxoid (TT) coated microcrystals

In all experiments half the volume of the aqueous solution was made up of the saturated amino acid solution. Ovalbumin was supplied as a powder. An appropriate amount of powder was weighed out to give a theoretical loading on the core material of 5, 10, 20 and 40%. To this either an amount of water was added to give a 50% saturated solution of the amino acid or in the cases where 2-methyl-2,4-pentanediol was also incorporated in the aqueous phase the volume of the diol added replaced an equal volume of water to keep the concentration of the amino acid constant. The co-precipitation of the protein and carrier was carried out in a volume of 2-propanol or 2-methyl-2,4-pentanediol ten times greater than the aqueous solution, giving a final percentage of $H_2O$ in the precipitating solvent of 9.1% for aqueous solutions without the addition of diol and 6.5% where 20% diol was added to the aqueous phase.

The aqueous solution was delivered by a syringe pump to the organic solvent contained in a small vial under magnetic stirring.

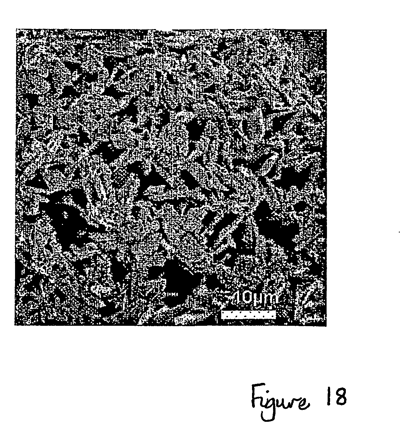

Figure 18 is an image of DT PCMCs with a 10% loading. The DT PCMCs have a crystalline core of L-glutamine and are precipitated in propan-2-ol.

### Mixed Diptheria Toxoid (DT), Tetanus Toxoid (TT) and Ovalbumin Coated Microcrystals

For mixed DT / TT PCMCs appropriate volumes of the DT stock solution (concentration = 19.5mg/ml) and TT stock solution (concentration = 27.5mg/ml) were added to the aqueous solution to be precipitated to give the required theoretical loading. For the ovalbumin / TT PCMCs the appropriate amount of ovalbumin was weighed out and to this was added the required volume of TT to give the required theoretical loadings. The crystals were then prepared as described above.

Table 30 - Ovalbumin

| No | protein loading (%) | Conditions | crystals (mg) |
|---|---|---|---|
| 1 | ovalbumin (10%) | dissolved in saturated D,L-valine/$H_2O$ soln (final volume =0.7ml) prec in 2-propanol (vol = 7ml) | 21 |
| 2 | ovalbumin (20%) | dissolved in saturated L-glutamine/$H_2O$ soln (final volume =0.7ml) prec in 2-propanol (vol = 7ml) | 12 |
| 3 | ovalbumin (10%) | dissolved in saturated D,L-valine/Tris-HCl, pH 7.8 soln (final volume =0.7ml) prec in 2-propanol (vol = 7ml) | 21 |

(continued)

| No | protein loading (%) | Conditions | crystals (mg) |
|---|---|---|---|
| 4 | ovalbumin (20%) | dissolved in saturated L-glutamine/Tris-HCl, pH 7.8 soln (final volume =0.7ml) prec in 2-propanol (vol = 7ml) | 13 |
| 5 | ovalbumin (10%) | dissolved in saturated D,L-valine/Tris-HCl, pH 7.8 soln (final volume =0.7ml) prec in 2-methyl-2,4-pentanediol (vol = 7ml) | 12 |
| 6 | ovalbumin (20%) | dissolved in saturated D,L-valine/Tris-HCl, pH 7.8 soln + 20% 2-methyl-2,4-pentanediol (final volume =0.7ml) prec in 2propanol (vol = 7ml) | 26 |

The coprecipitated ovalbumin showed no changes in structure or aggregation levels relative to ovalbumin in the initial aqueous preparation.

Table 31 - Diptheria Toxoid (DT)

| No | protein loading (%) | Conditions | crystals (mg) |
|---|---|---|---|
| 1 | DT (10%) | dissolved in saturated D,L-valine/Tris-HCl, pH 7.8 soln (final volume =0.7ml) prec in 2-propanol (vol = 7ml) | 21 |
| 2 | DT (5%) | dissolved in saturated L-glutamine/Tris-HCl, pH 7.8 soln (final volume =0.7ml) prec in 2-propanol (vol = 7ml) | 12 |
| 3 | DT (20%) | dissolved in saturated L-glutamine/Tris-HCl, pH 7.8 soln (final volume =0.7ml) prec in 2-propanol (vol = 7ml) | 21 |
| 4 | DT (40%) | dissolved in saturated L-glutamine/Tris-HCl, pH 7.8 soln (final volume =0.7ml) prec in 2-propanol (vol = 7ml) | 23 |
| 5 | DT (20%) | dissolved in saturated L-glutamine/Tris-HCl, pH 7.8 soln (final volume =0.7ml) prec in 2-methyl-2,4-pentanediol (vol = 7ml) | 12 |
| 6 | DT (20%) | dissolved in saturated D,L-valine/Tris-HCl, pH 7.8 soln + 20% 2-methyl-2,4-pentanediol (final volume =0.7ml) prec in 2 propanol (vol = 7ml) | 13 |

Table 32 - Tetanus Toxoid (TT)

| No | protein loading (%) | Conditions | crystals (mg) |
|---|---|---|---|
| 1 | TT (5%) | dissolved in saturated D,L-valine/Tris-HCl, pH 7.8 soln (final volume =1.4ml) prec in 2-propanol (vol = 14ml) | 21 |
| 2 | TT (20%) | dissolved in saturated L-glutamine/Tris-HCl, pH 7.8 soln (final volume =1.4ml) prec in 2-propanol (vol = 14ml) | 21 |
| 3 | TT (40%) | dissolved in saturated L-glutamine/Tris-HCl, pH 7.8 soln (final volume =1.4ml) prec in 2-propanol (vol = 14ml) | 23 |
| 4 | TT (20%) | dissolved in saturated L-glutamine/Tris-HCl, pH 7.8 soln (final volume =1.0ml) prec in 2-methyl-2,4-pentanediol (vol = 10ml) | 12 |
| 5 | TT (10%) | dissolved in saturated D,L-valine/Tris-HCl, pH 7.8 soln + 15% 2-methyl-2,4-pentanediol (final volume =1.4ml) prec in 2propanol (vol = 14ml) | 12 |
| 6 | TT (10%) | dissolved in saturated L-glutamine/Tris-HCl, pH 7.8 soln + 15% 2-methyl-2,4-pentanediol (final volume =1.4ml) prec in 2propanol (vol = 14ml) | 14 |

Table 33 - Mixed Crystals

| No | protein loading (%) | Conditions | crystals (mg) |
|---|---|---|---|
| 1 | DT(10%) TT(10%) | dissolved in saturated D,L-valine/Tris-HCl, pH 7.8 soln (final volume =1.4ml) prec in 2-propanol (vol = 14ml) | 23 |
| 2 | DT(10%) TT(10%) | dissolved in saturated L-glutamine /Tris-HCl, pH 7.8 soln (final volume =1.4ml) prec in 2-propanol (vol = 14ml) | 12 |
| 3 | DT(10%) TT(10%) | dissolved in saturated L-glutamine/Tris-HCl, pH 7.8 soln + 15% 2-methyl-2,4-pentanediol (final volume =1.4ml) prec in 2propanol (vol = 14ml) | 13 |
| 4 | DT(15%) TT(15%) | dissolved in saturated D,L-valine/Tris-HCl, pH 7.8 soln (final volume =1.4ml) prec in 2-propanol (vol = 14ml) | 14 |
| 5 | TT(10%) ovalbumin(10%) | dissolved in saturated D,L-valine/Tris-HCl, pH 7.8 soln (final volume =1.4ml) prec in 2-propanol (vol = 14ml) | 21 |
| 6 | TT(10%) ovalbumin(30%) | dissolved in saturated D,L-valine/Tris-HCl, pH 7.8 soln (final volume =1.4ml) prec in 2-propanol (vol = 14ml) | 26 |

**Diptheria Toxoid (DT) Formulation Made Up for Mouse Study**

Vaccine coated microcrystals were produced with a theoretical loading of DT of 5%. L-glutamine made up the crystalline core material and 2-propanol was used as the water miscible organic solvent.

DT was supplied as an aqueous solution at a concentration of 14.5 mg / ml. 276 $\mu$l of the DT solution was added to 2313 $\mu$l saturated L-glutamine solution. To this was added 2037 $\mu$l H$_2$O and 4.5ml of the mixture was co precipitated into 45 ml of L-glutamine saturated 2-propanol under magnetic stirring. Around 80 mg of DT-glutamine crystals were recovered and 50 mg used for a vaccine trial in mice. The DT-glutamine crystals were stored at 4°C.

**Variation of storage conditions prior to administration**

Comparable samples of DT in aqueous buffer and samples of dry DT-glutamine microcrystals were stored as follows:

incubation at 4 degrees C for 2 weeks;
incubation at room temperature for 2 weeks;
incubation at 37 degrees C for 2 week; and
incubation at 45 degrees C for 2 days.

**In vivo Immunological Experiments Using DT as Antigen**

Prior to administration to mice, the incubated microcrystals were suspended in phosphate-buffered saline (PBS). 1350 microgram of crystals (50 microgram of DT) were suspended in 500 microlitres of PBS. Each mouse received 50 microlitres of the suspension (i.e. 5 microgram of DT) by intramuscular administration in the left hind leg on day 1.

Mice were bled on day 21. Mice received a booster dose of DT - same mass of DT as before, on day 29. Mice were bled again on day 42. The sera were analysed using ELISA assays.

The primary and secondary immune responses showed that samples of DT-glutamine microcrystals gave rise to antibodies (humoral immunity) whatever the storage protocol. This proves that the production process for vaccine coated microcrystals leads to good retention of DT bioactivity and that following reconstitution and intramuscular administration the DT is freely bioavailable.

All DT samples stored in aqueous buffer also gave primary and secondary immune responses except for the sample stored at 45°C which showed no bioactivity.

The presence of a primary and secondary immune response for DT-glutamine microcrystals stored at 45°C shows that formulation of DT into microcrystals has imparted significantly enhanced storage stability at elevated temperature relative to in solution.

Such enhanced stability has important advantages for distribution and administration of vaccines in hostile environments, emergency situations and in the developing world.

It can therefore be concluded that forming PCMCs with a vaccine coating, imparts an extra amount of stability to the vaccine which makes the vaccine easier to store and transport. This may be useful in hot countries.

## Example 14

### Ex-vivo Measurement of Insulin Bioactivity on Insulin Coated D,L-valine Microcrystals.

Part 1

Insulin bioactivity assays were carried out on resistance arteries (<200ìm dimension) isolated from 12 week old male Wistar rats studied in heated (37°C) and gassed (95%$O_2$/5%$CO_2$) physiological salt solution (PSS) to achieve a pH of 7.4. A pressure myograph which allowed lumenal application of drug provided initial measures of sensitivity. In the pressure system, arteries mounted on opposing glass cannula (outer dimension $80\mu m$) were gradually pressurised from <5mmHg to 40 mmHg over 15 mins and held for 15 mins more before starting the assay. Responses were measured using proprietary video analysis software (MyoView). The pressure myograph is able to detect the vasodilatory effect of insulin at very low concentrations (lx $10^{-10}$ M)

**Table 34 Sample Preparation**

| Bioactive Molecule | Bioactive Molecule dissolved in Solvent | H$_2$O% (v/v) | Conc. of Bioactive Molecule in Solvent (mg/ml) | Addition of excipient | Wash Step | Crystallisation Process | % protein recovered | % max protein in crystal |
|---|---|---|---|---|---|---|---|---|
| 17mg USP Insulin (I8405) | 1.7ml of 0.01MHCl and then 85□1 of 1M NaOH added | 9.1 | 0.44 | 1.7ml of distilled water saturated with D,L-valine added to insulin giving a 49% saturation of D,L-valine | Dry propan-2-ol | 3.4ml of insulin in D,L-valine added dropwise to 34ml of propan-2-ol with constant agitation at room temp | | 17 |
| The particles were reconstituted at a concentration of 10nM protein in water | | | | | | | | |
| 17mg USP Insulin (I8405) | 1.7ml of 0.01MHCl and then 85□1 of 1M NaOH added | 9.1 | 0.44 | 1.7ml of distilled water saturated with D,L-valine added to insulin giving a 49% saturation of D,L-valine | Dry propan-2-ol | 3.4ml of insulin in D,L-valine added dropwise to 34ml of propan-2-ol with constant agitation at room temp | | 17 |
| The particles were reconstituted at a concentration of 10nM protein in water | | | | | | | | |

**Results**

Table 34 shows insulin mediated relaxation to noradrenaline preconstriction (100 = 100% constriction), mean of 3 (SD), the values show no significant difference between the microcrystals and the control (p > 0.05).

Table 35

| Log M | Commercial Insulin | Insulin coated D,L-valine microcrystals |
|---|---|---|
| -11 | 100 (0) | 100 (0) |
| -10 | 84 (7) | 84 (14) |
| -9 | 65 (23) | 68 (22) |

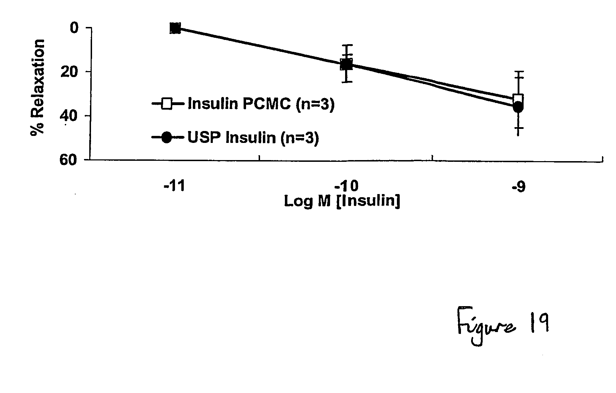

The degree of relaxation afforded by the insulin PCMC as shown in Figure 19 is similar to that of the USP insulin formulation indicating no insulin denaturation during production or room-temperature storage of the PCMC.

**Part 2**

**Wire Myograph studies**

A wire myograph was then used to provide greater throughput for subsequent studies (P110 & P660, Danish MyoTech, Aarhus. In the wire system, arteries were mounted between two 40im stainless steel wires, one connected to a micrometer, the other to a force transducer and set to a known standardised dimension to produce an optimal pharmacological response. Force production was captured by proprietary software (MyoDaq). All bioassays began with two washes of 123mM KCl, to stimulate contractile function in the arteries, followed by preconstriction by exposure to a vasoconstrictor agonist, thromboxane mimetic [U44169]. The arteries were then exposed to increasing concentrations of insulin either directly into the bath (wire) or by gradual infusion directly into the lumen via a fetal microcannulae inserted to the tip of the glass mounting cannula, at a constant pressure (pressure).

**Sample preparation**

The insulin used was USP bovine pancreas insulin (Sigma 18405) Mixing was always carried out by magnetic stirring

Crystals were isolated by filtering through Durapore membrane filters (0.4 microns) and were then dried in air in the fume hood

Protein loadings are based on maximum determined from yield of crystals

Table 36

| Bioactive Molecule | Bioactive Molecule dissolved in Solvent | $H_2O\%$ (v/v) | Conc. of Bioactive Molecule in Solvent (mg/ml) | Addition of excipient | Wash Step | Crystallisation Process | % protein recovered | % max protein in crystal |
|---|---|---|---|---|---|---|---|---|
| 20mg USP Insulin (18405) | 2.0ml of 0.01MHCl and then 100□1 of 1M NaOH added | 9.1 | 0.44 | 2ml of distilled water saturated with D,L-valine added to insulin giving a 49% saturation of D,L-valine | Dry propan-2-ol | 3.5ml of insulin in D,L-valine added dropwise to 35ml of propan-2-ol with constant agitation at room temp | | 17 |

(continued)

| Bioactive Molecule | Bioactive Molecule dissolved in Solvent | H$_2$O% (v/v) | Conc. of Bioactive Molecule in Solvent (mg/ml) | Addition of excipient | Wash Step | Crystallisation Process | % protein recovered | % max protein in crystal |
|---|---|---|---|---|---|---|---|---|
| The particles were reconstituted at a concentration of 0.9mM protein in water | | | | | | | | |
| 16mg USP Insulin (I8405) | 1.6ml of 0.01MHCl | 9.1 | 0.44 | 1.6ml of distilled water saturated with D,L-valine added to insulin giving a 49% saturation of D,L-valine | Dry propan-2-ol | 2.8ml of insulin in D,L-valine added dropwise to 28ml of propan-2-ol with constant agitation at room temp | | 17 |
| The particles were reconstituted at a concentration of 1mM protein in water | | | | | | | | |
| 12mg USP Insulin (I8405) | 1.2ml of 0.01MHCl and then 60□1 of 1M NaOH added | 9.1 | 0.44 | 1.2ml of distilled water saturated with D,L-valine added to insulin giving a 49% saturation of D,L-valine | Dry propan-2-ol | 2.1ml of insulin in D,L-valine added dropwise to 21ml of propan-2-ol with constant agitation at room temp | | 17 |
| 12mg USP Insulin (I8405) | 1.2ml of 0.01MHCl and then 6001 of 1M NaOH added | 9.1 | 0.44 | 1.2ml of distilled water saturated with D,L-valine added to insulin giving a 49% saturation of D,L-valine | Dry propan-2-ol | 2.1ml of insulin in D,L-valine added dropwise to 21ml of propan-2-ol with constant agitation at room temp | | 17 |
| The particles were reconstituted at a concentration of 0.9mM protein in water | | | | | | | | |

(continued)

| Bioactive Molecule | Bioactive Molecule dissolved in Solvent | H₂O% (v/v) | Conc. of Bioactive Molecule in Solvent (mg/ml) | Addition of excipient | Wash Step | Crystallisation Process | % protein recovered | % max protein in crystal |
|---|---|---|---|---|---|---|---|---|
| 12mg USP Insulin (18405) | 1.2ml of 0.01MHCl | 9.1 | 0.44 | 1.2ml of distilled water saturated with D,L-valine added to insulin giving a 49% saturation of D,L-valine | Dry propan-2-ol | 2.1ml of insulin in D,L-valine added dropwise to 21ml of propan-2-ol with constant agitation at room temp | | 17 |
| 12mg USP Insulin (18405) | 1.2ml of 0.01MHCl | 9.1 | 0.44 | 1.2ml of distilled water saturated with D,L-valine added to insulin giving a 49% saturation of D,L-valine | Dry propan-2-ol | 2.1ml of insulin in D,L-valine added dropwise to 21ml of propan-2-ol with constant agitation at room temp | | 17 |
| The particles were reconstituted at a concentration of 0.9mM protein in water | | | | | | | | |

### Results

Figure 19 shows a summary of the myograph results.

Following preconstriction with thromboxane mimetic [U44169] the insulin-mediated vasorelaxation profile is typical for insulin and exerts its effect mainly via the activation of nitric oxide synthase and the subsequent release of endothelial nitric oxide.

The insulin mediated vasorelaxation afforded by the insulin coated D,L-valine microcrystals was essentially identical to the USP insulin formulation. D,L-valine on it's own showed no bioactivity. These results show that the insulin bioactivity is unchanged either by the co-precipitation process or by long-term room-temperature storage of the insulin coated microcrystals. This is strong proof that the insulin has not been chemically modified, aggregated or undergone any irreversible denaturation during processing or storage. The absence of degradation was backed up by HPLC analysis that showed that immediately following reconstitution of the D,L-valine microcrystals more than 90% of the insulin was still present in the same form following coprecipitation and storage as a powder at room temperature for more than 6 months. In contrast insulin retained in the same aqueous solution used for coprecipitation underwent significant changes in less than 30 minutes. We have shown insulin coated D,L-valine microcrystals to be free-flowing powders which exhibit high fine-particle fractions in multi-stage impinger tests and so it is evident that bioactive molecule coated micrystals are very suitable for making pharmaceutical formulations with enhanced properties.

### Example 15

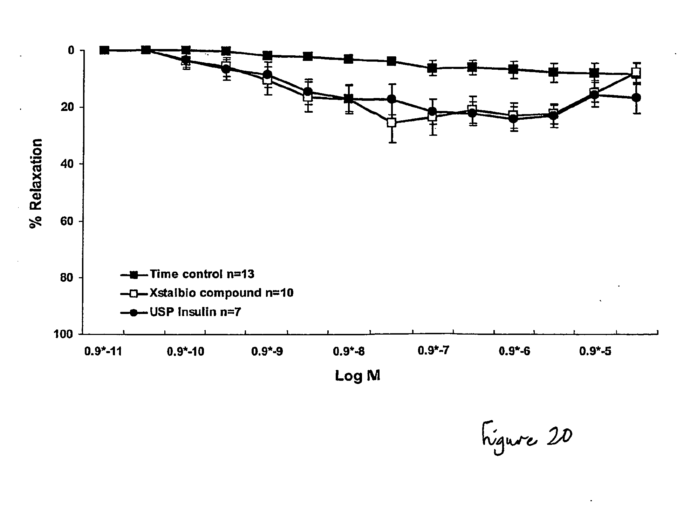

Figures 20 to 24 are SEM images of a selection of PCMCs made according to the present invention.

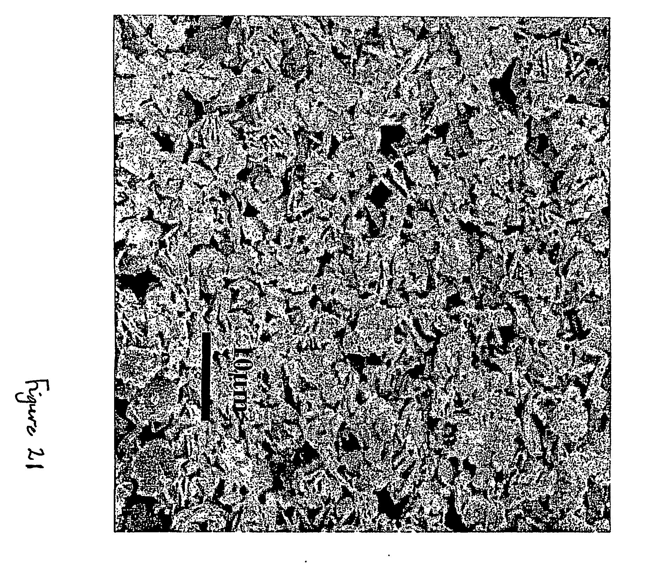

Figure 20 is an SEM image of insulin/D,L-valine PCMCs precipitated in propan-2-ol at X1600 magnification. Figure 21 is a further SEM image of insulin/D,L-valine precipitated in propan-2-ol at X6400 magnification. Figures 20 and 21 show that the crystals are flake-like and are substantially homogeneous in shape and size and that there is a substantially even coating of insulin.

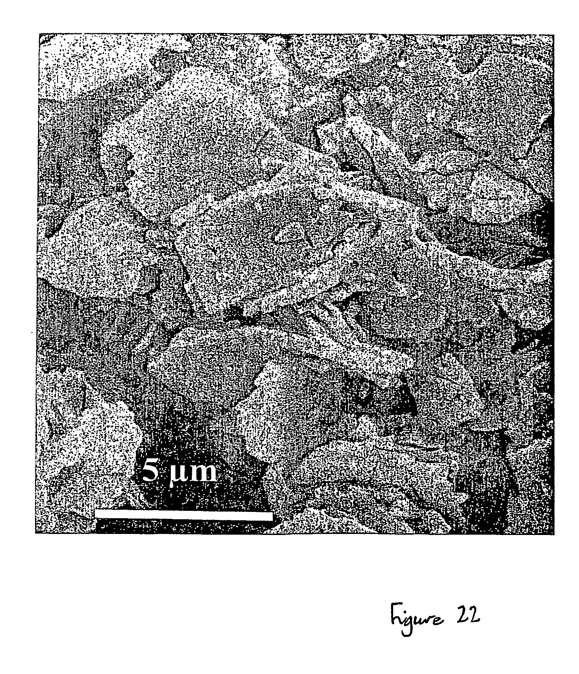

Figure 22 is an SEM image of albumin/L-glutamine PCMCs precipitated in propan-2-ol. The PCMCs in this instance are again homogeneous but are needle shaped.

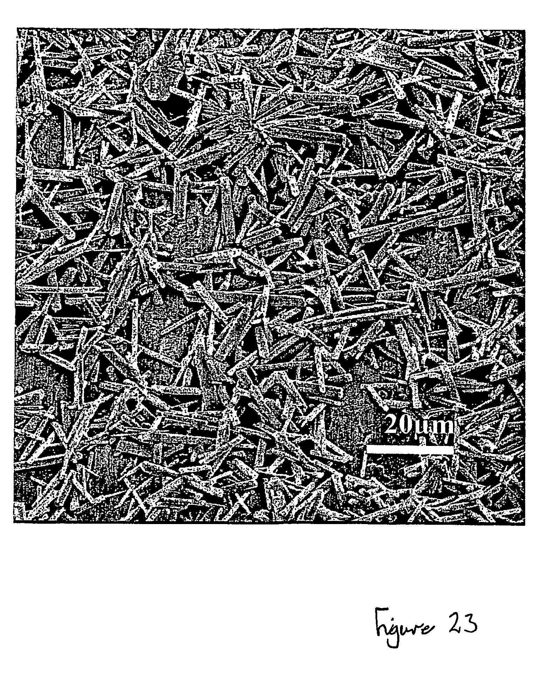

Figure 23 is an SEM image of insulin/L-histidine PCMCs precipitated in propan-2-ol which are homogeneous and flake-like.

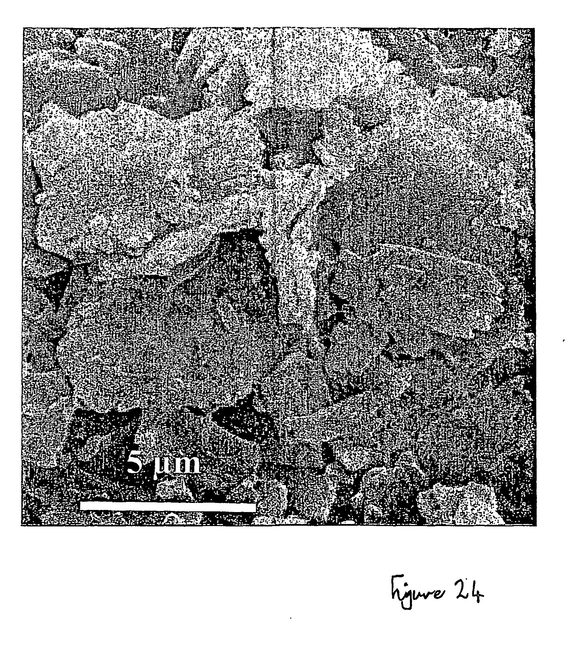

Figure 24 is an SEM image of $\alpha$-antitrypsin/D,L-valine PCMCs precipitated in propan-2-ol. The PCMCs are shown to be substantially homogeneous in shape and size and are flake-like.

### Example 16

**Tobramycin sulphate coated microcrystals**

In this example we demonstrate that surprisingly the coprecipitation process can also be used to make bioactive molecule coated microcrystals suitable for pharmaceutical formulations using water-soluble bioactive compounds that are much smaller than typical biological macromolecules. These formulations may be made either by a batch or by a continuous process and may advantageously employ a non-hygroscopic carrier such as D,L-valine. The process is demonstrated for the water-soluble antibiotic drug, tobramycin sulphate but can be applied to other antibiotics and other water-soluble bioactive molecules. Preferably the bioactive molecule should be polar and contain one or more functional groups that is ionised at the pH used for coprecipitation. This tends to lead to higher solubility in water and reduced solubility in water miscible organic solvent. The compound should also preferably have a largest dimension greater than that of the unit cell formed by the core material on crystallisation. This will favour formation of bioactive molecule coated microcrystals and minimise the possibility of inclusion of the bioactive molecule within the crystal lattice.

**Experimental**

**Batch Process**

Batches containing different theoretical loadings of bioactive molecule on the D,L-valine carrier crystals were prepared by using either 3 mg (*4.8 %w/w*), 6 mg (*9.1 %w/w*) or 12 mg (*16.7 %w/w*) of tobramycin sulphate (T-1783 from Sigma). In each case the weighed quantity of tobramycin sulphate was dissolved in 1ml of D,L-valine in distilled water (at 60mg/ml). 0.5ml of the above was added dropwise by 1ml pipette to 10ml of Pr2OH saturated with D,L-valine with mixing at 1500rpm. Crystals were filtered immediately under vacuum through Durapore 0.4 micron filters, washed with 10ml of Pr2OH (1% $H_2O$ v/v) and dried in air in the fume hood.

**Continuous Process**

*Theoretical loading 4.8 %w/w*

30 mg of Tobramycin sulphate (T-1783 from Sigma) was dissolved in 10 ml of D,L-valine in distilled water (at 60 mg/ml). 5ml of aqueous solution was mixed with Pr2OH saturated with D,L-valine (100 ml) on a continuous coprecipitation system as described in Example 9 with flow rates of 0.5ml/min for the aqueous pump and 10ml/min for the solvent pump using a dynamic mixer speed of 750 rpm. Crystals with a theoretical loading of 4.8 % w/w were collected, filtered under vacuum on Durapore 0.4 micron filters, washed with 50 ml of propan-2-ol ( containing 1% $H_2O$ v/v) and dried in air in the fume hood.

*Theoretical loading 1.6% w/w*

20mg of Tobramycin sulphate (T-1783 from Sigma) was dissolved in 20ml of D,L-valine in distilled water (at 60mg/ml). 5ml of the aqueous solution was mixed with propan-2-ol saturated with D,L-valine (100ml) on the continuous coprecipitation system described in Example 9 with flow rates of 0.5ml/min for the aqueous pump and 10ml/min for the solvent pump using a dynamic mixing speed of 750 rpm. Crystals were collected, filtered under vacuum on Durapore 0.4 micron filters, washed with 50ml of Pr2OH (1% $H_2O$ v/v) and dried in air in the fume hood.

**Results**

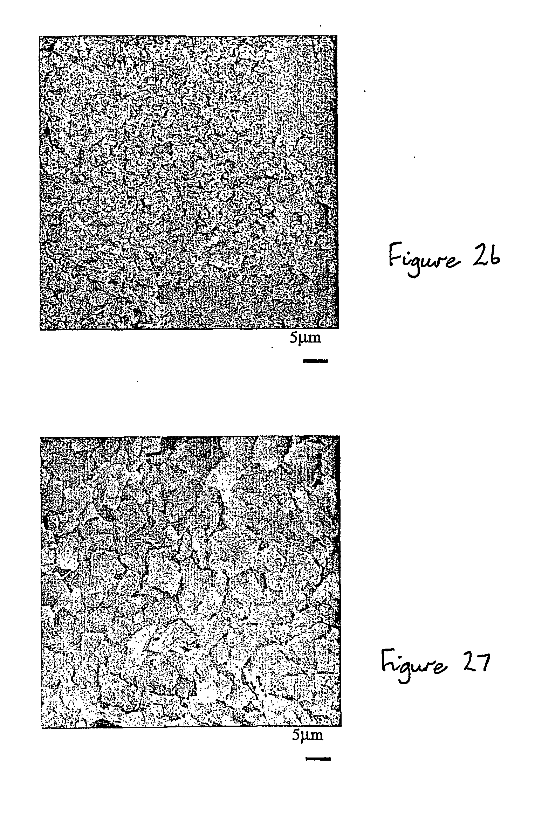

Tobramycin coated valine crystals prepared above are free flowing and non-hygroscopic and well suited for producing pharmaceutical formulations. SEM images of the particles prepared by the batch process show they have the flake-like morphology typical of valine microcrystals and an average maximum diameter of less than 5 microns making them suitable for pulmonary delivery. There are no obvious differences in size or morphology as the loading is changed. Figure 26 shows a sample prepared by the batch process with a loading of 9.1 % w/w. The particles prepared by the continuous process are also free flowing with a smooth well-defined morphology. The lower mixing rate and smaller impeller used in the continuous mixer leads to particles that are larger than in the batch process as shown in Figure 27.

Conclusion

Surprisingly bioactive molecule coated microcrystals where the active agent is not a biological macromoleule can be obtained and can be manufactured by a continuous coprecipitation process.

## Example 17

**Agents for changing the morphology and aggregation properties of bioactive molecule coated microcrystals**

The aggregation of microcrystals with, for example, needle-like morphology into larger more spherical particles can be advantageous for pharmaceutical formulations. Needle-like particles have poor flow properties while spheres can provide powders with good processing and drug delivery properties. Alternatively if the growth of microcrystal needles can be changed to produce a shorter rod-like morphology improved processing can also be obtained. Here we demonstrate that the addition of certain agents such as inorganic, organic salts or buffer salts at concentrations much lower than the coprecipitant can be used to modify the shape and aggregation properties of bioactive molecule coated microcrystals. Of particular advantage are pharmaceutically acceptable additives that have a second function such as pH buffering or isotonicity in the reconstituted formulation. The use of this type of additive minimises the number of components required in the final formulation.

**Rods and spherical aggregates of Subtilisin Carlsberg/L-Glutamine microcrystals**

**Experimental**

Either 5 mg (*0.7% w/w loading, G7*) or 25 mg (*6.4% w/w loading*, *G10*) of subtilisin Carlsberg was dissolved in 4ml of buffer (50 mM sodium citrate, 150 mM sodium chloride, pH 5.5) and 6ml of distilled water. To 0.25 ml of the above was added 0.75 ml of L-glutamine in distilled water (at 24.3 mg/ml). The aqueous solution was then added dropwise by 1ml pipette into 10ml of EtOH saturated with L-glutamine with mixing at 1500 rpm. An aliquot of crystals was applied directly to an SEM stub to assess morphology before drying (G7*, G10*). The remaining crystals were filtered immediately under vacuum onto a Durapore 0.4 micron filter, washed with 5ml of anhydrous Pr2OH and dried in air in the fume hood.

**Results**

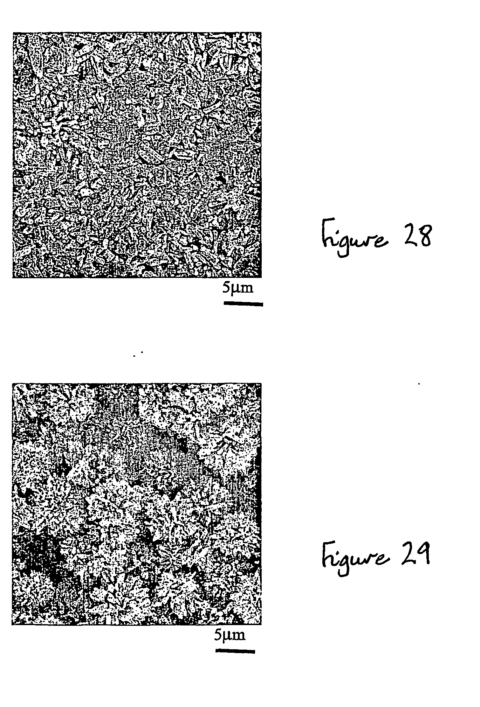

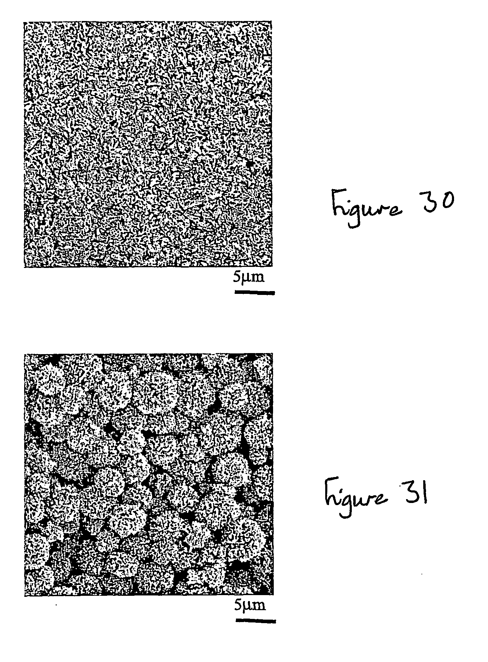

Protein coated L-glutamine microcrystals produced by coprecipitation from water into ethanol typically exhibit needle-like morphology with dimensions about 5 microns. Coprecipitation in the presence of low concentrations of sodium citrate and sodium chloride surprisingly leads to a significant reduction in the length of the needles. The change in length is further controlled by the concentration of protein with smaller rods being produced as the protein loading is increased. Figure 28 and Figure 30 show SEM images of typical bioactive molecule coated glutamine crystals coprecipitated in the presence of sodium citrate and sodium chloride. At 6.4 % w/w the rods are mainly less than 3 microns and on average less than 2 microns in length. Such a suspension of bioactive molecule coated microcrystals in ethanol may have advantageous properties for pharmaceutical formulations. For example, the suspension could be delivered by a pulmonary route using inhalation devices known in the art. Further increases in protein loading can be used to reduce the size microcrystal further. Isolation of the rods as a dry powder made up of individual crystals may be achieved by critical point drying. If conventional filtration of the microcrystals onto a filter membrane is used followed by air drying a remarkable transformation takes place and particles made up of spherical aggregates of the needles or rods are produced. These very high surface area spherical particles advantageously form a free-flowing powder and are non-hygroscopic. They can also be reconstituted very rapidly such as in less than 10 to 20 seconds in aqueous solution. SEM images showing the spherical aggregates are shown in Figure 29 and Figure 31. The transformation of needle-like microcrystals into spherical aggregates is very advantageous since spheres are much easier to process and use in pharmaceutical formulations. Very similar results to those shown here can be obtained with other proteins including therapeutic proteins.

**Conclusion**

The use of low concentrations of pharmaceutically acceptable agents such as buffers and salts in the coprecipitation process leads to surprisingly large and useful differences in the morphology and aggregation behaviour of bioactive molecule coated microcrystals. The concentration of modifying agent used should be such that it is present at less than 15 % w/w in the final formulation and preferably less than 10 % w/w. If the concentration of modifier is too high it may lead to phase separation from the bulk carrier crystals and formation of a second type of bioactive molecule coated crystal.

### Example 18

**Powder X-ray diffraction measurements on carrier microcrystals and protein coated microcrystals prepared by the continuous process.**

Microcrystals of L-glutamine, D,L-valine and glycine were prepared by precipitation into ethanol, isopropanol and isopropanol respectively using the continuous process described in Example 9. The same materials and solvents were used to prepare albumin coated microcrystals at 10 % w/w loading also by the continuous coprecipitation process. Powder X-ray diffraction was used to compare dry powder samples prepared with and without protein.

**Experimental**

Samples were analyzed using a Bruker AXS D8 Advance, with a PSD-detector with the following instrumental parameters:

| Radiation | CuK$\alpha$ radiation, $\lambda$=1.5418 Angstrom |
|---|---|
| Tube Power | 40kV, 40mA |
| Scan Range | 3 ° - 40 ° 2theta |
| Step Size | 0.014 ° 2theta |
| Time/Step | 0.5 sec |
| Sample Rotation | On |
| Sample Preparation | No grinding |

**Results**

Comparisons were made between samples with and without Albumin:

| Sample | Results |
|---|---|
| JV272 / 1/2 K$_2$SO$_4$ - Isopropanol<br>JV272 / 1/3 K$_2$SO$_{4/}$Albumin - Isopropanol | Diffraction patterns of sample without and with Albumin are consistent. Minor differences are likely due to orientation effects and degree of crystallinity of the samples. |
| JV272 / 2/2 DL-Valin Isopropanol<br>JV272 / 2/3 DL-Valin/Albumin - Isopropanol | Diffraction patterns of sample without and with Albumin are consistent. Minor differences are likely due to orientation effects and degree of crystallinity of the samples. |
| JV272 / 3/2 Glycin - Isopropanol<br>JV272 / 3/3 Glycin/Albumin - Isopropanol | Significant differences were noted in the diffraction patterns of the samples with and without Albumin. Most notably, the diffraction lines at approximately 18 and 23.8° two-theta present in the sample containing Albumin are absent in the sample without Albumin. |
| JV272 / 5/2 Glutamin - Ethanol<br>JV272 / 5/3 Glutamin/Albumin - Ethanol | Diffraction patterns of sample without and with Albumin are consistent. Minor differences are likely due to orientation effects and degree of crystallinity of the samples. |

**Glutamine**

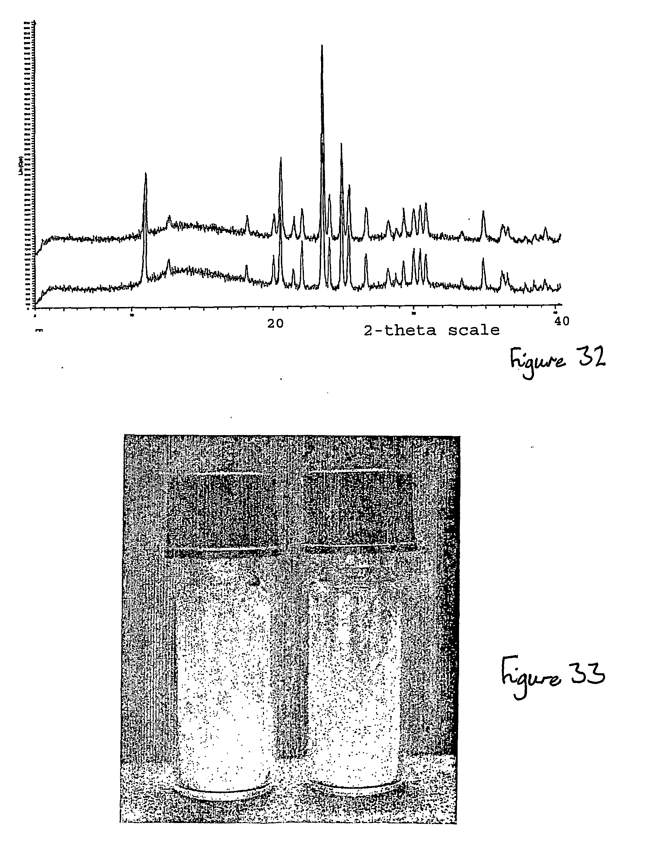

The PXRD data of glutamine precipitated in ethanol with and without protein were found to be in excellent agreement with each other and with a known single-crystal structure (orthorhombic $P2_12_12_1$, 16.020, 7.762, 5.119 - see Koetzle et al Acta Cryst. B 1973, 29, 2571). Figure 32 shows typical data obtained. The broad hump observed in the 12 to 18 degree region could be due either to amorphous material or may be an artifact of the experimental process. The peaks of the albumin sample lie at slightly higher angle than those of pure glutamine.

**Valine**

The PXRD patterns with and without protein are essentially identical. There are two possible known polymorphs (monoclinic $P2_1/c$ 5.21, 22.10, 5.41, beta = 109.2 Acta Cryst B 1969, 25, 296 and triclinic P-1 5.222, 5.406, 10.835, 90.89, 92.34, 110.02 Acta Cryst C 1996, 52, 1759). Identifying which polymorph is present is complicated by several factors. The large preferred orientation of the sample gives 3 large peaks - with all the rest of the pattern relatively small and difficult to differentiate from background. Thus the positions of these peaks are rather inaccurate. The triclinic sample was run at 120K. Thus it will have a slightly different unit cell to that at RT where the PXRDs were run and would not be expected to give a good fit to the observed data. The two polymorphs have several rather similar cell dimensions and are fairly closely related - they thus give similar predicted peaks. It is probable that the samples are in the monoclinic polymorph but this is not certain.

**Glycine**

The PXRD of glycine coprecipitated with albumin shows extra peaks compared with that of pure glycine. There are three reported forms of glycine (monoclinic $P2_1/n$, monoclinic $P2_1$ & trigonal -see Acta Cryst 1972, 28, 1827; Acta Cryst 1960, 13, 35 & Acta Cryst B 1980, 36, 115). There is no evidence for the trigonal form in either sample. The pure glycine PXRD is an excellent fit to the $P2_1/n$ polymorph. The extra peaks in the glycine/Alb sample can be explained by the presence of some $P2_1$ polymorph. This sample is thus a mixture of the 2 polymorphs a significant amount of both phases present.

**Conclusions**

PXRD data show that the core of the powder particles remains highly crystalline following coprecipitation with 10 % w/w protein. For glutamine and D,L-valine the protein coating does not change the polymorph of the core crystalline carrier compared to precipitation of the pure material. A highly crystalline core is advantageous for producing pharmaceutical formulations stable to elevated humidity and temperature. With glycine the protein appears to promote partial formation of a different polymorph. Directing which polymorph of a water soluble drug is formed by coprecipiation with a biological macromolecule could be advantageous for pharmaceutical formulation because for example bioactivity and bioavailability can be affected by which polymorph is present.

DSC was used to measure the melting temperatures. The valine and albumin coated valine microcrystal samples, JV272/2/2 and JV272/2/3, respectively, were both found to melt at a temperature of greater than 225 centigrade. The glutamine and albumin coated glutamine microcrystal samples, JV272/5/2 and JV272/5/3 were both found to melt at a temperature of greater than 160 centigrade.

**Example 19**

**Dry powders of bioactive molecule coated microcrystals prepared by critical point $CO_2$ drying of suspensions of microcrystals in solvent.**

Filtration of suspensions of microcrystals can lead to caking and compaction of the product. This may be reversible but requires another process step. Critical point drying can be advantageously used to obtain solvent free, low density, powders of bioactive coated microcrystals directly from a suspension in solvent. These powders have very attractive properties for preparing pulmonary formulations because they are non-hygroscopic and exhibit low electrostatic charge. Powders prepared by critical point $CO_2$ drying can be used to make pharmaceutical formulations with very low residual solvent content and increased fine particle fractions compared to conventional filtered samples. Critical point drying using supercritical CO2 is a well-established technique for tissue samples. It involves pumping subcritical or supercritical $CO_2$ into or through a sample pre-immersed or suspended in a miscible solvent such as acetone, isopropanol or ethanol. The solvent dissolves into the $CO_2$ leaving the sample immersed in a fluid that can be heated above its critical point and expanded through an exhaust outlet without formation of a liquid-gas interface. This minimises capillary forces and significantly reduces inter-particle aggregation and compaction. Critical point drying is not suitable for samples with high aqueous content because water is not sufficiently soluble in $CO_2$.

**Experimental**

Subtilisin Carlsberg was coprecipitated with D,L-valine (60mg/ml) into 2-propanol (saturated with D,L-valine) by a continuous process to give a theoretical protein loading of 10% and a water content in solvent of 3.9% v/v. The suspension was allowed to settle, excess solvent decanted and the remaining suspension rinsed successively with acetone to remove excess 2-propanol and bring the water content of solvent to 0.5% v/v. One aliquot of the suspension was dried by filtration on a Durapore 0.4 micron filter (SC/DLVal 2) a second sample was dried by critical point drying (SC/DLVal 3).

50 mg of each sample was weighed with the minimum of handling into a separate vial and following settling by gentle agitation a photograph of the two vials taken and is shown in Figure 33. The sample prepared by critical point drying is on the left and the powder is clearly fluffier and of lower tap density than the filtered sample on the right. Preferably critical point dried samples have a tap density of less than 0.1 g/ml than samples prepared by filtration and more preferably. The lower powder density is indicative of reduced particle-particle interactions and is particularly advantageous for pharmaceutical applications such as delivery to the lung. The favourable aerodynamic properties of dry power formulations made by critical point drying of bioactive molecule coated microcrystals mean they can be used directly within inhalor devices. They therefore do not need to be mixed with larger carrier particles. Particularly preferred are bioactive molecule coated D,L-valine microcrystals.

Critical Point Drying was carried out using a Polaron E3000 to produce the dried powder.

SEM images of the samples were captured using a Jeol JSM 6400 scanning microscope. These showed that the typical flake-like microcrystals observed on precipitation from isopropanol were retained following the acetone rinse and critical point drying. The protein content of the reconstituted samples was determined at 280 nm by UV spectroscopy. Loadings close to the expected value of 10% were obtained as shown in Table Critical drying. The discrepancy may be due to removal of solvent soluble impurities that absorb at 280 nm or loss of protein during processing. The activity of subtilisin Carlsberg was determined by monitoring the hydrolysis of nitrophenyl acetate using UV/vis spectroscopy. The table below shows the activity retained following processing and drying as a percentage of the initial activity of the protein before drying. The SC/DLVal 1 sample was isolated directly from the isopropanol suspension initially obtained. Determination of the activity values was carried out in duplicate. It can be seen that the critical point drying leads to reduced activity relative to samples that are immediately filtered and dried. Nevertheless activities of greater than 70% can be obtained without addition of typical stabilizing agents commonly used in protein drying such as sugars.

Table Critical drying

| Sample | Protein loading % | Activity retained % |
| --- | --- | --- |
| SC/DLVal 1, isopropanol rinse, Millipore filtration 3/12/03 | 9.3 | 91.5 |
| SC/DLVal 2, acetone rinse, Millipore filtration 3/12/03 | 8.9 | 88.0 |
| SC/DLVal 3, acetone processing, Critical Point Drying 3/12/03 | 9.4 | 70.5 |

**Example 20**

**Zeta potentials**

The core microcrystal and protein coating that are characteristic of protein coated coated microcrystals arise from a single continuous self-assembly process. In order to assess whether electrostatic binding of the bioactive molecule to pre-formed microcrystals might be important in the mechanism of this process it was of interest to measure the surface potential of the microcrystals in a non-aqueous medium. The liquid layer surrounding a charged particle exists as two parts; an inner region (Stern layer) where the ions are strongly bound and an outer (diffuse) region where they are less firmly associated. Within the diffuse layer there is a notional boundary inside which the ions and particles form a stable entity. When a particle moves (e.g. due to gravity), ions within the boundary move too. Those ions beyond the boundary do not travel with the particle. The potential at this boundary (surface of hydrodynamic shear) is the zeta potential. The sign and magnitude of the zeta potential depends on the surface charge of the particle with for example a negative zeta potential indicating a particle with an overall negative charge. A Malvern Zetasizer that employs laser Doppler velocimetry was used to measure the sign and approximate magnitude of the Zeta potential of microcrystals produced by precipitation of various core materials at fixed pH. The measurements were made on pre-prepared microcrystals or protein coated microcrystals suspended as dilute suspension in acetonitrile. A polystyrene latex was used to calibrate the machine. The data are shown in Table Zeta-potentials. Glycine, glutamine and valine microcrystals precipitated into solvent in the absence of protein all exhibit negative Zeta potentials. If electrostatic binding is important to the mechanism of formation it would be expected that only biomolecules with an overall positive charge would be expected to form a coating on these negatively charged materials. The charge on a protein is a function of pH. It will be negative at pH values above the pI and positive at a pH below the pI. Using the protein, adenosine deaminase (ADM) which has a reported pI of 4.85 it was found that protein coated microcrystals could be straightforwardly prepared with the above carrier materials by coprecipitation at a pH above the pI. The Zeta potential of these protein coated microcrystals are given in Table Zeta-potentials. The retained negative values are consistent with the adenosine deaminase molecules coating the crystals because at the pH of the coprecipitation the protein will also be negatively charged. There is a clear increase in Zeta potential due to the negative protein coating for the adenosine deaminase coated valine crystals (ADM/valine) prepared at pH 7.02. These results demonstrate that the negatively charged protein can be coated onto microcrystals of materials that exhibit the same negative surface charge via the coprecipitation process. This indicates that the mechanism of coating cannot be ascribed to electrostatic binding of the bioactive molecule to pre-formed microcrystals. Further indication of the absence of an electrostatic binding mechanism is given by the fact that polyanions such as nucleic acids can also be used to efficiently coat microcrystals by coprecipitation. For example DNA coated valine micocrystals can be produced despite the negative Zeta potentials observed for bare valine crystals. Coprecipitation hence provides a generic process for obtaining microcrystals coated with bioactive molecules and advantageously can be carried out efficiently over a wide range of pH and salt conditions.

Table Zeta potentials

| Sample | Precipitation pH | Zeta potential (mV) | Width |
|---|---|---|---|
| glycine | 6.04 | -49.7 | 11.5 |
| glutamine | 5.59 | -54.8 | 9.2 |
| Valine | 7.02 | -19.6 | 10.5 |
| ADM[a]/glycine | 6.04 | -55.7 | 12.0 |
| ADM[a]/glutamine | 5.59 | -56.4 | 13.6 |
| ADM[a]/valine | 7.02 | -36.1 | 8.8 |
| ADM = adenosine deaminase | | | |

**Example 21**

**Comparing bioactivities of samples prepared in a batch coprecipitator and a continuous flow precipitator**

Surprisingly it has been found that reconstituted bioactive molecule formulations prepared by a continuous flow coprecipitation can advantageously show higher bioactivity than samples prepared by the previously reported batch process. This effect is demonstrated here for the enzymes Glucose oxidase and Lactate dehydrogenase because their bioactivities .may be measured to a high degree of precision using standard enzyme assays. Similar improvements using the flow coprecipitator can be obtained with therapeutic biomolecules and other bioactive molecules. The bioactive molecules in formulations prepared by the continuous flow process can also show higher stability, for example, at elevated temperature and increased humidity and be more resistant to aggregation, chemical degradation or denaturation on storage. In the following examples the Samples were prepared using the same composition starting materials by either batch coprecipitation or continuous flow precipitation methods and their bioactivities compared.

The continuous flow precipitator system was similar to in Example 9 but refined by implementing back-pressure regulation. A minimum back-pressure of 100 psi is advantageous in that this ensures that the HPLC pump check valves function properly. A back pressure can be introduced by a number of methods including: introducing a sizeable length of narrow bore tubing, acting as a constriction in the line; introducing a static back flow regular, such as an Upchurch In-line Check Valve; implementing a manometric module e.g. a Gilson 302 manometric module, which monitors the back pressure experienced by the pumps. A manometric module can be used on the solvent line and narrow bore tubing on the aqueous line. Typically flow precision of <1%RSD should be achievable.

**Glucose oxidase coprecipitated with glycine into isopropanol**

Glucose oxidase (GO), 2.5mg/ml, was co-precipitated with glycine into isopropanol as an anti-solvent at 25°C.

In the batch process 0.5 ml of GO/glycine aqueous solution was co-precipitated by drop-wise addition into 9.5 ml of glycine/isopropanol, in a 30 ml vial, using a 25 mm stirrer bar stirring at 750 rpm. In the continuous flow process the flow of GO/glycine aqueous solution was 0.25 ml/min and the flow of glycine/isopropanol was 4.75 ml/min. The flow cell impeller speed was 750 rpm.

The samples were retained as suspensions prior to assay. Enzyme activity was measured using a standard glucose oxidase assay, monitoring the increase in absorbance at 460 nm resulting from the oxidation of O-dianisidine through a peroxidase-coupled system. Reaction conditions: 2.5 ml o-dianisidine-buffer mixture, 300 $\mu$l 18% glucose solution, 100 $\mu$l 0.2 mg/ml peroxidase solution and 100 $\mu$l 0.01 mg/ml GO preparation.

The results are shown in Table 37 Glucose oxidase.

**Table 37: Glucose oxidase**

| Batch process (dAbs/min) | Continuous process (dAbs/min) |
|---|---|
| 0.0123 [a] | 0.0188[a] |
| [a] %RSD < 2.5 | |

## Co-precipitation of Lactate dehydrogenase / L-glutamine in ethanol.

D-Lactate dehydrogenase (LDH) from Lactobacillus sp. was coprecipiated with L-glutamine. A saturated solution (~100 ml) of L-glutamine in deionised water, (~150mg/ml) was prepared, by stirring in an incubator at 40°C overnight cooling to room temperature and filtering through a 0.45 $\mu$m Durapore (Millipore) filter. The pH of this solution was adjusted to pH 7.3 with hydrochloric acid. LDH, (3.15 mg) and bovine serum albumin (16 mg) were dissolved in 10 ml of L-glutamine aqueous solution, swirling gently to aid dissolution. Albumin was used as a protein diluent and coprecipitates with the LDH. The final LDH concentration in the LDH/L-glutamine aqueous solution was 0.315 mg/ml. In the batch process 0.5 ml of LDH/L-glutamine aqueous solution was co-precipitated by drop-wise addition into 9.5 ml of L-glutamine saturated ethanol, in a 30 ml vial, using a 25 mm stirrer bar stirring at 750 rpm at 25°C. In the continuous flow precipitator, the flow of LDH/L-glutamine aqueous solution was 0.25 ml/min; the flow of L-glutamine saturated ethanol solution was 4.75 ml/min. The flow cell impeller speed was 750 rpm at 25°C.

LDH activity was measured at 25°C in 3 ml reaction mixture consisting of 2.8 ml of 0.2M Tris (hydroxymethyl)-aminomethane buffer, 100 $\mu$l of 6.6mM NADH solution and 100 $\mu$l of 30mM sodium pyruvate solution (Both NADH and sodium pyruvate prepared in 0.2M Tris buffer). The LDH preparation (100 $\mu$l of 0.0005 mg/ml) was added to the reaction mixture, the cuvette was inverted 3 times, then the absorbance increase at 340 nm was monitored for -30 minutes with a Beckmann Coulter DU800 spectrophotometer. Activity of PCMCs was measured approximately 24hrs after co-precipitation. The results are shown in Table 38: Lactate dehydrogenase.

**Table 38: Lactate dehydrogenase**

| Batch process (dAbs/min) | Continuous process (dAbs/min) |
|---|---|
| 0.031[a] | 0.039 [a] |
| [a] % RSD < 2.5 | |

## Conclusions

In these examples the bioactivity of protein samples prepared in a continuous flow precipitator are surprisingly found to be higher than those prepared in a batch reactor despite using the same starting compositions. It is not certain what causes this. During the mixing step the air-solvent interface in the flow-precipitator is considerably lower and also the bioactive molecule and the resultant coated microcrystals are exposed to shear forces arising from mixing for less time. This may maximise the percentage of coprecipitated molecules that remain in a stable native or near-native conformation. This is consistent with improvements observed in the storage stability of biomolecule formulations prepared using the flow coprecipitator. Better retention of bioactivity and enhanced stability towards elevated temperature and humidity are very advantageous properties for biopharmaceutical formulations. Higher bioactivity can produce increased therapeutic potency while enhanced stability of the bioactive molecule during storage will reduce the risk of adverse side effects such as immune reactions that can arise from administration of a small percentage of degraded product.

**Claims**

1. A continuous method of forming particles which comprise microcrystals with a non-hygroscopic inner crystalline core comprising coprecipitant molecules and an outer coating comprising one or more bioactive molecules, wherein the bioactive molecule is a peptide, polypeptide, protein, nucleic acid, sugar, vaccine component, or mixtures thereof comprising the following steps:

   (a) providing a continuous stream of an aqueous solution comprising coprecipitant molecules at a concentration of less than 150 mg/ml and bioactive molecules, each coprecipitant molecule having molecular weight of less than 4kDa, wherein the aqueous solution is capable of forming a coprecipitate which comprises the bioactive molecules and coprecipitant with a melting point of above about 90°C;
   (b) rapidly admixing the continuous stream of bioactive molecule/coprecipitant molecule solution with a greater volume of a continuous stream of a substantially water miscible organic solvent such that the coprecipitant and bioactive molecules coprecipitate from solution forming said particles, wherein the continuous streams are mixed in a continuous process;
   (c) optionally isolating the particles from the organic solvent; and wherein the continuous solvent stream is delivered 4-100 times faster than the continuous aqueous stream.

2. A method according to claim 1, wherein following mixing with the bioactive molecule the coprecipitant will be at between 5 and 100% or between 20 and 80% of its aqueous saturation solubility.

3. A method according to any preceding claim wherein the coprecipitant has a lower solubility in the miscible organic solvent than in the aqueous solution.

4. A method according to any preceding claim wherein an excess of fully water miscible organic solvent is such that the final water content of the solvent/aqueous mixture is less than 30 vol%, less than 10-20 vol% or less than 8 vol%.

5. A method according to any preceding claim wherein the water miscible organic solvent is selected from any of the following: methanol; ethanol propan-1-ol; propan-2-ol; acetone, ethyl lactate, tetrahydrofuran, 2-methyl-2,4-pentanediol, 1,5-pentanediol, and various size polyethylene glycol (PEGS) and polyols; or any combination thereof.

6. A method according to any preceding claim wherein the organic solvent is pre-saturated with the bioactive molecule and/or coprecipitate to ensure that on addition and mixing of the aqueous solution the two components precipitate out together.

7. A method according to any preceding claim wherein the aqueous phase is continuously mixed with a large excess of the solvent phase and a mixing process that is turbulent or near turbulent is used.

8. A method according to any preceding claim wherein the aqueous solution is added to organic solvent as a continual stream, spray or mist.

9. A method according to any preceding claim wherein the water miscible organic solvent or mixture of solvents is continuously flowed and mixed with a continuously flowed aqueous stream comprising a bioactive molecule and coprecipitant solution producing a combined output flow that contains suspended bioactive molecule coated microcrystal particles.

10. A method according to any preceding claim wherein upon admixing the bioactive molecule/coprecipitant solution to the excess of the water miscible organic solvent, precipitation of the bioactive and coprecipitant begins to occur almost immediately.

11. The method according to any preceding claim, wherein the molecules forming the crystalline core have a solubility in water of less than about 150 mg/ml or less than about 80 mg/ml.

12. The method according to any of preceding claim, wherein the molecules which make up the crystalline core are selected from any of the following: amino acids, zwitterions, peptides, sugars, buffer components, water soluble drugs, organic and inorganic salts, compounds that form strongly hydrogen bonded lattices or derivatives or any combinations thereof.

13. The method according to any preceding claim, wherein bioactive molecules forming a coating on the crystalline core are selected from any molecule capable of producing a therapeutic effect such as an active pharmaceutical ingredient (API).

14. The method according to any preceding claim, wherein the coating of bioactive molecules also comprises excipients commonly used in pharmaceutical formulations such as stabilizers, surfactants, isotonicity modifiers and pH/buffering agents.

15. The method according to any of the preceding claims, wherein the bioactive molecules comprise: anti-inflammatories, anti-cancer agents, anti-psychotic agents, anti-bacterial agents, anti-fungal agents; natural or unnatural peptides; proteins such as insulin, $\alpha$1-antitrypsin, $\alpha$-chymotrypsin, albumin, interferons, antibodies; nucleic acids such as fragments of genes, DNA from natural sources or synthetic oligonucleotides, anti-sense nucleotides and RNA; and sugars such as any mono-, di- or polysaccharides; and plasmids.

16. The method according to any preceding claim, wherein the particles are for use in a vaccine and include antigenic components of a disease causing agent, such as a bacterium or virus, such as diphtheria toxoid and/or tetanus toxoid.

17. The method according to claim 15, wherein the vaccine is a sub-unit, attenuated or inactivated organism vaccines such as diphtheria, tetanus, polio, pertussus and hepatitis A, B and C, HIV, rabies and influenza.

18. The method according to claim 16, wherein the vaccine is diphtheria toxoid coated D,L-valine or L-glutamine crystals.

19. The method according to any preceding claim, wherein the particles comprise polysaccharides linked to proteins such as HiB (haemopholis influenza B) and pneumococcal proteins, live viruses such as mumps, measles, rubella and flu such as MV A vectored influenza vaccine.

20. The method according to any preceding claim, wherein the particles are used for formulation of vaccines developed for cancers, especially human cancers, including melanomas, skin cancer, lung cancer, breast cancer, colon cancer and other cancers.

21. The method according to any preceding claim, wherein the particles comprise the following: a crystalline core of valine and a coating of insulin; a crystalline core of glycine and a coating of antitrypsin, a crystalline core of Na glutamate and a coating of insulin; a crystalline core of methionine and a coating of insulin; a crystalline core of alanine and a coating of insulin; a crystalline core of valine and a coating of insulin; a crystalline core of histidine and a coating of insulin; a crystalline core of glycine and a coating of $\alpha$-antitrysin; a crystalline core of glutamine and a coating of albumin: a crystalline core of valine and a coating of oligonucleotides DQA-HEX; a crystalline core of valine and a coating of $\alpha$1-antitrypsin with a further anti-oxidant outer coating of N-acetyl cystein; a crystalline core of valine and a coating of ovalbumin; a crystalline core of valine and a coating of diptheria toxoid; a crystalline glutamine and a coating of diptheria toxoid, a crystalline core of valine and a coating of diptheria toxoid; a crystalline core of the glutamine and a coating of tetanus toxoid; a crystalline core of valine and a coating of a mixture of diptheria toxoid and tetanus toxoid; a crystalline core of glutamine and a coating of a mixture of diptheria toxoid and tetanus toxoid.

22. The method according to any preceding claim, wherein following exposure to temperature of up to 60 °C for 1 week and reconstitution in aqueous solution the bioactive molecule retains a biological activity substantially similar to that of a freshly prepared formulation.

23. The method according to any preceding claim, wherein the formulation is intended to be delivered to a recipient by parenteral, pulmonary, nasal, sublingual, intraveneous, rectal, vaginal, intra-anal or oral-administration.

24. The method according to any preceding claim further comprising formulating a dry powder of bioactive molecule coated microcrystals with a bulk density of less than about 0.3g/ml or less than about 0.1 g/ml.

25. The method according to any preceding claim wherein parenteral or pulmonary formulations or other formulations are produced that on administration provide sustained or extended therapeutic effects

**Patentansprüche**

1. Kontinuierliches Verfahren zum Bilden von Partikeln, die Mikrokristalle mit einem nicht-wasseranziehenden inneren kristallinen Kern umfassen, der kopräzipitierende Moleküle und einen äußeren Überzug umfasst, der ein oder mehrere bioaktive Moleküle umfasst, wobei es sich bei dem bioaktiven Molekül um ein Peptid, ein Polypeptid, ein Protein, eine Nukleinsäure, Zucker, eine Vakzinkomponente oder Mischungen dieser handelt, wobei das Verfahren die folgenden Schritte umfasst:

   (a) das Erzeugen eines kontinuierlichen Stroms einer wässerigen Lösung, die kopräzipitierende Moleküle in einer Konzentration von weniger als 150 mg/ml und bioaktive Moleküle umfasst, wobei jedes kopräzipitierende Molekül ein Molekulargewicht von weniger als 4 kDa aufweist, wobei die wässerige Lösung ein Kopräzipitat bilden kann, das bioaktive Moleküle umfasst und mit einem Schmelzpunkt von über etwa 90 °C kopräzipitiert;
   (b) das schnelle Zumischen zu dem kontinuierlichen Strom einer Lösung aus bioaktivem Molekül/kopräzipitierendem Molekül eines größeren Volumens eines kontinuierlichen Stroms eines im Wesentlichen wassermischbaren organischen Lösungsmittels, so dass die kopräzipitierenden und bioaktiven Moleküle aus der Lösung kopräzipitieren, so dass die genannten Partikel gebildet werden, wobei die kontinuierlichen Ströme in einem kontinuierlichen Prozess gemischt werden;
   (c) das optionale Isolieren der Partikel aus dem organischen Lösungsmittel;

   und wobei der kontinuierliche organische Lösungsmittelstrom 4- bis 100-mal schneller bereitgestellt wird als der kontinuierliche wässerige Strom.

2. Verfahren nach Anspruch 1, wobei nach dem Mischen mit dem bioaktiven Molekül das Kopräzipitat bei 5 bis 100 % oder 20 bis 80 % seiner wässerigen Sättigungslöslichkeit ist.

3. Verfahren nach einem der vorstehenden Ansprüche, wobei das Kopräzipitat in dem mischbaren organischen Lösungsmittel eine geringere Löslichkeit aufweist als in der wässerigen Lösung.

4. Verfahren nach einem der vorstehenden Ansprüche, wobei ein Überschuss an vollständig wassermischbarem organischem Lösungsmittel so gegeben ist, dass der letztendliche Anteil aus Lösungsmittel/wässeriger Mischung geringer ist als 30 Volumenprozent, geringer als 10 bis 20 Volumenprozent oder geringer als 8 Volumenprozent.

5. Verfahren nach einem der vorstehenden Ansprüche, wobei das wassermischbare organische Lösungsmittel aus der folgenden Gruppe ausgewählt wird: Methanol; Ethanol; Propan-1-ol; Propan-2-ol; Aceton, Ethyllactat, Tetrahydrofuran, 2-Methyl-2,4-Pentandiol, 1,5-Pentandiol und Polyethylenglykol verschiedener Größen (PEGS) und Polyole; oder jeder Kombination dieser.

6. Verfahren nach einem der vorstehenden Ansprüche, wobei das organische Lösungsmittel vorgesättigt ist mit dem bioaktiven Molekül und/oder Kopräzipitat, um sicherzustellen, dass die beiden Komponenten beim Zusatz und Mischen mit der wässerigen Lösung gemeinsam ausfällen.

7. Verfahren nach einem der vorstehenden Ansprüche, wobei die wässerige Phase kontinuierlich mit einem großen Überschuss der Lösungsmittelphase gemischt wird, und wobei ein wirbelnder oder nahezu wirbelnder Mischprozess eingesetzt wird.

8. Verfahren nach einem der vorstehenden Ansprüche, wobei die wässerige Lösung dem organischen Lösungsmittel als ein kontinuierlicher Strom, Sprühnebel oder Dunstnebel hinzugefügt wird.

9. Verfahren nach einem der vorstehenden Ansprüche, wobei das wassermischbare organische Lösungsmittel oder die Mischung aus Lösungsmitteln kontinuierlich fließt und mit einem kontinuierlich fließenden wässerigen Strom gemischt wird, der ein bioaktives Molekül und eine kopräzipitierende Lösung umfasst, die einen kombinierten Ausgangsfluss erzeugen, der mit aufgelöstem bioaktivem Molekül überzogene Mikrokristallpartikel enthält.

10. Verfahren nach einem der vorstehenden Ansprüche, wobei nach dem Zumischen des bioaktiven Moleküls/der kopräzipitierenden Lösung zu dem Überschuss des wassermischbaren organischen Lösungsmittels, die Präzipitation des bioaktiven Moleküls und des Kopräzipitats nahezu unverzüglich beginnt.

11. Verfahren nach einem der vorstehenden Ansprüche, wobei die kristallinen Kern bildenden Moleküle eine Löslichkeit in Wasser von weniger als etwa 150 mg/ml oder von weniger als etwa 80 mg/ml aufweisen.

12. Verfahren nach einem der vorstehenden Ansprüche, wobei die Moleküle, die den kristallinen Kern bilden, aus den folgenden ausgewählt werden: Aminosäuren, Zwitterione, Peptide, Zuckerarten, Pufferkomponenten, wasserlösliche Arzneistoffe, organische und anorganische Salze, Verbindungen, die stark wasserstoffgebundene Gitter bilden oder Derivate oder Kombinationen dieser.

13. Verfahren nach einem der vorstehenden Ansprüche, wobei die bioaktiven Moleküle, welche einen Überzug auf dem kristallinen Kern bilden, aus allen Molekülen ausgewählt werden, die einen therapeutischen Effekt erzeugen können, wie zum Beispiel ein aktiver pharmazeutischer Wirkstoff (API).

14. Verfahren nach einem der vorstehenden Ansprüche, wobei der Überzug aus bioaktiven Molekülen ferner Arzneistoffträger umfasst, die für gewöhnlich in pharmazeutischen Zusammensetzungen eingesetzt werden, wie zum Beispiel Stabilisatoren, Tenside, Isotonie-Modifikatoren und pH/-Puffermittel.

15. Verfahren nach einem der vorstehenden Ansprüche, wobei die bioaktiven Moleküle folgendes umfassen: Substanzen zur Entzündungsbekämpfung, Substanzen zur Krebsbekämpfung, antipsychotische Substanzen, antibaktierelle Substanzen, Antimykotika; **natürliche oder künstliche Peptide; Proteine wie Insulin, $\alpha$1-Antitrypsin, $\alpha$-Chymotrypsin,** Albumin, Interferone, Antikörper; Nukleinsäuren wie etwa Fragmente von Genen, DNA natürlichen Ursprungs oder synthetische Oligonukleotide, Antisense-Nukleotide und RNA; sowie Zuckerarten wie etwa alle Mono-, Di- oder Polysaccharide; und Plasmide.

16. Verfahren nach einem der vorstehenden Ansprüche, wobei die Partikel für eine Verwendung in einem Impfstoff vorgesehen sind und antigene Komponenten einer eine Erkrankung verursachenden Substanz aufweisen, wie etwa eines Bakteriums oder eines Virus, wie etwa Diphterietoxoid und/oder Tetanustoxoid.

17. Verfahren nach Anspruch 15, wobei es sich bei dem Impfstoff um eine Untereinheit, gedämpfte oder inaktivierte Organismus-Impfstoffe wie etwa gegen Diphtherie, Tetanus, Polio, Pertussis und Hepatitis A, B und C, HIV, Tollwut und Influenza.

18. Verfahren nach Anspruch 16, wobei es sich bei dem Impfstoff um mit Diptherietoxoid überzogenes D,L-Valin oder L-Glutamin-Kristalle.

19. Verfahren nach einem der vorstehenden Ansprüche, wobei die Partikel Polysaccharide umfassen, die mit Proteinen verknüpft sind, wie etwa HiB (Haemophilus Influenza B) und Pneumokokkenproteine, lebende Viren wie Mumps, Masern, Röteln und Grippe wie etwa Influenza-Impfstoff mit MVA-Vektor.

20. Verfahren nach einem der vorstehenden Ansprüche, wobei die Partikel für die Zubereitung von Impfstoffen verwendet werden, die zur Behandlung von Krebserkrankungen entwickelt werden, insbesondere Krebserkrankungen von Menschen, darunter Melanome, Hautkrebs, Lungenkrebs, Brustkrebs, Dickdarmkrebs und andere Krebsarten.

21. Verfahren nach einem der vorstehenden Ansprüche, wobei die Partikel folgendes umfassen: einen kristallinen Kern aus Valin und einen Überzug aus Insulin; einen kristallinen Kern aus Glycin und einen Überzug aus Antritrypsin, einen kristallinen Kern aus Na-Glutamat und einen Überzug aus Insulin; einen kristallinen Kern aus Methionin und einen Überzug aus Insulin; einen kristallinen Kern als Alanin und einen Überzug aus Insulin; einen kristallinen Kern aus Valin und einen Überzug aus Insulin; einen kristallinen Kern aus Histidin und einen Überzug aus Insulin; einen kristallinen Kern aus Glycin und einen **Überzug aus $\alpha$-Antitrysin; einen kristallinen Kern aus Glutamin und einen Überzug aus** Albumin; einen kristallinen Kern aus Valin und einen Überzug aus Oligonukleotiden DQA-**HEX; einen kristallinen Kern aus Valin und einen Überzug aus $\alpha$1-Antitrypsin mit einem** weiteren äußeren Antioxidationsüberzug aus N-Acetylcystein; einen kristallinen Kern aus Valin und einen Überzug aus Ovalbumin; einen kristallinen Kern aus Valin und einen Überzug aus Diphterietoxoid und Tetanustoxoid; einen kristallinen Kern aus Valin und einen Überzug aus einer Mischung aus Diptherietoxoid und Tetanustoxoid; einen kristallinen Kern aus Glutamin und einen Überzug aus einer Mischung aus Diptherietoxoid und Tetanustoxoid.

22. Verfahren nach einem der vorstehenden Ansprüche, wobei das bioaktive Molekül, nachdem es eine Woche lang einer Temperatur von bis zu 60 °C ausgesetzt worden ist und nach einer Rekonstitution in wässeriger Lösung, eine biologische Aktivität erhält, die der biologischen Aktivität einer frisch hergestellten Präparation im Wesentlichen identisch ist.

23. Verfahren nach einem der vorstehenden Ansprüche, wobei die Zusammensetzung einem Empfänger verabreicht werden soll durch parenterale, pulmonale, nasale, sublinguale, intravenöse, rektale, vaginale intraanale oder orale Verabreichung.

24. Verfahren nach einem der vorstehenden Ansprüche, wobei dieses ferner die Herstellung eines trockenen Pulvers aus einem bioaktiven Molekül, überzogen mit Mikrokristallen mit einer Massendichte von unter etwa 0,3 g/ml oder unter etwa 0,1 g/ml umfasst.

25. Verfahren nach einem der vorstehenden Ansprüche, wobei parenterale oder pulmonale Zusammensetzungen oder sonstige Zusammensetzungen erzeugt werden, die bei Verabreichung nachhaltige oder anhaltende therapeutische Wirkungen bereitstellen.

**Revendications**

1. Procédé continu pour former des particules qui comprennent des microcristaux avec un noyau cristallin interne non hygroscopique comprenant des molécules de coprécipitant et un enrobage extérieur comprenant une ou plusieurs molécules bioactives, dans lequel la molécule bioactive est un peptide, un polypeptide, une protéine, de l'acide nucléique, du sucre, un composant vaccinal, ou des mélanges de ceux-ci, comprenant les étapes suivantes consistant à :

   (a) fournir un flux continu d'une solution aqueuse comprenant des molécules de coprécipitant à une concentration inférieure à 150 mg/ml et des molécules bioactives, chaque molécule de coprécipitant ayant un poids moléculaire inférieur à 4 kDa, dans lequel la solution aqueuse est capable de former un coprécipité qui comprend les molécules bioactives et un coprécipitant avec un point de fusion supérieur à environ 90 °C ;
   (b) mélanger rapidement le flux continu de solution de molécule bioactive/molécule de coprécipitant avec un plus grand volume d'un flux continu d'un solvant organique sensiblement miscible à l'eau de telle sorte que les molécules de coprécipitant et bioactive co-précipitent de la solution formant lesdites particules, dans lequel les flux continus sont mélangés dans un procédé continu ;
   (c) en option isoler les particules du solvant organique ;

   et dans lequel le flux de solvant continu est fourni 4 à 100 fois plus vite que le flux aqueux continu.

2. Procédé selon la revendication 1, dans lequel après le mélange avec la molécule bioactive le coprécipitant sera compris entre 5 et 100 % ou entre 20 et 80 % de sa solubilité de saturation aqueuse.

3. Procédé selon l'une quelconque des revendications précédentes, dans lequel le coprécipitant a une solubilité inférieure dans le solvant organique miscible que dans la solution aqueuse.

4. Procédé selon l'une quelconque des revendications précédentes, dans lequel un excès de solvant organique entièrement miscible à l'eau est tel que la teneur finale en eau du solvant/mélange aqueux est inférieure à 30 % en volume, inférieure de 10 à 20 % en volume ou inférieure à 8 % en volume.

5. Procédé selon l'une quelconque des revendications précédentes, dans lequel le solvant organique miscible à l'eau est choisi parmi l'un quelconque de : méthanol ; éthanol propan-1-ol ; propan-2-ol ; acétone ; lactate d'éthyle, tétrahydrofurane, 2-méthyl-2,4-pentanediol, 1,5-pentanediol, et polyéthylèneglycols (PEG) et polyols de différentes tailles, ou toute combinaison de ceux-ci.

6. Procédé selon l'une quelconque des revendications précédentes, dans lequel le solvant organique est pré-saturé avec la molécule bioactive et/ou le coprécipitant pour garantir que lors de l'ajout et du mélange de la solution aqueuse les deux composants précipitent ensemble.

7. Procédé selon l'une quelconque des revendications précédentes, dans lequel la phase aqueuse est mélangée en continu avec un large excès de la phase de solvant et un procédé de mélange qui est agité ou presque agité est utilisé.

8. Procédé selon l'une quelconque des revendications précédentes, dans lequel la solution aqueuse est ajoutée au solvant organique sous forme de flux, pulvérisation ou brouillard continu.

**9.** Procédé selon l'une quelconque des revendications précédentes, dans lequel le solvant organique miscible à l'eau ou le mélange de solvants est écoulé et mélangé en continu avec un flux aqueux s'écoulant en continu comprenant une solution de molécule bioactive et de coprécipitant produisant un flux de sortie combiné qui contient des particules de microcristaux enrobées de molécule bioactive en suspension.

**10.** Procédé selon l'une quelconque des revendications précédentes, dans lequel lors du mélange de la solution de molécule bioactive/coprécipitant à l'excès de solvant organique miscible à l'eau, la précipitation de la molécule bioactive et du coprécipitant commence à se produire presque immédiatement.

**11.** Procédé selon l'une quelconque des revendications précédentes, dans lequel les molécules formant le noyau cristallin ont une solubilité dans l'eau inférieure à environ 150 mg/ml ou inférieure à environ 80 mg/ml.

**12.** Procédé selon l'une quelconque des revendications précédentes, dans lequel les molécules qui constituent le noyau cristallin sont choisies parmi l'un des éléments suivants : acides aminés, zwitterions, peptides, sucres, composants tampons, médicaments solubles dans l'eau, sels organiques et inorganiques, composés qui forment des treillis fortement liés par hydrogène ou dérivés ou toute combinaison de ceux-ci.

**13.** Procédé selon l'une quelconque des revendications précédentes, dans lequel les molécules bioactives formant un enrobage sur le noyau cristallin sont choisies parmi toute molécule capable de produire un effet thérapeutique, comme un principe actif pharmaceutique (en anglais « active pharmaceutical ingredient » - API).

**14.** Procédé selon l'une quelconque des revendications précédentes, dans lequel l'enrobage de molécules bioactives comprend également des excipients couramment utilisés dans les formulations pharmaceutiques tels que des stabilisants, agents tensioactifs, modificateurs d'isotonicité et agents de pH/tampons.

**15.** Procédé selon l'une quelconque des revendications précédentes, dans lequel les molécules bioactives comprennent : anti-inflammatoires, agents anticancéreux, agents antipsychotiques, agents anti-bactériens, agents anti-fongiques ; peptides naturels ou non **naturels ; protéines telles que insuline, $\alpha$1-antitrypsine, $\alpha$-chymotrypsine, albumine,** interférons, anticorps ; acides nucléiques tels que fragments de gènes, ADN de sources naturelles ou oligonucléotides synthétiques, nucléotides et ARN anti-sens ; et sucres tels que tout mono-, di- ou polysaccharide ; et plasmides.

**16.** Procédé selon l'une quelconque des revendications précédentes, dans lequel les particules doivent être utilisées dans un vaccin et comprennent des composants antigéniques d'un agent pathogène, comme une bactérie ou un virus, tels que l'anatoxine diphtérique et/ou l'anatoxine tétanique.

**17.** Procédé selon la revendication 15, dans lequel le vaccin est un vaccin à germe inactivé, atténué ou à sous-unités tel que diphtérie, tétanos, poliomyélite, coqueluche et hépatite A, B et C, VIH, rage et grippe.

**18.** Procédé selon la revendication 16, dans lequel le vaccin est des cristaux de L-glutamine ou D,L-valine à enrobage d'anatoxine diphtérique.

**19.** Procédé selon l'une quelconque des revendications précédentes, dans lequel les particules comprennent des polysaccharides liés à des protéines telles que des protéines de HIB (haemopholis influenza de type B) et de pneumocoque, virus vivants tels que oreillons, rougeole, rubéole et grippe comme vaccin contre la grippe vectorisé MV A.

**20.** Procédé selon l'une quelconque des revendications précédentes, dans lequel les particules sont utilisées pour la formulation de vaccins développés pour les cancers, notamment les cancers humains, y compris les mélanomes, cancer de la peau, cancer du poumon, cancer du sein, cancer du colon et autres cancers.

**21.** Procédé selon l'une quelconque des revendications précédentes, dans lequel les particules comprennent les éléments suivants : un noyau cristallin de valine et un enrobage d'insuline ; un noyau cristallin de glycine et un enrobage d'antitrypsine ; un noyau cristallin de glutamate de Na et un enrobage d'insuline ; un noyau cristallin de méthionine et un enrobage d'insuline ; un noyau cristallin d'alanine et un enrobage d'insuline ; un noyau cristallin de valine et un enrobage d'insuline ; un noyau cristallin d'histidine et un enrobage **d'insuline ; un noyau cristallin de glycine et un enrobage de $\alpha$-antitrysine ; un noyau** cristallin de glutamine et un enrobage d'albumine ; un noyau cristallin de valine et un **enrobage d'oligonucléotides DQA-HEX ; un noyau cristallin de valine et un enrobage de** $\alpha$**1**-antitrypsine avec un autre enrobage externe anti-oxydant de N-acétyl cystéine ; un noyau cristallin de valine et un enrobage d'ovalbumine ; un noyau cristallin de valine et un enrobage d'anatoxine diphtérique ; un noyau cristallin de glutamine et un enrobage d'anatoxine diphtérique ; un noyau cristallin de valine et un enrobage d'anatoxine diphtérique ; un noyau cristallin de glutamine et un enrobage d'anatoxine tétanique ; un noyau cristallin de valine et un enrobage d'anatoxine diphtérique et d'anatoxine tétanique ; un noyau cristallin de glutamine et un enrobage d'un mélange d'anatoxine diphtérique et d'anatoxine tétanique.

22. Procédé selon l'une quelconque des revendications précédentes, dans lequel, après exposition à une température maximale de 60 °C pendant 1 semaine et reconstitution dans une solution aqueuse, la molécule bioactive conserve une activité biologique sensiblement semblable à celle d'une formulation fraîchement préparée.

23. Procédé selon l'une quelconque des revendications précédentes, dans lequel la formulation est destinée à être administrée à un destinataire par voie parentérale, pulmonaire, nasale, sublinguale, intraveineuse, rectale, vaginale, intra-anale ou orale.

24. Procédé selon l'une quelconque des revendications précédentes, comprenant en outre la formulation d'une poudre sèche de microcristaux enrobés de molécule bioactive ayant une masse volumique apparente inférieure à environ 0,3 g/ml ou inférieure à environ 0,1 g/ml.

25. Procédé selon l'une des revendications précédentes, dans lequel il est produit des formulations parentérales ou pulmonaires ou autres formulations qui, une fois administrée, ont des effets thérapeutiques durables ou prolongés.

Figure1.     Insulin / glycine precipitated in 2-propanol

Figure2.     chymotrypsin / alanine precipitated in 2-propanol

Figure 3.15mg chymotrypsin was dissolved in 3ml of 50 %saturated DL valine solution. 6 ml of the aqueous solution was precipitated in 35 ml of DL valine saturated 2-propanol. The particles were dried using Millipore filtration system.

Figure 4.0.2ml of saturated DL valine solution was precipitated in 60ml unsaturated 2-propanol using Hamilton syringe in mastersizer sample chamber, with a stirrer speed = 2000rpm. Particles formed inside Mastersizer and were directly measured.

Figure 5.,    insulin / L-histidine precipitated in 2-propanol

Figure 6.0.2ml of saturated DL valine precipitated in 60ml unsaturated 2-propanol in mastersizer sample chamber, with a stirrer speed = 1500rpm. Particles formed inside Mastersizer and were directly measured.

Figure 7. 0.6ml L-glutamine saturated solution precipitated in 6ml L-glutamine saturated 2-propanol solution using 5ml pipette under fast stirring. The particles were dried using Millipore filtration system.

Figure 8. 0.6ml L-glutamine saturated solution precipitated in 6ml of L-glutamine saturated 2-propanol solution using small syringe pump under fast stirring. The particles were dried using Millipore filtration system.

Figure 9.5%loading albumin /L-glutamine prec in 2-prop, medium stirring
1mg of albumin dissolved in 0.6ml L-glutamine saturated solution. 0.5ml of this solution was precipitated into 5ml 2-propanol saturated with L-glutamine using syringe pump under medium stirring. The particles were dried using Millipore filtration system.

**File:** L-glutamine(3).XLS
**Sample:** L-glut 1ml pipette medium stirring

DVS Change In Mass (dry) Plot

Target RH

dm

Change In Mass (%) - Dry

Target RH (%)

Time/mins

Figure 10

**File:** glycine.XLS
**Sample:** glycine 9.1% h2O Jo14/11/01

DVS Change In Mass (dry) Plot

Target RH

dm

Change In Mass (%) - Dry

Target RH (%)

Time/mins

Figure 11

Figure 12

Figure 13

Figure 14

Figure 15

Figure 1b

Distribution of D,L valine crystals coated with a blend of DQA-HEX and crude oligonucleotides in the artificial lung. 2-PrOH was used as precipitating solvent. Loading was 18.4% (this was calculated as weight DNA measured by $UV_{260nm}$ per weight OCMC). The fine particle fraction (FPF) was 29.9%.

Figure 17

Figure 18

Figure 19

Figure 20

Figure 21

5 μm

Figure 22

Figure 23

Figure 24

Figure 25

Figure 26

5μm

Figure 27

5μm

Figure 28

5μm

Figure 29

5μm

5μm

Figure 30

5μm

Figure 31

Figure 32

Figure 33

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• WO 0069887 A

**Non-patent literature cited in the description**

• **D. GRAHAM ; B.J. MALLINDER ; D. WHITCOMBE ; N.D WATSON ; W.E SMITH.** *Anal. Chem.,* 2002, vol. 74, 1069-1074
• **KOETZLE et al.** *Acta Cryst. B,* 1973, vol. 29, 2571
• *Acta Cryst B,* 1969, vol. 25, 296
• *Acta Cryst,* 1972, vol. 28, 1827
• *Acta Cryst,* 1960, vol. 13, 35
• *Acta Cryst B,* 1980, vol. 36, 115